(19) Europäisches Patentamt
European Patent Office
Office européen des brevets

(11) Numéro de publication : **0 376 821 B1**

# (12) FASCICULE DE BREVET EUROPEEN

(45) Date de publication du fascicule du brevet : **26.08.92 Bulletin 92/35**

(51) Int. Cl.⁵ : **A61K 7/06,** A61K 7/42

(21) Numéro de dépôt : **89403616.9**

(22) Date de dépôt : **22.12.89**

(54) **Composition pour induire et stimuler la croissance des cheveux et/ou freiner leur chute, à base de dérivés de pyrimidine et de filtres solaires.**

(30) Priorité : **30.12.88 FR 8817466**

(43) Date de publication de la demande : **04.07.90 Bulletin 90/27**

(45) Mention de la délivrance du brevet : **26.08.92 Bulletin 92/35**

(84) Etats contractants désignés : **AT BE CH DE ES FR GB IT LI NL SE**

(56) Documents cités :
**WO-A-83/02558**
**WO-A-87/00427**
**DE-A- 1 492 322**

(73) Titulaire : **L'OREAL**
**14, Rue Royale**
**F-75008 Paris (FR)**

(72) Inventeur : **Grollier, Jean-François**
**16bis Boulevard Morland**
**F-75004 Paris (FR)**
Inventeur : **Richoux, Isabelle**
**54, rue Villiers de l'Isle Adam**
**F-75020 Paris (FR)**

(74) Mandataire : **Casalonga, Axel et al**
**BUREAU D.A. CASALONGA - JOSSE**
**Morassistrasse 8**
**W-8000 München 5 (DE)**

EP 0 376 821 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention est relative à de nouvelles compositions pour induire et stimuler la croissance des cheveux et/ou freiner leur chute contenant, en association, des dérivés de pyrimidine et au moins certains agents filtrant les rayonnements UV.

On recherche depuis de nombreuses années dans l'industrie cosmétique ou pharmaceutique des compositions permettant de supprimer et de réduire l'alopécie et notamment d'induire et de stimuler la croissance des cheveux et/ou de diminuer leur chute.

L'alopécie, comme cela est bien connu, est due, notamment, à une perturbation du cycle pilaire, dans la mesure où l'on constate généralement qu'elle survient lorsque la phase de croissance dite "phase anagène" est raccourcie, ce qui fait que le passage des cheveux en phase télogène est plus précoce et les cheveux tombent en plus grand nombre.

Les cycles de croissance successifs aboutissent à des cheveux de plus en plus fins et de plus en plus courts, se transformant peu à peu en un duvet non pigmenté qui peut conduire à la calvitie.

La variation de ces différentes catégories de cheveux peut être déterminée grâce au trichogramme et en particulier au phototrichogramme.

On a déjà proposé dans cette optique des composés tels que l'amino-6 dihydro-1,2 hydroxy-1 imino-2 pipéridino-4 pyrimidine et ses dérivés. De tels composés sont décrits plus particulièrement dans le brevet US-A-4139619.

On a également décrit dans la demande WO-A-83.02558 des compositions à base de rétinoïdes et de Minoxidil, utilisées notamment pour stimuler la croissance des cheveux humains et traiter certains types d'alopécie.

On a décrit dans la demande EP-A-0336813 des compositions à base de minoxidil et de dérivés d'urée et/ou d'allantoïne tels que le complexe d'allantoïne et de l'acide para aminobenzoïque.

On a cependant constaté que l'amino-6 dihydro-1,2 hydroxy-1 imino-2 pipéridino-4 pyrimidine, encore connue sous la dénomination Minoxidil, présentait des problèmes de solubilisation, ce qui fait que des lotions préconisées en application topique pour le cuir chevelu ne renferment généralement ce composé à l'état solubilisé qu'à des concentrations relativement faibles.

On a donc recherché des moyens susceptibles de modifier avantageusement la solubilité de ces dérivés de pyrimidine, tant pour permettre une dissolution plus rapide de la substance active, que pour empêcher la recristallisation au cours du temps, notamment la cristallisation du Minoxidil sur le cuir chevelu engendrée par l'évaporation du solvant, ce qui entraîne une perte en actif par un phénomène de poudrage dont l'effet cosmétique est en outre indésirable, ou permettre l'emploi de solutions concentrées en Minoxidil diluables avec d'autres compositions contenant des substances actives, au moment de l'emploi.

Par ailleurs, et dans la mesure où ces compositions sont destinées à une application topique sur le cuir chevelu et que généralement cette application n'est pas suivie d'une étape de rinçage, il s'est avéré indispensable que la solubilisation de la substance active s'opère avec des agents cosolubilisants dont les effets sur les cheveux des personnes traitées soient cosmétiques, c'est-à-dire qu'ils n'entraînent pas que les cheveux poissent, collent ou graissent.

On connaît, par ailleurs et depuis longtemps, des agents filtrant les radiations UV utilisés généralement pour la protection de l'épiderme humain, notamment pour préserver la peau des effets néfastes du rayonnement ultraviolet.

La demanderesse a découvert, ce qui fait l'objet de l'invention, que, de façon surprenante, certains agents filtrant les rayonnements UV en protégeant le cuir chevelu des sujets alopéciques directement exposé aux effets éventuellement néfastes des rayonnements UV, permettaient également d'obtenir, pour certains d'entre eux, une solubilisation améliorée des dérivés de pyrimidine.

Cet effet est particulièrement surprenant lorsqu'on sait que les propriétés filtrantes solaires n'ont absolument rien à voir avec les propriétés de cosolubilisation de ces agents filtrants solaires.

Un objet de l'invention est donc constitué par l'association des dérivés de pyrimidine et de certains agents filtrant les radiations UV, dans une composition destinée à induire et à stimuler la croissance des cheveux et diminuer leur chute.

Un autre objet de l'invention est constitué par les compositions cosmétiques et/ou pharmaceutiques contenant une telle association.

Un objet de l'invention est également constitué par les procédés de traitement mettant en oeuvre une telle association, ainsi que les dispositifs de mise en oeuvre de l'association.

D'autres objets de l'invention apparaîtront à la lecture de la description et des exemples qui suivent.

La composition destinée à induire ou à stimuler la croissance des cheveux et à diminuer leur chute, conforme à l'invention, est essentiellement caractérisée par le fait qu'elle renferme dans un milieu physiologiquement acceptable, au moins un dérivé de pyrimidine répondant à la formule :

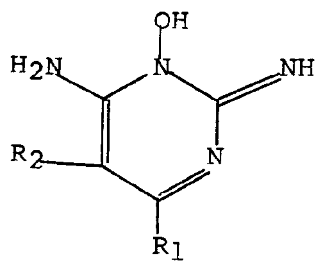

(I)

dans laquelle $R_1$ désigne un groupement

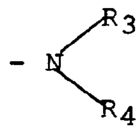

dans lequel $R_3$ et $R_4$ peuvent être choisis parmi l'hydrogène, un groupement alkyle, alcényle, alkylaryle ou cycloalkyle inférieur, $R_3$ et $R_4$ peuvent également former un hétérocycle avec l'atome d'azote auquel ils sont liés choisi parmi les groupements aziridinyle, azétidinyle, pyrrolidinyle, pipéridinyle, hexahydroazépinyle, heptaméthylèneimine, octaméthylène imine, morpholine et alkyle inférieur-4 pipérazinyle, les groupements hétérocycliques pouvant être substitués sur les atomes de carbone par un à trois groupements alkyle inférieur, hydroxy ou alcoxy; le groupement $R_2$ est choisi parmi l'hydrogène, un groupement alkyle, alcényle, alcoxyalkyle, cycloalkyle, aryle, alkylaryle, arylalkyle, alkylarylalkyle, alcoxyarylalkyle, ou haloarylalkyle inférieur, ainsi que les sels d'addition d'acides physiologiquement acceptables;
et au moins un agent filtrant le rayonnement UV choisi parmi les composés suivants :

- la 2-hydroxy 4-méthoxy benzophénone;
- le para-diméthylaminobenzoate de 2-éthyl hexyle;
- le para-diméthylaminobenzoate de pentyle;
- le paraméthoxycinnamate de 2-éthylhexyle;
- le 4-(1,1-diméthyléthyl) 4'-méthoxy dibenzoylméthane;
- le (N-2-éthyl hexyl)3-[(3'-méthoxy 4'-n-butoxy)benzylidène]10-camphosulfonamide;
- le 3-(4-méthylbenzylidène)camphre;
- le salicylate d'homomenthyle;
- le salicylate de 2-éthylhexyle;
- l'acide para-aminobenzoïque;
- l'acide 2-hydroxy 4-méthoxybenzophénone 5-sulfonique; ainsi que leurs mélanges.

Dans la formule (I), les groupements alkyle ou alcoxy désignent de préférence un groupement ayant 1 à 4 atomes de carbone; alcényle désigne de préférence un groupement ayant 2 à 5 atomes de carbone; aryle désigne de préférence phényle; le groupement cycloalkyle désigne de préférence un groupement ayant entre 4 et 6 atomes de carbone; halogène désigne de préférence chlore ou brome.

Les composés de formule (I) plus particulièrement préférés sont choisis parmi les composés dans lesquels $R_2$ désigne hydrogène et $R_1$ représente un groupement :

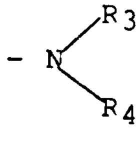

dans lequel $R_3$ et $R_4$ forment un cycle pipéridinyle, ainsi que leurs sels, tels que par exemple le sulfate. Le composé particulièrement préféré est constitué par l'amino-6 dihydro-1,2 hydroxy-1 imino-2 pipéridino-4 pyrimidine, encore appelé "Minoxidil".

Les dérivés de pyrimidine de formule (I) sont utilisés conformément à l'invention, dans des proportions comprises de préférence entre 0,1 et 10% en poids et plus particulièrement entre 1 et 5% en poids par rapport

au poids total de la composition.

Les agents filtrant le rayonnement UV, définis ci-dessus, sont présents de préférence dans des proportions suffisantes pour augmenter la solubilité du dérivé de pyrimidine de formule (I) dans le milieu considéré, cette augmentation étant de préférence supérieure à 10% et en particulier supérieure à 20% par rapport à la solubilité du dérivé de pyrimidine de formule (I) dans ce milieu.

Les agents filtrant le rayonnement solaire UV sont de préférence utilisés dans des proportions comprises entre 0,1 et 10% et de préférence entre 0,3 et 4% par rapport au poids total de la composition.

Les compositions conformes à l'invention peuvent être aqueuses ou bien anhydres, le milieu aqueux ou anhydre étant physiologiquement acceptable.

On appelle milieu aqueux un milieu constitué par de l'eau ou un mélange d'eau et d'un solvant physiologiquement acceptable.

On appelle milieu anhydre, conformément à l'invention, un milieu solvant contenant moins de 1% d'eau. Ce milieu anhydre peut être constitué d'un solvant ou d'un mélange de solvants choisi plus particulièrement parmi les alcools inférieurs en $C_2$-$C_4$ comme l'alcool éthylique, les alkylèneglycols comme le propylèneglycol et les alkyléthers d'alkylèneglycols ou de dialkylèneglycols, les radicaux alkyle ou alkylène étant des radicaux ayant 1 à 4 atomes de carbone.

Ces mêmes solvants, notamment l'alcool éthylique, peuvent être utilisés dans le milieu aqueux.

Les compositions conformes à l'invention peuvent également contenir différents adjuvants habituellement utilisés dans les compositions destinées à une application topique en cosmétique ou en pharmacie, dont les agents conservateurs, les colorants, les parfums.

Ces compositions peuvent également contenir des agents épaississants bien connus dans l'état de la technique.

Une forme de réalisation particulièrement avantageuse de l'invention consiste à utiliser, en plus du dérivé de pyrimidine et de l'agent filtrant solaire, au moins un rétinoïde qui peut être utilisé soit en association dans la même composition ou dans un mélange avec la composition définie ci-dessus au moment de l'emploi, soit en application séquentielle avant ou après l'application de la composition contenant le dérivé de pyrimidine et l'agent filtrant solaire, de façon successive ou décalée dans le temps.

Les rétinoïdes sont en particulier choisis parmi les composés répondant à la formule :

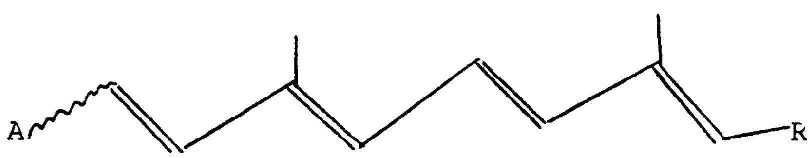

(II)

dans laquelle :

a) A est un groupement choisi parmi les groupements de formules :

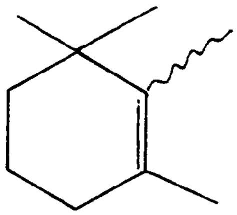
(IIIa)

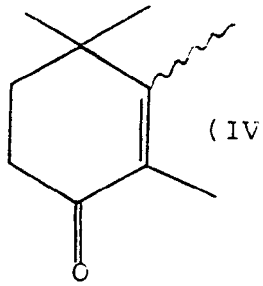
(IV)

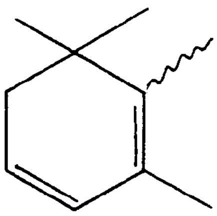
(IIIb)

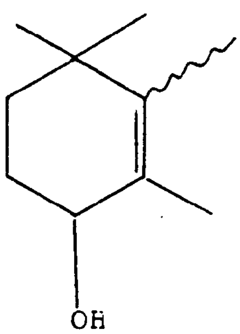
(V)

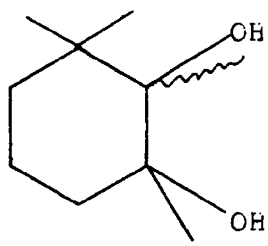
(VI)

dans laquelle :

lorsque A désigne un groupement de formule (IIIa), R est choisi parmi les groupements suivants :

CHO ; $CH_2OR_5$ ,

dans lequel $R_5$ désigne hydrogène, alkyle inférieur en $C_1$-$C_4$ ;

le groupement

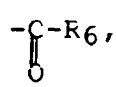

où $R_5$ désigne alkyle linéaire ou ramifié en $C_1$-$C_{16}$ ;

$CH_2SR_7$, dans lequel $R_7$ désigne hydrogène ou méthyle ;

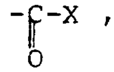

dans lequel X désigne :

(i) OH ;

(ii) $OR_8$ où $R_8$ désigne un radical alkyle en $C_1$-$C_{15}$, arylalkyle en $C_1$-$C_4$, éventuellement substitué sur le groupement aryle, arylcarboxyalkyle en $C_1$-$C_4$, éventuellement substitué sur le groupement aryle,

hydroxyalkyle en $C_1$-$C_4$, amidoalkyle en $C_1$-$C_4$ ;

(iii) $NR_9R_{10}$, dans lequel $R_9$ et $R_{10}$, identiques ou différents, désignent hydrogène, alkyle en $C_1$-$C_6$, hydroxyalkyle en $C_1$-$C_4$, aryle éventuellement substitué ;

$R_9$ ou $R_{10}$ pouvant représenter un hétérocycle éventuellement substitué ou pouvant former conjointement avec l'atome d'azote auquel ils sont reliés, un hétérocycle, lui-même éventuellement substitué ;

(iv) le groupement $N_3$ ;

ou bien un groupement de formule $CH_2NHR_{11}$, dans lequel $R_{11}$ désigne un radical benzoyle éventuellement substitué;

lorsque A désigne un groupement de formule (IIIb), (IV), (V) ou (VI), R désigne COOH ainsi que sa forme salifiée ou estérifiée;

b) A est un groupement choisi parmi les groupements aryle ou aryle substitué, un hétérocycle ou un hétérocycle substitué, un groupement arylhétérocyclique éventuellement substitué sur l'hétérocycle ou arylhomocyclique éventuellement substitué sur le noyau aromatique, R désignant dans ce cas un groupement COOH, $COOR_{12}$ où $R_{12}$ désigne un radical alkyle en $C_1$-$C_4$ ou bien un groupement amide substitué par un groupement alkyle en $C_1$-$C_4$ ainsi que leurs sels et leurs esters physiologiquement acceptables.

Dans la formule précitée, alkyle en $C_1$-$C_4$ désigne de préférence méthyle, éthyle, n-butyle, t-butyle; alkyle en $C_1$-$C_{16}$ désigne de préférence éthyle, propyle, palmityle; aryle désigne de préférence phényle ou benzyle, les substituants des groupements aryle sont de préférence des groupements alkyle en $C_1$-$C_4$, alcoxy en $C_1$-$C_{12}$, hydroxyle, halogène, nitro, les groupements alcoxy ou alkyle pouvant eux-mêmes éventuellement être substitués par un groupement OH.

Les groupements hétérocycliques peuvent être choisis entre autre parmi les groupements dérivés de la phtalimide, de la succinimide, des hétérocycles de 4 à 6 chaînons comportant un ou plusieurs atomes d'oxygène, et/ou un ou plusieurs atomes d'azote.

Les composés de la famille des rétinoïdes définis ci-dessus, sont en particulier choisis parmi :

le rétinal, le rétinol, l'acétate, le propionate, le palmitate de rétinyle, l'acide rétinoïque sous les formes tout-trans, 13-cis, 9-cis, 11-cis, 9,13-dicis, 11,13-dicis, les rétinoates de zinc correspondants, les rétinoates d'ammonium quaternaires de formule :

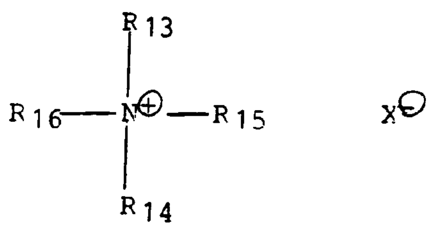

(VII)

dans laquelle $X^{\ominus}$ désigne un radical rétinoate tout-trans ou 13-cis ; et

(i) $R_{13}$, $R_{14}$, $R_{15}$, identiques ou différents, désignent un groupement alcoyle linéaire en $C_1$-$C_4$ pouvant porter dans la chaîne un ou plusieurs groupement(s) hydroxyle, $R_{16}$ désignant alcoyle ou alcényle linéaire en $C_{12}$-$C_{18}$ ;

(ii) $R_{15}$ désigne un groupement :

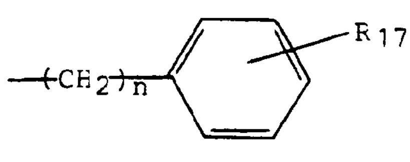

dans lequel :

n est égal à 0 ou 1,

$R_{17}$ représente un atome d'hydrogène ou halogène, un groupement hydroxyle, alcoyle ou hydroxyalkyle en $C_1$-$C_{18}$, ou acyle en $C_2$-$C_{18}$ ;

$R_{13}$, $R_{14}$ et $R_{16}$ ayant les significations indiquées sous (i) ;

(iii) $R_{13}$ et $R_{14}$ peuvent former un hétérocycle aliphatique comportant au moins un atome d'oxygène, un atome d'azote ou un atome de soufre ;

$R_{15}$ ou $R_{16}$ ayant les significations indiquées sous (i) et (ii).

D'autres composés entrant dans la définition des rétinoïdes, particulièrement utilisables conformément à l'invention, sont choisis parmi :

le tout-trans rétinoyloxyacétamide, le mélange de rétinoates tout-trans de 2-hydroxy 1-propyle et de 1-hydroxy 2-propyle, le 2-hydroxyéthyl tout-trans rétinoate, le 4-nitrobenzyl tout-trans rétinoate, le benzyl tout-trans rétinoate, le 4-(tout-trans rétinoyl oxyacétyl) catéchol, le 2-cyclohexyléthyl tout-trans rétinoate, le 10-carboxyméthyldécyl tout-trans rétinoate, le 4-hydroxybutyl tout-trans rétinoate, le cholestéryl tout-trans rétinoate, le 4-bromobenzyl tout-trans rétinoate, le cholestéryl tout-trans rétinoyloxy acétate, le tout-trans rétinoyloxyacétylbenzène, le 4-(tout-trans rétinoyloxyacétyl)-bromobenzène, le 4-(tout-trans rétinoyloxyacétyl)-nitrobenzène, le 4-(tout-trans rétinoyloxyacétyl)-benzonitrile, le tout-trans rétinoyloxyacétyl-2,4 dichlorobenzène, le N-(tout-trans rétinoyloxy)phtalimide, le N-(tout-trans rétinoyl oxy)succinimide, le 4-(tout-trans rétinoyloxyacétyl)méthoxybenzène, le 4-(tout-trans rétinoyloxyacétyl)phénol, le 4-(tout-trans rétinoyloxyacétyl)3, 4,5-triméthoxybenzène, le 4-(tout-trans rétinoyloxyacétyl) 2,4,6-triméthylbenzène, le 4-(tout-trans rétinoyloxy acétyl)toluène, le 4-(tout-trans rétinoyloxyacétyl) éthoxybenzène, le 4-(tout-trans rétinoyloxyacétyl) acétoxybenzène, le 4-(tout-trans rétinoyloxyacétyl) naphtalène, le 4-(tout-trans rétinoyloxyacétyl) biphényle, le 4-(tout-trans rétinoyloxyacétyl)-2,5-diméthoxybenzène, le 1-(tout-trans rétinoyloxyacétyl) 2,4-diméthylbenzène, le 1-(tout-trans rétinoyloxy acétyl)3,4-diacétoxybenzène, le tout-trans rétinamide, le 2-hydroxyéthyl tout-trans rétinamide, le N-éthyl tout-trans rétinamide, le 4-(tout-trans rétinoyl) aminophénol, le N-méthyldiméthyldioxolane rétinamide, le N-(orthocarboxyphényl) rétinamide, le N-(p-carboxy phényl) rétinamide, le N-hydroxypropyl tout-trans rétinamide, le N-(hydroxypropyl)13-cis rétinamide, le N-(5-tétrazolyl) tout-trans rétinamide, le N-(5-tétra zolyl)13-cis rétinamide, le N-(3,4-méthylène dioxy phénylméthyl) tout-trans rétinamide, le N-(n-propyl) tout-trans rétinamide, le N-tertiobutyl tout-trans rétinamide, le N-(1,1,3,3 tétraméthyl) butyl tout-trans rétinamide, le N-(4-carboxyméthyl 3-hydroxyphényl) tout-trans rétinamide, le N-[β-(3,4-diméthoxyphényl) éthyl] tout-trans rétinamide, le 2-(tout-trans rétinoyl amino) benzotriazole, le 1-(tout-trans rétinoyl)1,2,4 triazole, le N-(tout-trans rétinoyl)imidazole, la 1-nicotinoyl 2-(tout-trans rétinoyl) hydrazine, la N-(tout-trans rétinoyl) morpholine, la trans-β-ionone (tout-trans rétinoyl) hydrazone, la N,N'-dicyclohexyl N-(tout-trans rétinoyl) urée, l'acétone (tout-trans rétinoyl) hydrazone, la N-benzoylrétinylamine, l'azoture de rétinoyle.

Les groupements représentés par A et définis ci-dessus dans le paragraphe (b) en liaison avec les groupements aryle, aryle substitué, hétérocycle, hétérocyclique substitué, aryl-hétérocycle substitué sur l'hétérocycle ou l'aryl-homocycle substitué sur le noyau aromatique, sont en particulier choisis parmi les groupements :

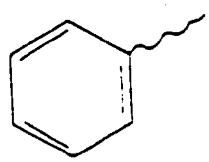
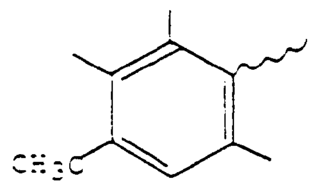
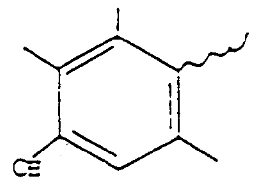

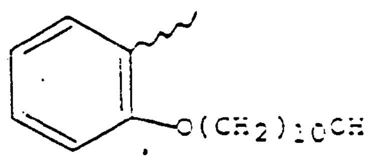
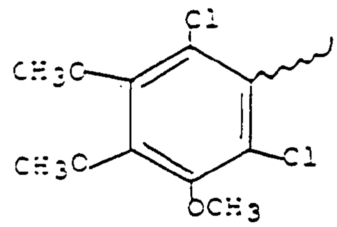

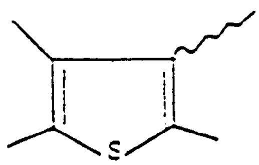
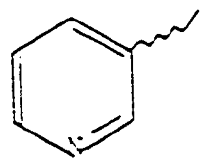
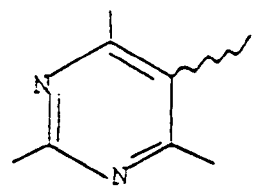

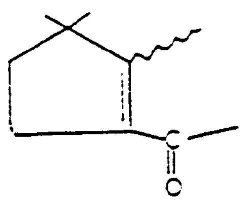
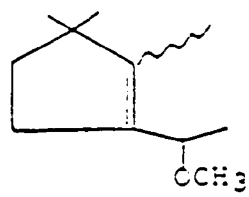

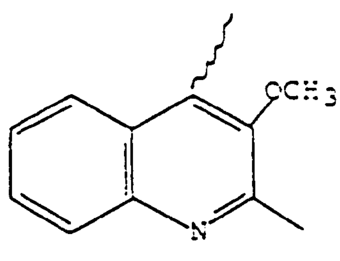
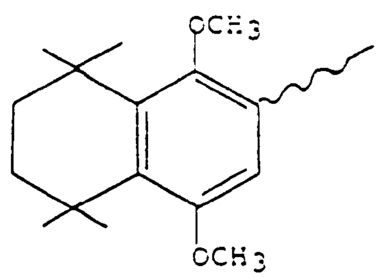

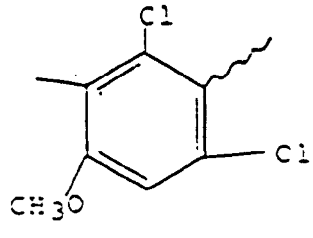

Le groupement R peut avoir les significations COOH, $CONHC_2H_5$, $COOC_2H_5$.

Les composés particulièrement préférés dans cette famille sont le motrétinide et l'étrétinate.

D'autres rétinoïdes utilisables conformément à l'invention répondent aux formules suivantes ou leurs sels

ou esters physiologiquement acceptables.

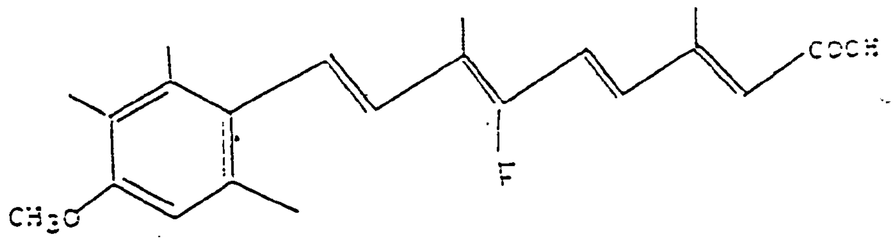

(VII)

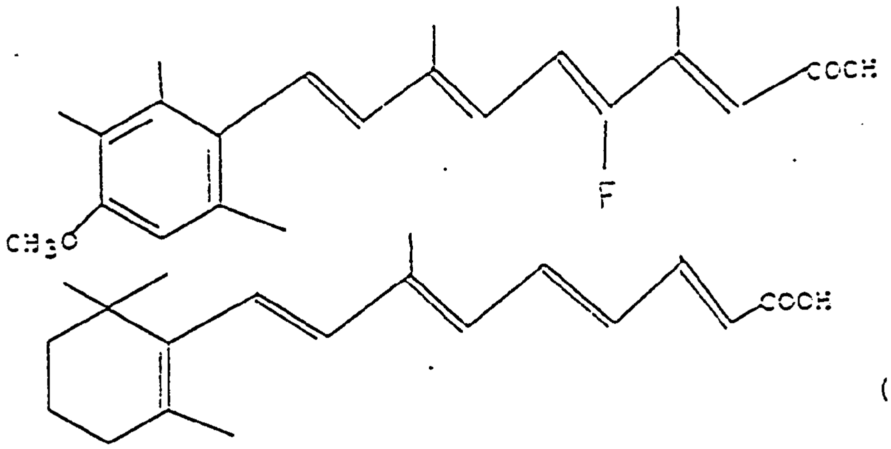

(VIII)

(IX)

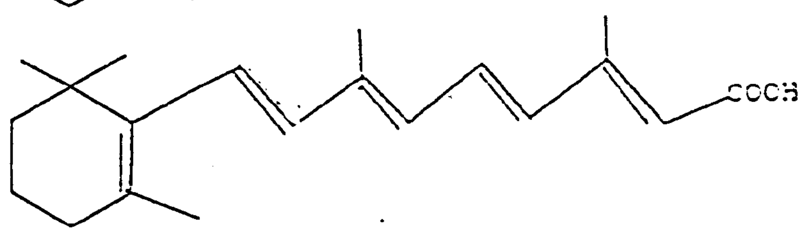

(X)

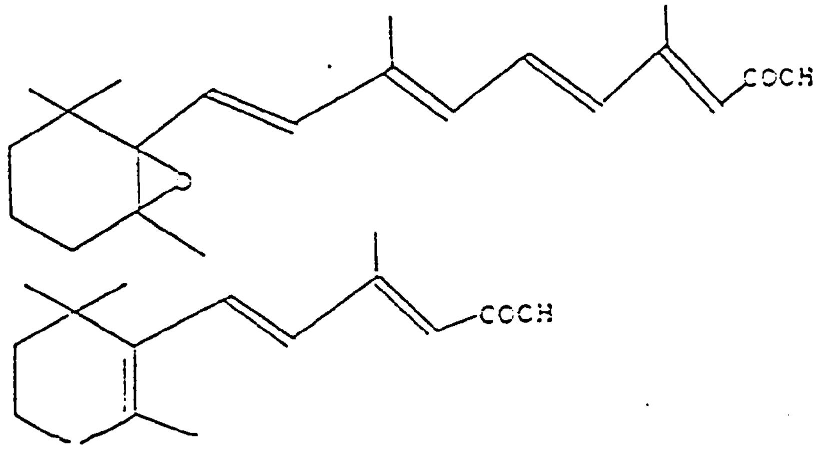

(XI)

(XII)

Les composés de la famille des rétinoïdes utilisables conformément à l'invention, sont en particulier décrits dans les brevets US-A-4.190.594, 4.126.698, EP-A-010.209, EP-A-010.208, EP-A-097.76, le brevet français 2.293.193 et l'EP-A-033.095 ou dans Cancer Research 40, 3413-3425, October 1980 ou dans Annals of the New York Academy of Sciences, Vol. 359.

Des rétinoïdes particulièrement préférés sont ceux répondant à la formule générale (II) indiquée ci-dessus, dans laquelle R désigne le radical

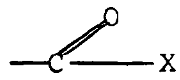

dans lequel X désigne OH, OY, Y désignant un groupement alkyle ayant de 1 à 15 atomes de carbone, X pouvant désigner également un groupement amino éventuellement mono- ou di-substitué par un groupement alkyle inférieur ayant de préférence 1 à 6 atomes de carbone, R pouvant également désigner un groupement $-CH_2OH$, -CHO, et A désignant un groupement :

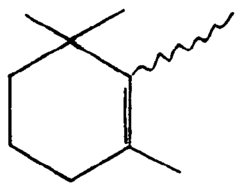

ou

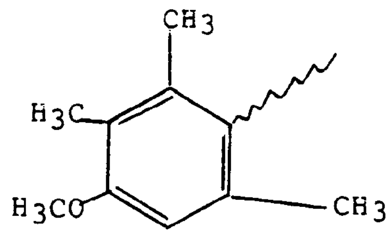

Ces composés étant de préférence sous la forme des isomères tout-trans ou 13-cis.

Parmi les dérivés particulièrement préférés, on peut citer les produits dénommés couramment : trétinoïne, isotrétinoïne, rétinol, motrétinide, étrétinate et les dérivés du rétinol, tels que l'acétate, le palmitate ou le propionate, le tout-trans rétinoate de zinc, et plus particulièrement encore la trétinoïne ou acide tout-trans rétinoïque.

Les rétinoïdes utilisés conformément à l'invention peuvent être présents dans la même composition que le dérivé de pyrimidine et l'agent filtrant solaire, et de préférence dans des proportions comprises entre 0,001 et 2% en poids et en particulier entre 0,01 et 0,5% en poids par rapport au poids total de la composition.

Une autre forme de l'invention peut consister dans une association comportant un composant (A) constitué par la composition à base du dérivé de pyrimidine de formule (I) et de l'agent filtrant solaire défini ci-dessus et un composant (B) contenant dans un milieu physiologiquement acceptable un rétinoïde.

Le milieu physiologiquement acceptable peut être du type de celui défini ci-dessus, en relation avec la composition contenant le dérivé de pyrimidine de formule (I) et l'agent filtrant solaire.

Le rétinoïde est présent dans le composant (B) dans les proportions indiquées ci-dessus, à savoir comprises de préférence entre 0,001 et 2% en poids et en particulier entre 0,01 et 0,5% en poids par rapport au poids total du composant (B).

Le traitement du cuir chevelu en vue d'induire et de stimuler la croissance des cheveux et diminuer leur chute, peut être effectué suivant un procédé consistant, selon une première variante, à appliquer, dans un premier temps, l'association conforme à l'invention constituée d'un composant (A), contenant, dans un milieu physiologiquement acceptable, le dérivé de pyrimidine de formule (I) et les agents filtrant les radiations UV définis ci-dessus.

Selon une seconde variante, on applique soit simultanément, soit successivement, ou même de façon décalée dans le temps, un composant (B) constitué par une composition contenant, dans un milieu physiologiquement acceptable, un rétinoïde tel que défini ci-dessus.

Selon une forme de réalisation particulièrement préférée de l'invention, on applique tout d'abord le composant (B) contenant le rétinoïde et après une durée de contact d'une minute à 12 heures, on applique le composant (A) contenant le dérivé de pyrimidine de formule (I) avec l'agent filtrant les radiations UV.

Il est également possible, selon une troisième variante particulièrement préférée, de procéder au mélange des composants (A) et (B) de façon extemporanée tout juste avant l'utilisation.

L'application s'effectue de préférence en utilisant des doses de 0,5 à 2 $cm^3$ de chacun des composants ou des deux composants réunis en un mélange extemporané.

L'association conforme à l'invention et plus particulièrement dans la variante mettant en oeuvre le composant (A) et le composant (B), peut être conditionnée sous forme d'un dispositif à plusieurs compartiments encore appelé "kit" ou nécessaire, dont le premier compartiment contient le composant (A) à base du dérivé de pyrimidine de formule (I) et l'agent filtrant les radiations UV tel que défini ci-dessus, et un composant (B) contenant dans un milieu physiologiquement acceptable un rétinoïde.

Un autre objet de l'invention est constitué par un procédé de solubilisation d'un dérivé de pyrimidine de formule (I), grâce à l'utilisation conjointe d'un agent filtrant les radiations UV tel que défini ci-dessus.

Le procédé conforme à l'invention vise essentiellement un traitement thérapeutique de la chute des cheveux en agissant en particulier sur les fonctions et le mécanisme biologique à l'origine de la croissance des cheveux et notamment par action sur ce mécanisme de croissance, en prolongeant la phase anagène.

Ce procédé conforme à l'invention peut également être appliqué dans le cadre d'un traitement cosmétique, dans la mesure où il permet d'améliorer l'esthétique des cheveux en leur donnant une plus grande vigueur et un aspect meilleur.

Les exemples suivants sont destinés à illustrer l'invention sans pour autant présenter un caractère limitatif.

EXEMPLE 1

On prépare la composition suivante :

| | | |
|---|---|---|
| - Minoxidil | | 3,40 g |
| - 2-hydroxy 4-méthoxy benzophénone (UVINUL M 40 vendu par la Société GAF) | | 3,00 g |
| - Propylèneglycol 6,5 g/alcool éthylique 93,5 g | qsp | 100,00 g |

La solubilité du Minoxidil est augmentée par rapport à sa solubilité dans le même milieu sans le filtre solaire.

EXEMPLE 2

On prépare la composition suivante :

| | | |
|---|---|---|
| - Minoxidil | | 3,30 g |
| - p-diméthylaminobenzoate de 2-éthyl hexyle (ESCALOL 507 vendu par la Société VAN DYK) | | 3,00 g |
| - Propylèneglycol 6,5 g/alcool éthylique 93,5 g | qsp | 100,00 g |

La solubilité du Minoxidil est augmentée par rapport à sa solubilité dans le même milieu sans le filtre solaire.

EXEMPLE 3

On prépare la composition suivante :

| | | |
|---|---|---|
| - Minoxidil | | 3,20 g |
| - p-méthoxycinnamate de 2-éthylhexyle (PARSOL MCX vendu par la Société GIVAUDAN) | | 3,00 g |
| - Propylèneglycol 6,5 g/alcool éthylique 93,5 g | qsp | 100,00 g |

La solubilité du Minoxidil est augmentée par rapport à sa solubilité dans le même milieu sans filtre solaire.

La solubilité du Minoxidil est multipliée par deux, par rapport à sa solubilité dans le même milieu sans filtre solaire.

EXEMPLE 4

On prépare la composition suivante :

| | | |
|---|---|---|
| - Minoxidil | | 3,25 g |
| - 4-(1,1-diméthyléthyl)4'-méthoxy dibenzoylméthane (PARSOL 1789 vendu par la Société GIVAUDAN) | | 3,00 g |
| - Propylèneglycol 6,5 g/alcool éthylique 93,5 g | qsp | 100,00 g |

La solubilité du Minoxidil est multipliée par deux, par rapport à sa solubilité dans le même milieu sans filtre solaire.

EXEMPLE 5

On prépare les composants (A) et (B) suivants que l'on conditionne en kit :

Composant (A)

| | | |
|---|---|---|
| - Composition de l'exemple 1 | | 100,00 g |

Composant (B)

| | | |
|---|---|---|
| - Acide tout-trans rétinoïque | | 0,078 g |
| - Butylhydroxytoluène | | 0,025 g |
| - Propylèneglycol 6,5 g/alcool éthylique 93,5 g | qsp | 100,00 g |

On applique un mélange (A + B) extemporané 60/40 en poids sur le cuir chevelu à raison de 1 $cm^3$ par application.

EXEMPLE 6

On prépare les composants (A) et (B) suivants que l'on conditionne en kit :

| | | |
|---|---|---|
| Composant (A) | | |
| - Composition de l'exemple 2 | | 100,00 g |
| Composant (B) | | |
| - Acide tout-trans rétinoïque | | 0,062 g |
| - Butylhydroxyanisole | | 0,020 g |
| - Propylèneglycol 6,5 g/alcool éthylique 93,5 g | qsp | 100,00 g |

On applique 1 $cm^3$ du mélange (A + B) extemporané et équipondéral sur le cuir chevelu à chaque application.

EXEMPLE 7

On prépare la composition suivante :

| | | |
|---|---|---|
| - Minoxidil | | 6,60 g |
| - p-méthoxycinnamate de 2-éthylhexyle (PARSOL MCX vendu par la Société GIVAUDAN) | | 3,00 g |
| - Alcool éthylique 75 g/Eau 25 g | qsp | 100,00 g |

La solubilité du Minoxidil est augmentée par rapport à sa solubilité dans le même milieu sans filtre solaire.

EXEMPLE 8

On prépare la composition suivante :

| | | |
|---|---|---|
| - Minoxidil | | 6,10 g |
| - (N-2-éthylhexyl)3-[(3'-méthoxy 4'-n-butoxy)benzylidène]10-camphosulfonamide | | 1,00 g |
| - Alcool éthylique 75 g/Eau 25 g | qsp | 100,00 g |

La solubilité du Minoxidil est augmentée par rapport à sa solubilité dans le même milieu sans filtre solaire.

EXEMPLE 9

On prépare la composition suivante :

| | | |
|---|---|---|
| - Minoxidil | | 6,20 g |
| - Salicylate d'homomenthyle | | 2,00 g |
| - Alcool éthylique 75 g/Eau 25 g | qsp | 100,00 g |

La solubilité du Minoxidil est augmentée par rapport à sa solubilité dans le même milieu sans filtre solaire.

EXEMPLE 10

On prépare la composition suivante :

| | | |
|---|---|---|
| - Minoxidil | | 6,10 g |
| - Salicylate de 2-éthylhexyle | | 2,00 g |
| - Alcool éthylique 75 g/Eau 25 g | qsp | 100,00 g |

La solubilité du Minoxidil est augmentée par rapport à sa solubilité dans le même milieu sans filtre solaire.

EXEMPLE 11

On prépare la composition suivante :

| | | |
|---|---|---|
| - Minoxidil | | 4,10 g |
| - Acide 2-hydroxy 4-méthoxy benzophénone 5-sulfonique | | 2,00 g |
| - Alcool éthylique 40,5 g/Eau 59,5 g | qsp | 100,00 g |

La solubilité du Minoxidil est augmentée par rapport à sa solubilité dans le même milieu sans filtre solaire.

Les compositions des exemples 8 à 11 sont appliquées sur les zones alopéciques du cuir chevelu présentant une surface de 100 à 200 $cm^2$ à raison de 2 ml par jour pendant 3 mois.

EXEMPLE 12

On prépare la composition suivante :

| | | |
|---|---|---|
| - Minoxidil | | 3,10 g |
| - Acide p-aminobenzoïque | | 2,30 g |
| - Alcool éthylique 40,5 g/Eau 59,5 | qsp | 100,00 g |

La solubilité du Minoxidil est augmentée par rapport à sa solubilité dans le même milieu sans filtre solaire.

EXEMPLE 13

On prépare la composition suivante :

| | | |
|---|---|---|
| - Minoxidil | | 6,30 g |
| - p-diméthylaminobenzoate de pentyle | | 3,00 g |
| - Alcool éthylique 75 g/Eau 25 g | qsp | 100,00 g |

La solubilité du Minoxidil est augmentée par rapport à sa solubilité dans le même milieu sans filtre solaire.

EXEMPLE 14

On prépare la composition suivante :

| | | |
|---|---|---|
| - Minoxidil | | 5,80 g |
| - 3-(4-méthylbenzylidène)camphre | | 3,00 g |
| - Alcool éthylique 75 g/Eau 25 g | qsp | 100,00 g |

La solubilité du Minoxidil est augmentée par rapport à sa solubilité dans le même milieu sans filtre solaire.

EXEMPLE 15

On prépare les composants (A) et (B) que l'on conditionne en kit :

Composant (A)

| | | |
|---|---|---|
| - Minoxidil | | 4,70 g |
| - (N-2-éthylhexyl)3-[(3'-méthoxy 4'-n-butoxy)benzylidène]10-camphosulfonamide | | 0,30 g |
| - Alcool éthylique 75 g/Eau 25 g | qsp | 100,00 g |

Composant (B)

| | | |
|---|---|---|
| - Acide tout-trans rétinoïque | | 0,031 g |
| - Butylhydroxyanisole | | 0,010 g |
| - Propylèneglycol 6,5 g/Alcool éthylique 93,5 g | qsp | 100,00 g |

Sur les zones alopéciques du cuir chevelu, on applique les deux compositions (A) et (B) séparément ou de façon décalée dans le temps, soit l'une après l'autre, (A) le matin, (B) le soir, ou vice versa, ou à un intervalle de temps de 5 minutes à quelques heures.

EXEMPLE 16

On prépare les composants (A) et (B) que l'on conditionne en kit :

Composant (A)

| | | | |
|---|---|---|---|
| - Minoxidil | | 4,70 | g |
| - Salicylate d'homomenthyle | | 0,50 | g |
| - Alcool éthylique 75 g/Eau 25 g | qsp | 100,00 | g |

Composant (B)

| | | | |
|---|---|---|---|
| - Acide tout-trans rétinoïque | | 0,031 | g |
| - Butylhydroxyanisole | | 0,010 | g |
| - Propylèneglycol 6,5 g/Alcool éthylique 93,5 g | qsp | 100,00 | g |

Sur une zone alopécique du cuir chevelu, on applique les deux compositions (A) et (B) séparément ou de façon décalée dans le temps, soit l'une après l'autre, (A) le matin, (B) le soir ou vice versa, ou à un intervalle de temps de 5 minutes à quelques heures.

EXEMPLE 17

On conditionnne en kit les deux compositions (A) et (B) suivantes :

Composition (A)

| | | | |
|---|---|---|---|
| - Minoxidil | | 3,00 | g |
| - Acide 2-hydroxy 4-méthoxy benzophénone 5-sulfonique | | 1,00 | g |
| - Alcool éthylique 40,5 g/Eau 59,5 g | qsp | 100,00 | g |

Composition (B)

| | | | |
|---|---|---|---|
| - Acide tout-trans rétinoïque | | 0,031 | g |
| - Butylhydroxytoluène | | 0,0125 | g |
| - Propylèneglycol 6,5 g/Alcool éthylique 93,5 g | qsp | 100,00 | g |

Sur une zone alopécique du cuir chevelu, on applique les deux compositions (A) et (B) séparément ou de façon décalée dans le temps, soit l'une après l'autre, (A) le matin, (B) le soir ou vice versa, ou à un intervalle de temps de 5 minutes à quelques heures.

Pour les différentes compositions des exemples 1 à 17, on ne constate pratiquement pas de cristallisation du Minoxidil sur le cuir chevelu.

## Revendications

**Revendications pour les Etats contractants suivants : DE, GB, FR, IT, NL, SE, CH, BE, AT.**

1. Composition destinée à induire et stimuler la croissance des cheveux et à diminuer leur chute, caractérisée par. le fait qu'elle renferme, dans un milieu physiologiquement acceptable, au moins un dérivé de pyrimidine répondant à la formule :

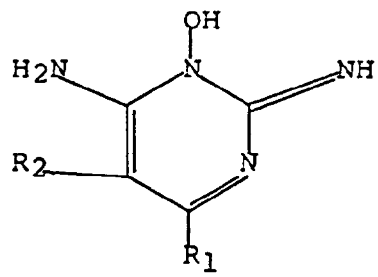

(I)

dans laquelle $R_1$ désigne un groupement

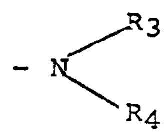

dans lequel $R_3$ et $R_4$ peuvent être choisis parmi l'hydrogène, un groupement alkyle, alcényle, alkylaryle ou cycloalkyle inférieur, $R_3$ et $R_4$ peuvent également former un hétérocycle avec l'atome d'azote auquel ils sont liés choisi parmi les groupements aziridinyle, azétidinyle, pyrrolidinyle, pipéridinyle, hexahydroazépinyle, heptaméthylèneimine, octaméthylène imine, morpholine et alkyle inférieur-4 pipérazinyle, les groupements hétérocycliques pouvant être substitués sur les atomes de carbone par un à trois groupements alkyle inférieur, hydroxy ou alcoxy; le groupement $R_2$ est choisi parmi l'hydrogène, un groupement alkyle, alcényle, alcoxyalkyle, cycloalkyle, aryle, alkylaryle, arylalkyle, alkylarylalkyle, alcoxyarylalkyle, ou haloarylalkyle inférieur, ainsi que les sels d'addition d'acides physiologiquement acceptables; et au moins un agent filtrant le rayonnement UV choisi parmi les composés suivants :
   - la 2-hydroxy 4-méthoxy benzophénone;
   - le para-diméthylaminobenzoate de 2-éthyl hexyle;
   - le para-diméthylaminobenzoate de pentyle;
   - le paraméthoxycinnamate de 2-éthylhexyle;
   - le 4-(1,1-diméthyléthyl) 4'-méthoxy dibenzoylméthane;
   - le (N-2-éthyl hexyl)3-[(3'-méthoxy 4'-n-butoxy) benzylidène]10-camphosulfonamide;
   - le 3-(4-méthylbenzylidène)camphre;
   - le salicylate d'homomenthyle;
   - le salicylate de 2-éthylhexyle;
   - l'acide para-aminobenzoïque non complexé avec l'allantoïne
   - l'acide 2-hydroxy 4-méthoxybenzophénone 5-sulfonique; ainsi que leurs mélanges.

2. Composition selon la revendication 1, caractérisée par le fait que le dérivé de pyrimidine de formule (I) est choisi parmi les composés de formule (I) dans laquelle $R_2$ désigne hydrogène et $R_1$ désigne un groupement :

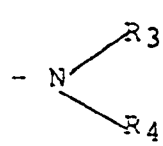

dans lequel $R_3$ et $R_4$ forment un cycle pipéridinyle, ainsi que leurs sels.

3. Composition selon la revendication 1 ou 2, caractérisée par le fait que le dérivé de pyrimidine de formule (I) est présent dans la composition dans des proportions comprises entre 0,1 et 10% en poids et plus particulièrement entre 1 et 5% en poids par rapport au poids total de la composition.

4. Composition selon l'une quelconque des revendications 1 à 3, caractérisée par le fait que l'agent filtrant le rayonnement UV est présent dans des proportions suffisantes pour augmenter la solubilité du dérivé de pyrimidine, dans le milieu physiologiquement acceptable, d'au moins 10% et de préférence d'au moins 20% par rapport à la solubilité du dérivé de pyrimidine de formule (I) dans ce même milieu.

5. Composition selon la revendication 4, caractérisée par le fait que les agents filtrant le rayonnement UV sont présents dans des proportions comprises entre 0,1 à 10% et de préférence entre 0,3 et 4% par rapport au poids total de la composition.

6. Composition selon l'une quelconque des revendications 1 à 5, caractérisée par le fait qu'elle se présente sous forme anhydre.

7. Composition selon la revendication 6, caractérisée par le fait qu'elle comporte un milieu solvant anhydre constitué d'un solvant ou d'un mélange de solvants choisi parmi les alcools inférieurs en $C_2$-$C_4$, les alkylèneglycols et les alkyléthers d'alkylèneglycols ou de dialkylèneglycols.

8. Composition selon l'une quelconque des revendications 1 à 5, caractérisée par le fait qu'elle se présente sous forme aqueuse ou hydroalcoolique.

9. Composition selon la revendication 8, caractérisée par le fait qu'elle comporte un milieu physiologiquement acceptable aqueux constitué d'eau et d'un solvant ou d'un mélange de solvants choisi parmi les alcools inférieurs, les alkylèneglycols et les alkyléthers d'alkylèneglycols ou de dialkylèneglycols.

10. Association destinée à induire et à stimuler la croissance des cheveux et diminuer leur chute, caractérisée par le fait qu'elle comprend :
   (a) un composant (A) constitué par une composition telle que définie dans l'une quelconque des revendications 1 à 9; et
   (b) un composant (B) contenant, dans un milieu physiologiquement acceptable, au moins un rétinoïde.

11. Association selon la revendication 10, caractérisée par le fait que le rétinoïde est choisi parmi les composés de formule :

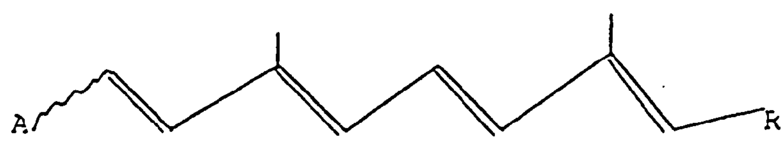

(II)

dans laquelle :
   (a) A est un groupement choisi parmi les groupements de formules :

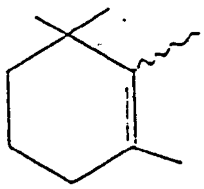

(IIIa)

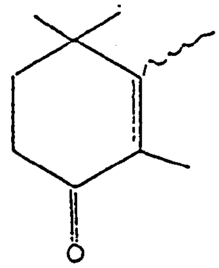

(IV)

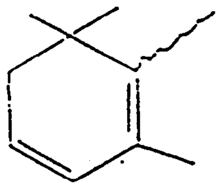

(IIIb)

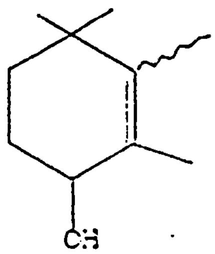

(V)

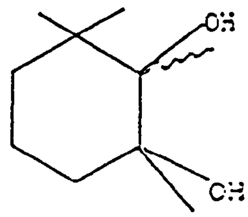

(VI)

– lorsque A désigne un groupement de formule (IIIa), R est choisi parmi les groupements suivants : CHO ; $CH_2OR_5$,
dans lequel $R_5$ désigne hydrogène, alkyle inférieur en $C_1$-$C_4$;
le groupement

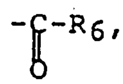

où $R_6$ désigne alkyle linéaire ou ramifié en $C_1$-$C_{16}$;
$CH_2SR_7$, dans lequel $R_7$ désigne hydrogène ou méthyle;

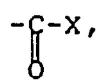

dans lequel X désigne :
(i) OH;
(ii) $OR_8$ où $R_5$ désigne un radical alkyle en $C_1$-$C_{15}$, arylalkyle en $C_1$-$C_4$, éventuellement substitué sur le groupement aryle, arylcarboxyalkyle en $C_1$-$C_4$, éventuellement substitué sur le groupement aryle, hydroxyalkyle en $C_1$-$C_4$, amidoalkyle en $C_1$-$C_4$;
(iii) $NR_9R_{10}$, dans lequel $R_9$ et $R_{10}$, identiques ou différents, désignent hydrogène, alkyle en $C_1$-$C_6$, hydroxyalkyle en $C_1$-$C_4$, aryle éventuellement substitué;
$R_9$ ou $R_{10}$ pouvant représenter un hétérocycle éventuellement substitué ou pouvant former conjointement avec l'atome d'azote auquel ils sont reliés, un hétérocycle, lui-même éventuellement substitué;
(iv) le groupement $N_3$;
ou bien un groupement de formule $CH_2NHR_{11}$, dans lequel $R_{11}$ désigne un radical benzoyle éventuellement substitué;
– lorsque A désigne un groupement de formule (IIIb), (IV), (V) ou (VI), R désigne COOH ainsi que sa forme salifiée ou estérifiée;
(b) A est un groupement choisi parmi les groupements aryle ou aryle substitué, un hétérocycle ou un

hétérocycle substitué, un groupement arylhétérocycle éventuellement substitué sur l'hétérocycle ou arylhomocyclique éventuellement substitué sur le noyau aromatique, R désignant dans ce cas un groupement COOH, $COOR_{12}$ où $R_{12}$ désigne un radical alkyle en $C_1$-$C_4$ ou bien un groupement amide substitué par un groupement alkyle en $C_1$-$C_4$, ainsi que leurs sels et leurs esters physiologiquement acceptables.

12. Association selon l'une quelconque des revendications 10 et 11, caractérisée par le fait que le rétinoïde est choisi parmi le rétinal, le rétinol, l'acétate, le propionate, le palmitate de rétinyle, l'acide rétinoïque sous les formes tout-trans, 13-cis, 9-cis, 11-cis, 9,13-dicis, 11,13-dicis, les rétinoates de zinc correspondants, les rétinoates d'ammonium quaternaire de formule :

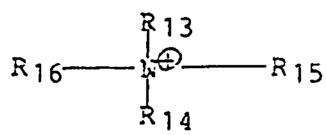 $X^{\ominus}$ (VII)

dans laquelle $X^{\ominus}$ désigne un radical rétinoate tout-trans ou 13-cis; et

(i) $R_{13}$, $R_{14}$, $R_{15}$, identiques ou différents désignent un groupement alcoyle linéaire en $C_1$-$C_4$ pouvant porter dans la chaîne un ou plusieurs groupement(s) hydroxyle,

$R_{16}$ désignant alcoyle ou alcényle linéaire en $C_{12}$-$C_{18}$;

(ii) $R_{15}$ désigne un groupement :

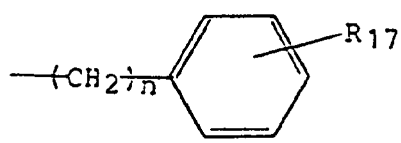

dans lequel :

n est égal à 0 ou 1,

$R_{17}$ représente un atome d'hydrogène, ou halogène, un groupement hydroxyle, alcoyle ou hydroxyalcoyle en $C_1$-$C_{18}$, ou acyle en $C_2$-$C_{18}$;

$R_{13}$, $R_{14}$ et $R_{16}$ ayant les significations indiquées sous (i);

(iii) $R_{13}$ et $R_{14}$ peuvent former un hétérocycle aliphatique comportant au moins un atome d'oxygène, un atome d'azote ou un atome de soufre;

$R_{15}$ ou $R_{16}$ ayant les significations indiquées sous (i) et (ii);

le tout-trans rétinoyloxyacétamide, le mélange des rétinoates tout-trans de 2-hydroxy 1-propyle et de 1-hydroxy 2-propyle, le 2-hydroxyéthyl tout-trans rétinoate, le 4-nitrobenzyl tout-trans rétinoate, le benzyl tout-trans rétinoate, le 4-(tout-trans rétinoyloxyacétyl)catéchol, le 2-cyclohexyléthyl tout-trans rétinoate, le 10-carboxyméthyldécyl tout-trans rétinoate, le 4-hydroxybutyl tout-trans rétinoate, le cholestéryl tout-trans rétinoate, le 4-bromobenzyl tout-trans rétinoate, le cholestéryl tout-trans rétinoyloxyacétate, le tout-trans rétinoyloxyacétyl benzène, le 4-(tout-trans rétinoyloxyacétyl)-bromo benzène, le 4-(tout-trans rétinoyloxyacétyl)-nitrobenzène, le 4-(tout-trans rétinoyloxyacétyl)benzonitrile, le tout-trans rétinoyloxyacétyl-2,4 dichlorobenzène, le N-(tout-trans rétinoyloxy)phtalimide, le N-(tout-trans rétinoyloxy)succinimide, le 4-(tout-trans rétinoyloxyacétyl)-méthoxybenzène, le 4-(tout-trans rétinoyloxyacétyl)-phénol, le 4-(tout-trans rétinoyloxyacétyl)3,4,5-triméthoxybenzène, le 4-(tout-trans rétinoyloxyacétyl)2,4,6-triméthylbenzène, le 4-(tout-trans rétinoyloxyacétyl)toluène, le 4-(tout-trans rétinoyloxyacétyl)éthoxybenzène, le 4-(tout-trans rétinoyloxyacétyl)acétoxybenzène, le 4-(tout-trans rétinoyloxyacétyl)naphtalène, le 4-(tout-trans rétinoyloxyacétyl)biphényle, le 4-tout-trans rétinoyloxy acétyl)-2,5-diméthoxybenzène, le 1-(tout-trans rétinoyloxyacétyl)2,4-diméthylbenzène, le 1-(tout-trans rétinoyloxyacétyl)3,4-diacétoxybenzène, le tout-trans rétinamide, le 2-hydroxyéthyl tout-trans rétinamide, le N-éthyl tout-trans rétinamide, le 4-(tout-trans rétinoyl) aminophénol, le N-méthyldiméthyldioxolane rétinamide, le N-(orthocarboxyphényl)rétinamide, le N-(p-carboxyphényl)rétinamide, le N-hydroxypropyl tout-trans rétinamide, le N-(hydroxypropyl)13-cis rétinamide, le N-(5-tétrazolyl)tout-trans rétinamide, le N-(5-tétrazolyl)13-cis rétinamide, le N-(3,4 méthylène dioxyphénylméthyl) tout-trans rétinamide, le N-(n-propyl) tout-trans rétinamide, le N-tertiobutyl tout-trans rétinamide, le N-(1,1,3,3 tétraméthyl) butyl tout-trans rétinamide, le N-(4-carboxyméthyl 3-hydroxyphényl) tout-trans rétinamide, le N-[β-(3,4 diméthoxyphényl) éthyl]tout-trans rétinamide, le 2-(tout-trans rétinoylamino) benzotriazole, le 1-(tout-trans rétinoyl) 1,2,4 triazole, le N-(tout-trans rétinoyl)imidazole, la 1-nicotinoyl 2-(tout-trans rétinoyl) hydrazine, la N-(tout-trans rétinoyl) morpholine, la trans-β-ionone (tout-trans rétinoyl) hydrazone, la N,N'-dicyclohexyl N-(tout-trans rétinoyl) urée, l'acétone (tout-trans rétinoyl) hydrazone, la N-benzoylrétinylamine, l'azoture de rétinoyle.

**13.** Association selon la revendication 10, caractérisée par le fait que les rétinoïdes sont choisis parmi les composés de formule (II), dans laquelle :

A désigne l'un quelconque des groupements suivants :

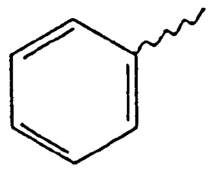

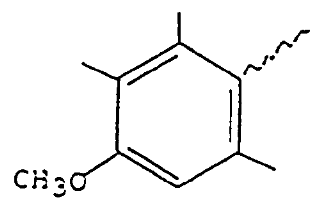

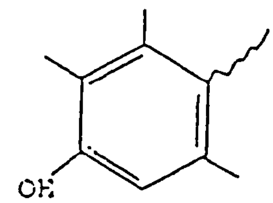

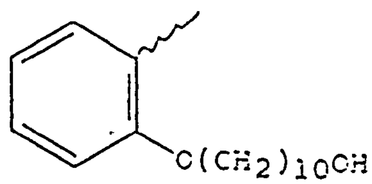

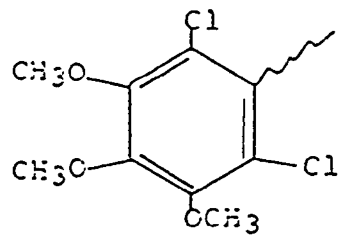

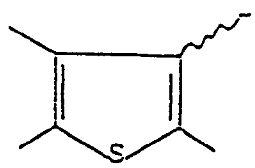

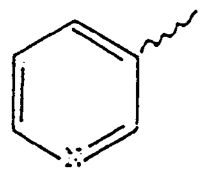

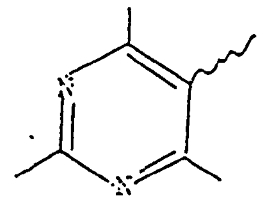

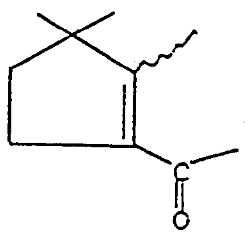

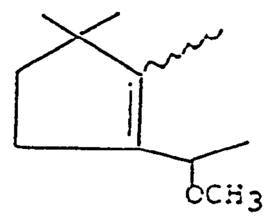

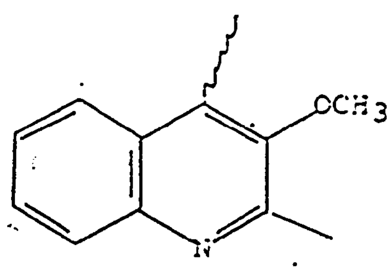

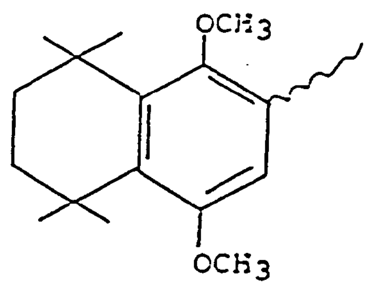

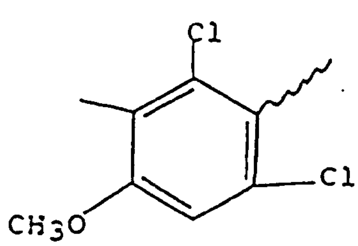

et R désigne COOH, $CONHC_2H_5$ ou $COOC_2H_5$.

**14.** Association selon la revendication 10, caractérisée par le fait que le rétinoïde est choisi parmi les compo-

sés de formules :

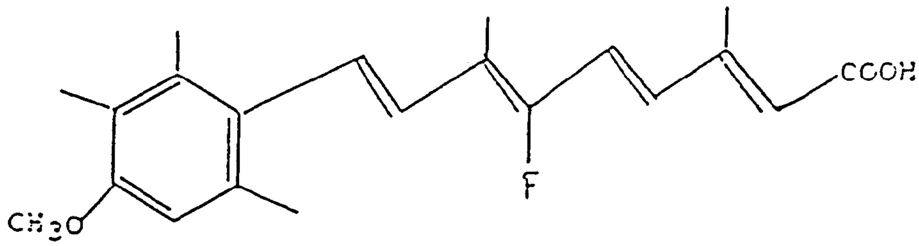

(VII)

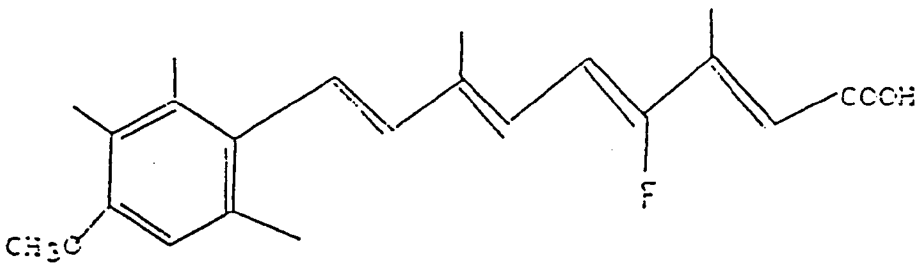

(VIII)

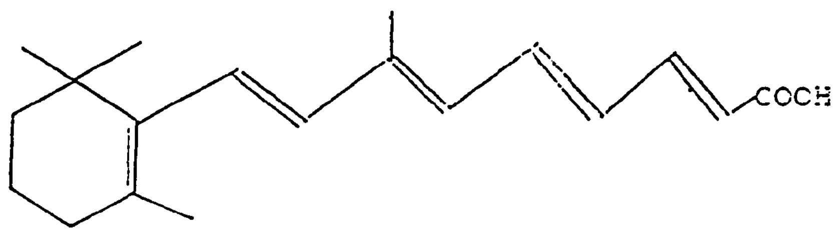

(IX)

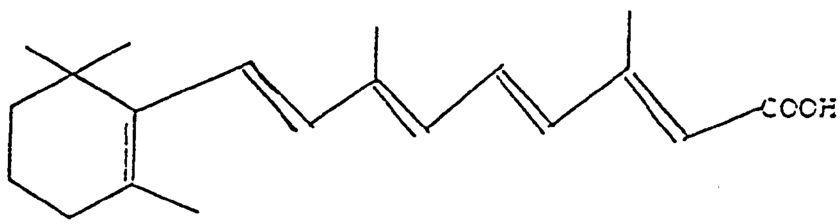

(X)

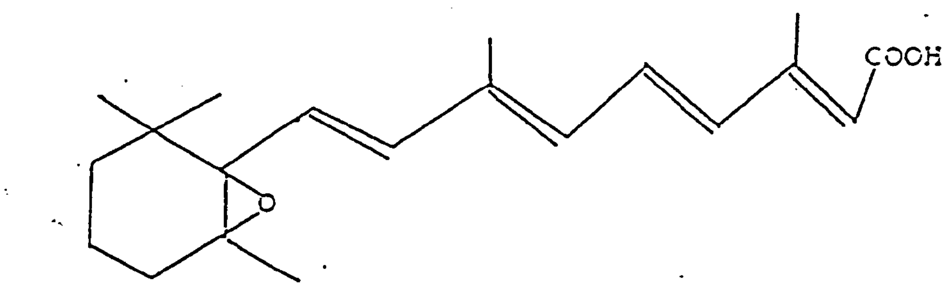

(XI)

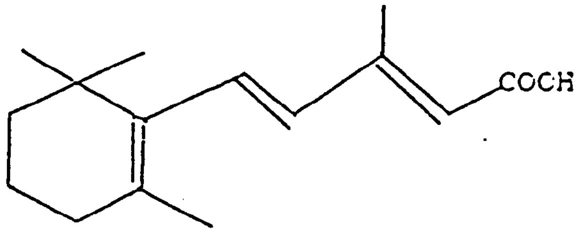

(XII)

ainsi que leurs sels ou esters physiologiquement acceptables.

**15.** Association selon l'une quelconque des revendications 10 à 14, caractérisée par le fait que le rétinoïde est choisi parmi les composés de formule (II) :

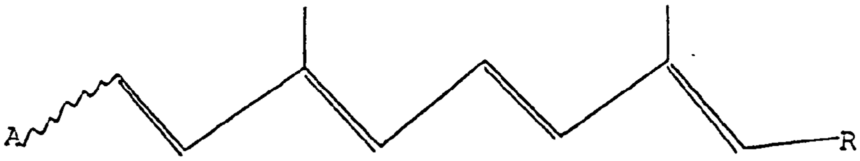

(II)

sous la forme all-trans ou 13-cis, dans laquelle R désigne un groupement

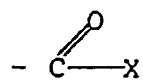

où X peut désigner un groupement OH, OY, Y désignant un groupement alkyle ayant 1 à 15 atomes de carbone, X pouvant également désigner un groupement amino, éventuellement mono- ou disubstitué par un groupement alkyle inférieur ayant 1 à 6 atomes de carbone, R désignant également un groupement $-CH_2OH$, -CHO, et A désignant un groupement :

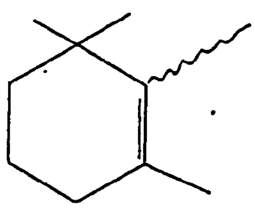

ou

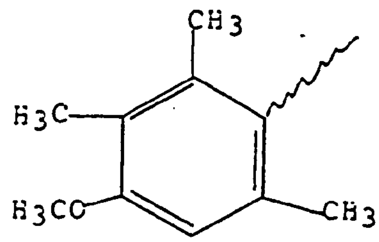

ainsi que les sels pharmaceutiquement ou cosmétiquement acceptables.

**16.** Association selon l'une quelconque des revendications 10 à 13, caractérisée par le fait que le rétinoïde est choisi parmi la trétinoïne, l'isotrétinoïne, le rétinol ou vitamine A et ses dérivés tels que l'acétate, le palmitate ou le propionate, le motrétinide, l'étrétinate, le tout-trans rétinoate de zinc.

**17.** Association selon l'une quelconque des revendications 10 à 16, caractérisée par le fait que les composants (A) et (B) font partie d'une même composition.

**18.** Association selon l'une quelconque des revendications 10 à 16, caractérisée par le fait que les composants (A) et (B) sont destinés à être mélangés de façon extemporanée tout juste avant l'emploi.

**19.** Association selon l'une quelconque des revendications 10 à 16, caractérisée par le fait que les composants (A) et (B) sont destinés à être appliqués de façon séparée ou décalée dans le temps.

**20.** Association selon l'une quelconque des revendications 10 à 19, caractérisée par le fait que le composant (B) contient le rétinoïde dans des proportions comprises entre 0,001 et 2% en poids par rapport au poids total de la composition.

**21.** Dispositif à plusieurs compartiments, caractérisé par le fait qu'il comporte au moins deux compartiments dont l'un contient le composant (A) et l'autre le composant (B) tels que définis dans l'une quelconque des revendications 10 à 20.

**22.** Composition selon l'une quelconque des revendications 1 à 9, pour son application dans le traitement thérapeutique de l'alopécie.

**23.** Association selon l'une quelconque des revendications 10 à 20, pour son utilisation dans le traitement thérapeutique de l'alopécie.

**24.** Procédé de traitement cosmétique des cheveux, caractérisé par le fait que l'on applique sur le cuir chevelu au moins une composition ou une association telle que définie dans l'une quelconque des revendications 1 à 20.

**25.** Procédé de traitement selon la revendication 24, caractérisé par le fait que l'on applique dans un premier temps le composant (B) contenant le rétinoïde et qu'après une durée de contact d'une minute à 12 heures, on applique le composant (A) contenant le dérivé de pyrimidine de formule (I) et l'agent filtrant les rayonnements UV.

**26.** Procédé de traitement cosmétique du cuir chevelu, caractérisé par le fait qu'on applique sur le cuir chevelu une composition résultant du mélange des composants (A) et (B) de l'association définie dans l'une quelconque des revendications 10 à 20.

**27.** Procédé de solubilisation d'un dérivé de pyrimidine répondant à la formule (I) définie dans la revendication 1, dans un milieu physiologiquement acceptable, caractérisé par le fait que l'on ajoute au milieu physiologiquement acceptable au moins un filtre solaire choisi parmi la 2-hydroxy 4-méthoxybenzophénone, le para-diméthylaminobenzoate de 2-éthyl hexyle, le para-diméthylaminobenzoate de pentyle, le paraméthoxycinnamate de 2-éthylhexyle, le 4-(1,1-diméthyléthyl) 4'-méthoxy dibenzoylméthane, le (N-2-éthyl hexyl)3-[(3'-méthoxy 4'-n-butoxy)benzylidène] 10-camphosulfonamide, le 3-(4-méthylbenzylidène)camphre, les salicylates d'homomenthyle et de 2-éthylhexyle, l'acide para-aminobenzoïque, l'acide 2-hydroxy 4-méthoxy benzophénone 5-sulfonique.

**Revendications pour l'Etat contractant suivant : ES**

**1.** Procédé de préparation d'une composition destinée à induire et stimuler la croissance des cheveux et à diminuer leur chute, caractérisé par le fait qu'elle est obtenue en introduisant dans un milieu physiologiquement acceptable, au moins un dérivé de pyrimidine répondant à la formule :

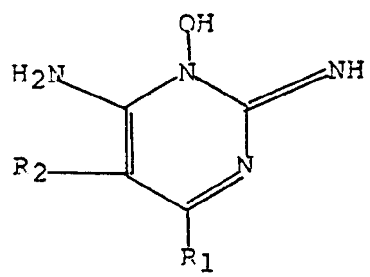

(I)

dans laquelle $R_1$ désigne un groupement

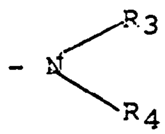

dans lequel $R_3$ et $R_4$ peuvent être choisis parmi l'hydrogène, un groupement alkyle, alcényle, alkylaryle ou cycloalkyle inférieur, $R_3$ et $R_4$ peuvent également former un hétérocycle avec l'atome d'azote auquel ils sont liés choisi parmi les groupements aziridinyle, azétidinyle, pyrrolidinyle, pipéridinyle, hexahydroazépinyle, heptaméthylèneimine, octaméthylène imine, morpholine et alkyle inférieur-4 pipérazinyle, les groupements hétérocycliques pouvant être substitués sur les atomes de carbone par un à trois groupements alkyle inférieur, hydroxy ou alcoxy; le groupement $R_2$ est choisi parmi l'hydrogène, un groupement alkyle, alcényle, alcoxyalkyle, cycloalkyle, aryle, alkylaryle, arylalkyle, alkylarylalkyle, alcoxyarylalkyle, ou haloarylalkyle inférieur, ainsi que les sels d'addition d'acides physiologiquement acceptables;
et au moins un agent filtrant le rayonnement UV choisi parmi les composés suivants :

- la 2-hydroxy 4-méthoxy benzophénone;
- le para-diméthylaminobenzoate de 2-éthyl hexyle;
- le para-diméthylaminobenzoate de pentyle;

- le paraméthoxycinnamate de 2-éthylhexyle;
- le 4-(1,1-diméthyléthyl) 4′-méthoxy dibenzoylméthane;
- le (N-2-éthyl hexyl)3-[(3′-méthoxy 4′-n-butoxy) benzylidène]10-camphosulfonamide;
- le 3-(4-méthylbenzylidène)camphre;
- le salicylate d'homomenthyle;
- le salicylate de 2-éthylhexyle;
- l'acide para-aminobenzoïque non complexé avec l'allantoïne
- l'acide 2-hydroxy 4-méthoxybenzophénone 5-sulfonique;

ainsi que leurs mélanges.

2. Composition destinée à induire et stimuler la croissance des cheveux et à diminuer leur chute, caractérisée par le fait qu'elle renferme, dans un milieu physiologiquement acceptable, au moins un dérivé de pyrimidine répondant à la formule :

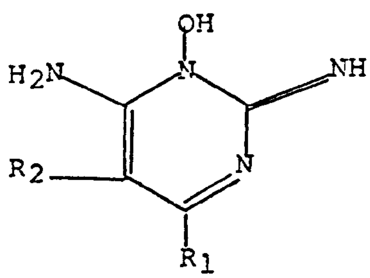

(I)

dans laquelle $R_1$ désigne un groupement

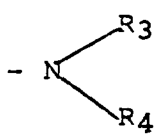

dans lequel $R_3$ et $R_4$ peuvent être choisis parmi l'hydrogène, un groupement alkyle, alcényle, alkylaryle ou cycloalkyle inférieur, $R_3$ et $R_4$ peuvent également former un hétérocycle avec l'atome d'azote auquel ils sont liés choisi parmi les groupements aziridinyle, azétidinyle, pyrrolidinyle, pipéridinyle, hexahydroazépinyle, heptaméthylèneimine, octaméthylène imine, morpholine et alkyle inférieur-4 pipérazinyle, les groupements hétérocycliques pouvant être substitués sur les atomes de carbone par un à trois groupements alkyle inférieur, hydroxy ou alcoxy; le groupement $R_2$ est choisi parmi l'hydrogène, un groupement alkyle, alcényle, alcoxyalkyle, cycloalkyle, aryle, alkylaryle, arylalkyle, alkylarylalkyle, alcoxyarylalkyle, ou haloarylalkyle inférieur, ainsi que les sels d'addition d'acides physiologiquement acceptables;
et au moins un agent filtrant le rayonnement UV choisi parmi les composés suivants :

- la 2-hydroxy 4-méthoxy benzophénone;
- le para-diméthylaminobenzoate de 2-éthyl hexyle;
- le para-diméthylaminobenzoate de pentyle;
- le paraméthoxycinnamate de 2-éthylhexyle;
- le 4-(1,1-diméthyléthyl) 4′-méthoxy dibenzoylméthane;
- le (N-2-éthyl hexyl)3- [(3′-méthoxy 4′-n-butoxy) benzylidène]10-camphosulfonamide;
- le 3-(4-méthylbenzylidène)camphre;
- le salicylate d'homomenthyle;
- le salicylate de 2-éthylhexyle;
- l'acide para-aminobenzoïque non complexé avec l'allantoïne
- l'acide 2-hydroxy 4-méthoxybenzophénone 5-sulfonique;

ainsi que leurs mélanges.

3. Composition selon la revendication 2, caractérisée par le fait que le dérivé de pyrimidine de formule (I) est choisi parmi les composés de formule (I) dans laquelle $R_2$ désigne hydrogène et $R_1$ désigne un groupement :

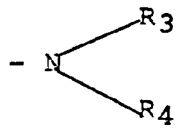

dans lequel $R_3$ et $R_4$ forment un cycle pipéridinyle, ainsi que leurs sels.

4. Composition selon la revendication 2 ou 3, caractérisée par le fait que le dérivé de pyrimidine de formule (I) est présent dans la composition dans des proportions comprises entre 0,1 et 10% en poids et plus particulièrement entre 1 et 5% en poids par rapport au poids total de la composition.

5. Composition selon l'une quelconque des revendications 2 à 4, caractérisée par le fait que l'agent filtrant le rayonnement UV est présent dans des proportions suffisantes pour augmenter la solubilité du dérivé de pyrimidine, dans le milieu physiologiquement acceptable, d'au moins 10% et de préférence d'au moins 20% par rapport à la solubilité du dérivé de pyrimidine de formule (I) dans ce même milieu.

6. Composition selon la revendication 5, caractérisée par le fait que les agents filtrant le rayonnement UV sont présents dans des proportions comprises entre 0,1 et 10% et de préférence entre 0,3 et 4% par rapport au poids total de la composition.

7. Composition selon l'une quelconque des revendications 2 à 6, caractérisée par le fait qu'elle se présente sous forme anhydre.

8. Composition selon la revendication 7, caractérisée par le fait qu'elle comporte un milieu solvant anhydre constitué d'un solvant ou d'un mélange de solvants choisi parmi les alcools inférieurs en $C_2$-$C_4$, les alkylèneglycols et les alkyléthers d'alkylèneglycols ou de dialkylèneglycols.

9. Composition selon l'une quelconque des revendications 2 à 6, caractérisée par le fait qu'elle se présente sous forme aqueuse ou hydroalcoolique.

10. Composition selon la revendication 9, caractérisée par le fait qu'elle comporte un milieu physiologiquement acceptable aqueux constitué d'eau et d'un solvant ou d'un mélange de solvants choisi parmi les alcools inférieurs, les alkylèneglycols et les alkyléthers d'alkylèneglycols ou de dialkylèneglycols.

11. Procédé de préparation d'un agent destiné à induire et stimuler la croissance des cheveux et diminuer leur chute, caractérisé par le fait qu'il est obtenu en associant
    (a) un composant (A) constitué par une composition telle que définie dans l'une quelconque des revendications 2 à 10, avec
    (b) un composant (B) contenant, dans un milieu physiologiquement acceptable, au moins un rétinoïde.

12. Association destinée à induire et à stimuler la croissance des cheveux et diminuer leur chute, caractérisée par le fait qu'elle comprend :
    (a) un composant (A) constitué par une composition telle que définie dans l'une quelconque des revendications 2 à 10; et
    (b) un composant (B) contenant, dans un milieu physiologiquement acceptable, au moins un rétinoïde.

13. Association selon la revendication 12, caractérisée par le fait que le rétinoïde est choisi parmi les composés de formule :

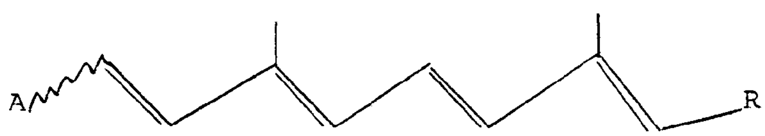 (II)

dans laquelle :
    (a) A est un groupement choisi parmi les groupements de formules :

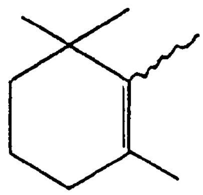

(IIIa)

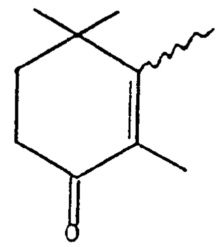

(IV)

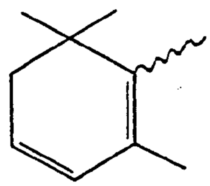

(IIIb)

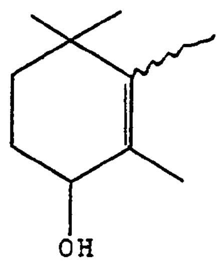

(V)

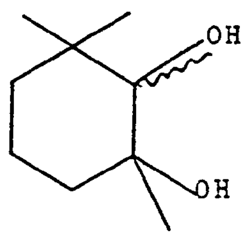

(VI)

– lorsque A désigne un groupement de formule (IIIa), R est choisi parmi les groupements suivants : CHO ; $CH_2OR_5$,
dans lequel $R_5$ désigne hydrogène, alkyle inférieur en $C_1$-$C_4$;
le groupement

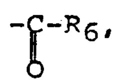

où $R_6$ désigne alkyle linéaire ou ramifié en $C_1$-$C_{16}$;
$CH_2SR_7$, dans lequel $R_7$ désigne hydrogène ou méthyle;

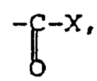

dans lequel X désigne :
(i) OH;
(ii) $OR_8$ où $R_5$ désigne un radical alkyle en $C_1$-$C_{15}$, arylalkyle en $C_1$-$C_4$, éventuellement substitué sur le groupement aryle, arylcarboxyalkyle en $C_1$-$C_4$, éventuellement substitué sur le groupement aryle, hydroxyalkyle en $C_1$-$C_4$, amidoalkyle en $C_1$-$C_4$;
(iii) $NR_9R_{10}$, dans lequel $R_9$ et $R_{10}$, identiques ou différents, désignent hydrogène, alkyle en $C_1$-$C_6$, hydroxyalkyle en $C_1$-$C_4$, aryle éventuellement substitué;

$R_9$ ou $R_{10}$ pouvant représenter un hétérocycle éventuellement substitué ou pouvant former conjointement avec l'atome d'azote auquel ils sont reliés, un hétérocycle, lui-même éventuellement substitué;
(iv) le groupement $N_3$;

ou bien un groupement de formule $CH_2NHR_{11}$, dans lequel $R_{11}$ désigne un radical benzoyle éventuellement substitué;

– lorsque A désigne un groupement de formule (IIIb), (IV), (V) ou (VI), R désigne COOH ainsi que sa forme salifiée ou estérifiée;

(b) A est un groupement choisi parmi les groupements aryle ou aryle substitué, un hétérocycle ou un hétérocycle substitué, un groupement arylhétérocycle éventuellement substitué sur l'hétérocycle ou arylhomocyclique éventuellement substitué sur le noyau aromatique, R désignant dans ce cas un groupement COOH, $COOR_{12}$ où $R_{12}$ désigne un radical alkyle en $C_1$-$C_4$ ou bien un groupement amide substitué par un groupement alkyle en $C_1$-$C_4$, ainsi que leurs sels et leurs esters physiologiquement acceptables.

**14.** Association selon l'une quelconque des revendications 12 et 13, caractérisée par le fait que le rétinoïde est choisi parmi le rétinal, le rétinol, l'acétate, le propionate, le palmitate de rétinyle, l'acide rétinoïque sous les formes tout-trans, 13-cis, 9-cis, 11-cis, 9,13-dicis, 11,13-dicis, les rétinoates de zinc correspondants, les rétinoates d'ammonium quaternaire de formule :

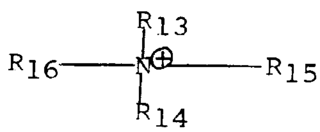 $X^{\ominus}$ (VII)

dans laquelle $X^{\ominus}$ désigne un radical rétinoate tout-trans ou 13-cis; et

(i) $R_{13}$, $R_{14}$, $R_{15}$, identiques ou différents, désignent un groupement alcoyle linéaire en $C_1$-$C_4$ pouvant porter dans la chaîne un ou plusieurs groupement(s) hydroxyle,

$R_{16}$ désignant alcoyle ou alcényle linéaire en $C_{12}$-$C_{18}$;

(ii) $R_{15}$ désigne un groupement :

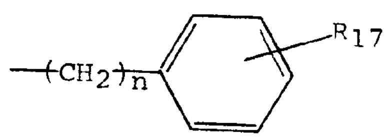

dans lequel :

n est égal à 0 ou 1,

$R_{17}$ représente un atome d'hydrogène, ou halogène, un groupement hydroxyle, alcoyle ou hydroxyalcoyle en $C_1$-$C_{18}$, ou acyle en $C_2$-$C_{18}$;

$R_{13}$, $R_{14}$ et $R_{16}$ ayant les significations indiquées sous (i);

(iii) $R_{13}$ et $R_{14}$ peuvent former un hétérocycle aliphatique comportant au moints un atome d'oxygène, un atome d'azote ou un atome de soufre;

$R_{15}$ ou $R_{16}$ ayant les significations indiquées sous (i) et (ii);

le tout-trans rétinoyloxyacétamide, le mélange des rétinoates tout-trans de 2-hydroxy 1-propyle et de 1-hydroxy 2-propyle, le 2-hydroxyéthyl tout-trans rétinoate, le 4-nitrobenzyl tout-trans rétinoate, le benzyl tout-trans rétinoate, le 4-(tout-trans rétinoyloxyacétyl)catéchol, le 2-cyclohexyléthyl tout-trans rétinoate, le 10-carboxyméthyldécyl tout-trans rétinoate, le 4-hydroxybutyl tout-trans rétinoate, le cholestéryl tout-trans rétinoate, le 4-bromobenzyl tout-trans rétinoate, le cholestéryl tout-trans rétinoyloxyacétate, le tout-trans rétinoyloxyacétyl benzène, le 4-(tout-trans rétinoyloxyacétyl)-bromo benzène, le 4-(tout-trans rétinoyloxyacétyl)-nitrobenzène, le 4-(tout-trans rétinoyloxyacétyl)benzonitrile, le tout-trans rétinoyloxyacétyl-2,4 dichlorobenzène, le N-(tout-trans rétinoyloxy)phtalimide, le N-(tout-trans rétinoyloxy)succinimide, le 4-(tout-trans rétinoyloxyacétyl)-méthoxybenzène, le 4-(tout-trans rétinoyloxyacétyl)-phénol, le 4-(tout-trans rétinoyloxyacétyl)3,4,5-triméthoxybenzène, le 4-(tout-trans rétinoyloxyacétyl)2,4,6-triméthylbenzène, le 4-(tout-trans rétinoyloxyacétyl)toluène, le 4-(tout-trans rétinoyloxyacétyl)éthoxybenzène, le 4-(tout-trans rétinoyloxyacétyl)acétoxybenzène, le 4-(tout-trans rétinoyloxyacétyl)naphtalène, le 4-(tout-trans rétinoyloxyacétyl)biphényle, le 4-(tout-trans rétinoyloxy acétyl)-2,5-diméthoxybenzène, le 1-(tout-trans rétinoyloxyacétyl)2,4-diméthylbenzène, le 1-(tout-trans rétinoyloxyacétyl)3,4-diacétoxybenzène, le tout-trans rétinamide, le 2-hydroxyéthyl tout-trans rétinamide, le N-éthyl tout-trans rétinamide, le 4-(tout-trans rétinoyl) aminophénol, le N-méthyldiméthyldioxolane rétinamide, le N-(orthocarboxyphényl)rétinamide, le N-(p-carboxyphényl)rétinamide, le N-hydroxypropyl tout-trans rétinamide, le N-(hydroxypropyl)13-cis rétinamide, le N-(5-tétrazolyl)tout-trans rétinamide, le N-(5-tétrazolyl)13-cis rétinamide, le N-(3,4 méthylène dioxyphénylméthyl)tout-trans rétinamide, le N-(n-propyl) tout-trans rétinamide, le N-tertiobutyl tout-trans rétinamide, le N-(1,1,3,3 tétraméthyl) butyl tout-trans rétinamide, le N-(4-carboxyméthyl 3-hydroxyphényl) tout-trans rétinamide, le N-[β-(3,4 diméthoxyphényl) éthyl] tout-trans rétinamide, le 2-(tout-trans rétinoylamino) benzotriazole, le 1-(tout-trans rétinoyl) 1,2,4 triazole, le N-(tout-trans rétinoyl)imidazole, la 1-nicotinoyl 2-(tout-trans rétinoyl) hydrazine, la N-(tout-trans rétinoyl) morpholine, la trans-β-ionone (tout-trans rétinoyl) hydrazone, la N,N'-dicycloaexyl N-(tout-trans rétinoyl) urée, l'acétone (tout-trans rétinoyl) hydrazone, la N-benzoylrétinylamine, l'azoture de rétinoyle.

**15.** Association selon la revendication 12, caractérisée par le fait que les rétinoïdes sont choisis parmi les composés de formule (II), dans laquelle :

A désigne l'un quelconque des groupements suivants :

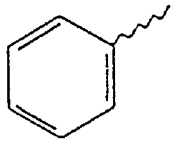
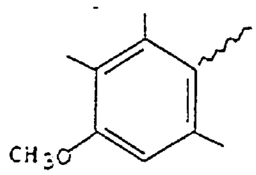
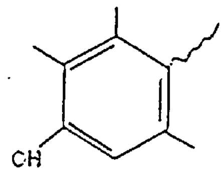

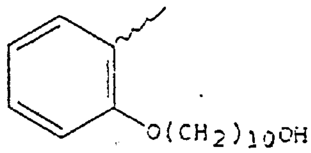
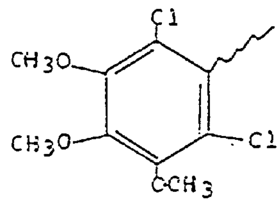

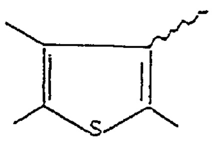
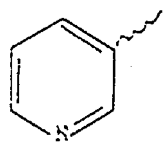
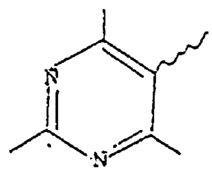

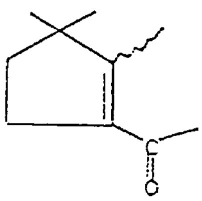
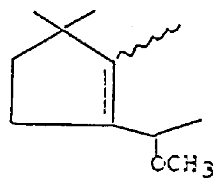

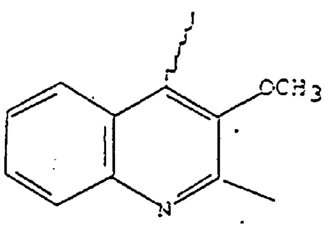
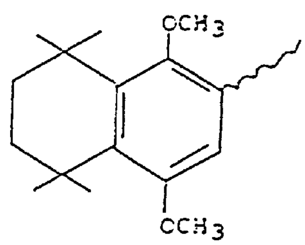

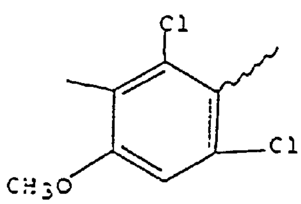

et R désigne COOH, $CONHC_2H_5$ ou $COOC_2H_5$.

**16.** Association selon la revendication 12, caractérisée par le fait que le rétinoïde est choisi parmi les composés de formules :

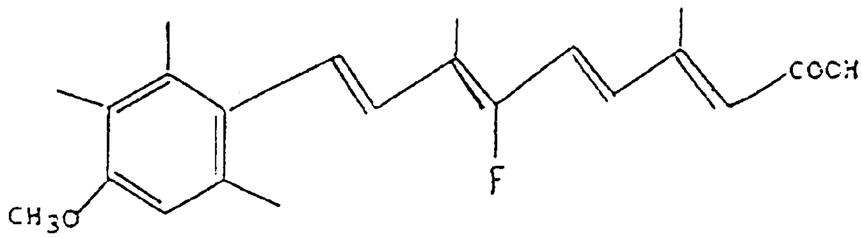

(VII)

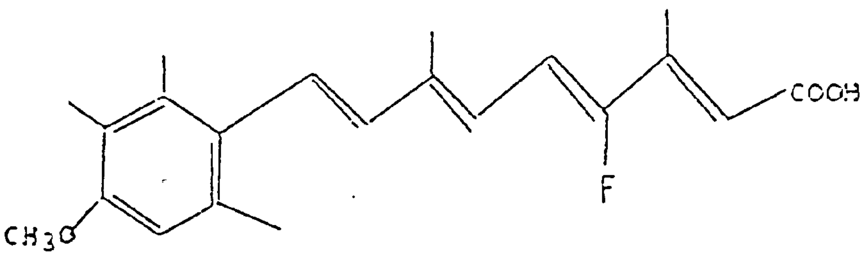

(VIII)

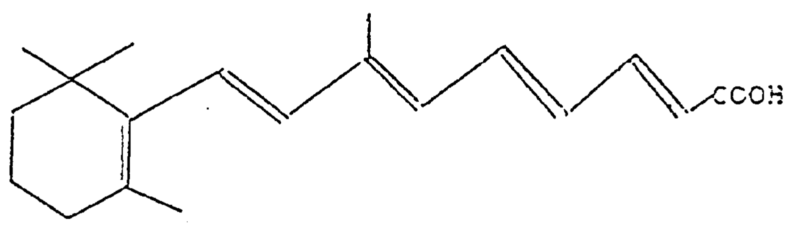

(IX)

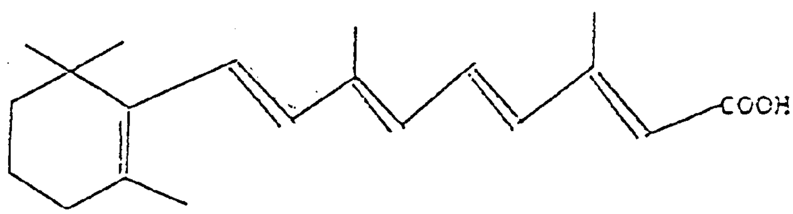

(X)

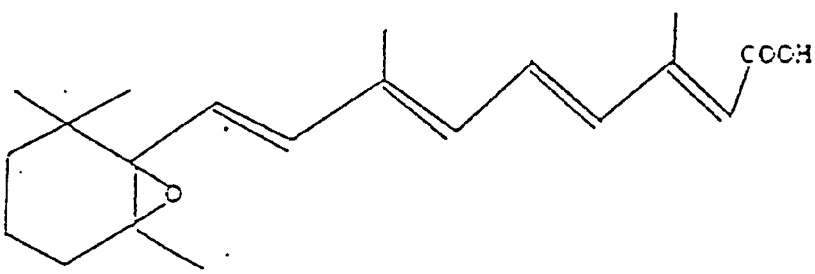

(XI)

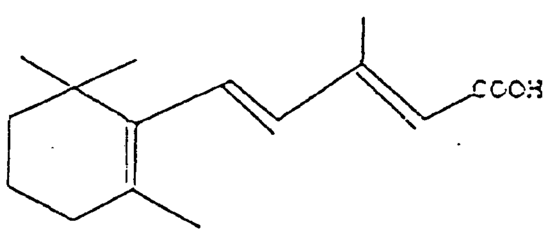

(XII)

ainsi que leurs sels ou esters physiologiquement acceptables.

**17.** Association selon l'une quelconque des revendications 17 à 16, caractérisée par le fait que le rétinoïde est choisi parmi les composés de formule (II) :

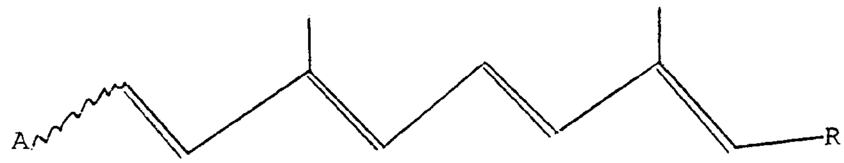

(II)

sous la forme all-trans ou 13-cis, dans laquelle R désigne un groupement

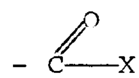

où X peut désigner un groupement OH, OY, Y désignant un groupement alkyle ayant 1 à 15 atomes de carbone, X pouvant également désigner un groupement amino, éventuellement mono- ou disubstitué par un groupement alkyle inférieur ayant 1 à 6 atomes de carbone, R désignant également un groupement $-CH_2OH$, -CHO, et A désignant un groupement :

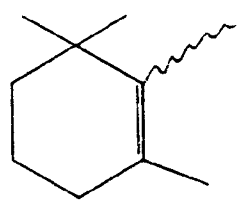

ou

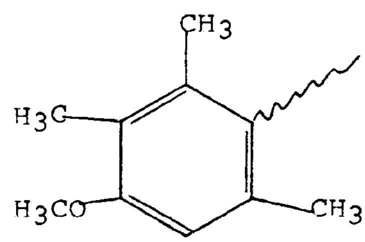

ainsi que les sels pharmaceutiquement ou cosmétiquement acceptables.

**18.** Association selon l'une quelconque des revendications 12 à 15, caractérisée par le fait que le rétinoïde est choisi parmi la trétinoïne, l'isotrétinoïne, le rétinol ou vitamine A et ses dérivés tels que l'acétate, le palmitate ou le propionate, le motrétinide, l'étrétinate, le tout-trans rétinoate de zinc.

**19.** Association selon l'une quelconque des revendications 12 à 17, caractérisée par le fait que les composants (A) et (B) font partie d'une même composition.

**20.** Association selon l'une quelconque des revendications 12 à 18, caractérisée par le fait que les composants (A) et (B) sont destinés à être mélangés de façon extemporanée tout juste avant l'emploi.

**21.** Association selon l'une quelconque des revendications 12 à 18, caractérisée par le fait que les composants (A) et (B) sont destinés à être appliqués de façon séparée ou décalée dans le temps.

**22.** Association selon l'une quelconque des revendications 12 à 21, caractérisée par le fait que le composant (B) contient le rétinoïde dans des proportions comprises entre 0,001 et 2% en poids par rapport au poids total de la composition.

**23.** Dispositif à plusieurs compartiments, caractérisé par le fait qu'il comporte au moins deux compartiments dont l'un contient le composant (A) et l'autre le composant (B) tels que définis dans l'une quelconque des revendications 12 à 22.

**24.** Utilisation de la composition telle que définie dans l'une quelconque des revendications 2 à 10, pour la préparation d'un médicament destiné au traitement de l'alopécie.

**25.** Utilisation de l'association telle que définie dans l'une quelconque des revendications 12 à 22, pour la préparation d'un médicament pour le traitement de l'alopécie.

**26.** Procédé de traitement cosmétique des cheveux, caractérisé par le fait que l'on applique sur le cuir chevelu au moins une composition ou une association telle que définie dans l'une quelconque des revendications 1 à 20.

**27.** Procédé de traitement selon la revendication 26, caractérisé par le fait que l'on applique dans un premier temps le composant (B) contenant le rétinoïde et qu'après une durée de contact d'une minute à 12 heures, on applique le composant (A) contenant le dérivé de pyrimidine de formule (I) et l'agent filtrant les rayonnements UV.

**28.** Procédé de traitement cosmétique du cuir chevelu, caractérisé par le fait qu'on applique sur le cuir chevelu une composition résultant du mélange des composants (A) et (B) de l'association définie dans l'une quelconque des revendications 12 à 22.

**29.** Procédé de solubilisation d'un dérivé de pyrimidine répondant à la formule (I) définie dans la revendication 1, dans un milieu physiologiquement acceptable, caractérisé par le fait que l'on ajoute au milieu physiologiquement acceptable au moins un filtre solaire choisi parmi la 2-hydroxy 4-méthoxybenzophénone, le para-diméthylaminobenzoate de 2-éthyl hexyle, le para-diméthylaminobenzoate de pentyle, le paraméthoxycinnamate de 2-éthylhexyle, le 4-(1,1-diméthyléthyl) 4'-méthoxy dibenzoylméthane, le (N-2-éthyl hexyl)3-[(3'-méthoxy 4'-n-butoxy)benzylidène]10-camphosulfonamide, le 3-(4-méthylbenzylidène)camphre, les salicylates d'homomenthyle et de 2-éthylhexyle, l'acide para-aminobenzoïque, l'acide 2-hydroxy 4-méthoxy benzophénone 5-sulfonique.

## Claims

**Claims for the following Contracting States : DE, GB, FR, IT, NL, SE, CH, BE, AT**

**1.** Composition intended for inducing and stimulating hair growth and decreasing its loss, characterised in that it contains, in a physiologically acceptable medium, at least one pyrimidine derivative corresponding to the formula:

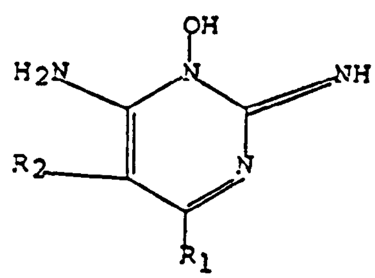

(I)

in which $R_1$ denotes a group

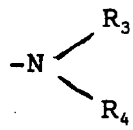

in which $R_3$ and $R_4$ may be selected from hydrogen and a lower cycloalkyl, alkyl, alkenyl or alkylaryl group, it also being possible for $R_3$ and $R_4$, with the nitrogen atom to which they are attached, to form a heterocycle selected from aziridinyl, azetidinyl, pyrrolidinyl, piperidyl, hexahydroazepinyl, heptamethylenimine, octamethylenimine, morpholine and 4-(lower alkyl)piperazinyl groups, it being possible for the heterocyclic groups to be substituted on the carbon atoms with one to three lower alkyl, hydroxy or alkoxy groups; and the group $R_2$ is selected from hydrogen and a lower haloarylalkyl, alkyl, alkenyl, alkoxyalkyl, cycloalkyl,

aryl, alkylaryl, arylalkyl, alkylarylalkyl or alkoxyarylalkyl group; as well as the addition salts with physiologically acceptable acids; and at least one agent screening out UV radiation, selected from the following compounds:

- 2-hydroxy-4-methoxybenzophenone;
- 2-ethylhexyl para-dimethylaminobenzoate;
- pentyl para-dimethylaminobenzoate;
- 2-ethylhexyl para-methoxycinnamate;
- 4-(1,1-dimethylethyl)-4′-methoxydibenzoylmethane;
- N-(2-ethylhexyl)-3-[(3′-methoxy-4′-n-butoxy)benzylidene]-10-camphorsulphonamide;
- 3-(4-methylbenzylidene)camphor;
- homomenthyl salicylate;
- 2-ethylhexyl salicylate;
- para-aminobenzoic acid not complexed with allantoin; and
- 2-hydroxy-4-methoxybenzophenone-5-sulphonic acid;

as well as mixtures thereof.

**2.** Composition according to Claim 1, characterised in that the pyrimidine derivative of formula (I) is selected from the compounds of formula (I) in which $R_2$ denotes hydrogen and $R_1$ denotes a group:

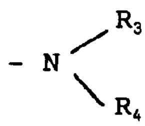

in which $R_3$ and $R_4$ form a piperidyl ring, as well as their salts.

**3.** Composition according to Claim 1 or 2, characterised in that the pyrimidine derivative of formula (I) is present in the composition in proportions of between 0.1 and 10% by weight, and more especially between 1 and 5% by weight, relative to the total weight of the composition.

**4.** Composition according to any one of Claims 1 to 3, characterised in that the agent screening out UV radiation is present in proportions sufficient to increase the solubility of the pyrimidine derivative in the physiologically acceptable medium by at least 10%, and preferably by at least 20%, relative to the solubility of the pyrimidine derivative of formula (I) in this same medium.

**5.** Composition according to Claim 4, characterised in that the agents screening out UV radiation are present in proportions of between 0.1 and 10%, and preferably between 0.3 and 4%, relative to the total weight of the composition.

**6.** Composition according to any one of Claims 1 to 5, characterised in that it is presented in anhydrous form.

**7.** Composition according to Claim 6, characterised in that it contains an anhydrous solvent medium consisting of a solvent or mixture of solvents selected from $C_2$-$C_4$ lower alcohols, alkylene glycols and alkylene glycol or dialkylene glycol alkyl ethers.

**8.** Composition according to any one of Claims 1 to 5, characterised in that it is presented in aqueous or aqueous-alcoholic form.

**9.** Composition according to Claim 8, characterised in that it contains a physiologically acceptable aqueous medium consisting of water and a solvent or mixture of solvents selected from lower alcohols, alkylene glycols and alkylene glycol or dialkylene glycol alkyl ethers.

**10.** Combination intended for inducing and stimulating hair growth and decreasing its loss, characterised in that it comprises:

(a) a component (A) consisting of a composition as defined in any one of Claims 1 to 9; and
(b) a component (B) containing at least one retinoid in a physiologically acceptable medium.

**11.** Combination according to Claim 10, characterised in that the retinoid is selected from the compounds of formula:

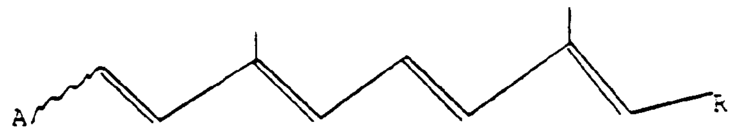 (II)

in which:

(a) A is a group selected from the groups of formulae:

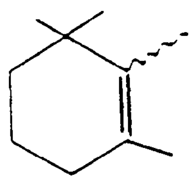

(IIIa)

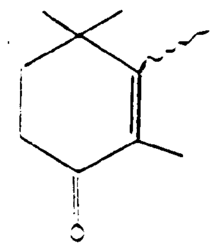 (IV)

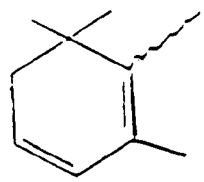 (IIIb)

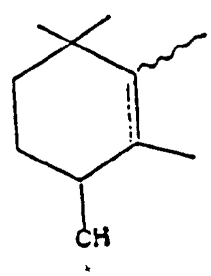 (V)

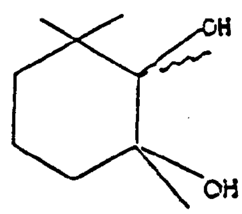 (VI)

– when A denotes a group of formula (IIIa), R is selected from the following groups:
CHO; $CH_2OR_5$,
in which $R_5$ denotes hydrogen or $C_1$-$C_4$ lower alkyl; the group

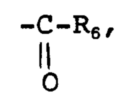

where $R_5$ denotes $C_1$-$C_{16}$ linear or branched alkyl;
$CH_2SR_7$, in which $R_7$ denotes hydrogen or methyl;

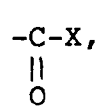

in which X denotes:

(i) OH;

(ii) $OR_5$, where $R_8$ denotes a $C_1$-$C_{15}$ alkyl radical, $C_1$-$C_4$ arylalkyl radical optionally substituted on the aryl group, $C_1$-$C_4$ arylcarboxyalkyl radical optionally substituted on the aryl group, or $C_1$-$C_4$ hydroxyalkyl or $C_1$-$C_4$ amidoalkyl radical;

(iii) $NR_9R_{10}$, in which $R_9$ and $R_{10}$, which may be identical or different, denote hydrogen, $C_1$-$C_6$ alkyl, $C_1$-$C_4$ hydroxyalkyl or optionally substituted aryl;

it being possible for $R_9$ or $R_{10}$ to represent an optionally substituted heterocycle or, together

with the nitrogen atom to which they are attached, to form a heterocycle which is itself optionally substituted;

(iv) the $N_3$ group;

or alternatively a group of formula $CH_2NHR_{11}$, in which $R_{11}$ denotes an optionally substituted benzoyl radical;

– when A denotes a group of formula (IIIb), (IV), (V) or (VI), R denotes COOH as well as its salified or esterified form;

(b) A is a group selected from aryl or substituted aryl groups, a heterocycle or substituted heterocycle, an aryl-heterocyclic group optionally substituted on the heterocycle or an aryl-homocyclic group optionally substituted on the aromatic ring, R in this case denoting a COOH group, a group $COOR_{12}$ where $R_{12}$ denotes a $C_1$-$C_4$ alkyl radical or alternatively an amide group substituted with a $C_1$-$C_4$ alkyl group, as well as their physiologically acceptable salts and esters.

**12.** Combination according to either of Claims 10 and 11, characterised in that the retinoid is selected from retinal, retinol, retinyl acetate, propionate and palmitate, retinoic acid in all-trans, 13-cis, 9-cis, 11-cis, 9,13-dicis and 11,13-dicis forms, the corresponding zinc retinoates and the quaternary ammonium retinoates of formula:

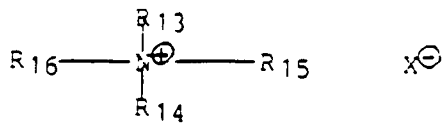

(VII)

in which $X^{\ominus}$ denotes an all-trans- or 13-cis-retinoate radical; and

(i) $R_{13}$, $R_{14}$ and $R_{15}$, which may be identical or different, denote a $C_1$-$C_4$ linear alkyl group which can bear one or more hydroxyl group(s) in the chain,

$R_{16}$ denoting $C_{12}$-$C_{18}$ linear alkenyl or alkyl;

(ii) $R_{15}$ denotes a group:

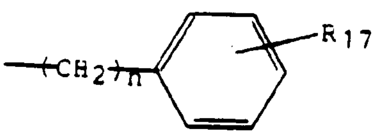

in which:

n is equal to 0 or 1,

$R_{17}$ represents a hydrogen or halogen atom or a hydroxyl, $C_1$-$C_{18}$ alkyl or hydroxyalkyl or $C_2$-$C_{18}$ acyl group;

$R_{13}$, $R_{14}$ and $R_{16}$ having the meanings stated under (i);

(iii) $R_{13}$ and $R_{14}$ can form an aliphatic heterocycle containing at least one oxygen atom, one nitrogen atom or one sulphur atom;

$R_{15}$ or $R_{16}$ having the meanings stated under (i) and (ii);

all-trans-retinoyloxyacetamide, a mixture of 2-hydroxy-1-propyl and 1-hydroxy-2-propyl all-trans-retinoates, 2-hydroxyethyl all-trans-retinoate, 4-nitrobenzyl all-trans-retinoate, benzyl all-trans-retinoate, 4-(all-trans-retinoyloxyacetyl)catechol, 2-cyclohexylethyl all-trans-retinoate, 10-carboxymethyldecyl all-trans-retinoate, 4-hydroxybutyl all-trans-retinoate, cholesteryl all-trans-retinoate, 4-bromobenzyl all-trans-retinoate, cholesteryl all-trans-retinoyloxyacetate, all-trans-retinoyloxyacetylbenzene, 4-(all-trans-retinoyloxyacetyl)bromobenzene, 4-(all-trans-retinoyloxyacetyl)nitrobenzene, 4-(all-trans-retinoyloxyacetyl) benzonitrile, all-trans-retinoyloxyacetyl-2,4-dichlorobenzene, N-(all-trans-retinoyloxy)phthalimide, N-(all-trans-retinoyloxy)succinimide, 4-(all-trans-retinoyloxyacetyl)methoxybenzene, 4-(all-trans-retinoyloxyacetyl)phenol, 4-(all-trans-retinoyloxyacetyl)-3,4,5-trimethoxybenzene, 4-(all-trans-retinoyloxyacetyl)-2,4,6-trimethylbenzene, 4-(all-trans-retinoyloxyacetyl)toluene, 4-(all-trans-retinoyloxyacetyl)ethoxybenzene, 4-(all-trans-retinoyloxyacetyl)acetoxybenzene, 4-(all-trans-retinoyloxyacetyl)naphthalene, 4-(all-trans-retinoyloxyacetyl)biphenyl, 4-(all-trans-retinoyloxyacetyl)-2,5-dimethoxybenzene, 1-(all-trans-retinoyloxyacetyl)-2,4-dimethylbenzene, 1-(all-trans-retinoyloxyacetyl)-3,4-diacetoxybenzene, all-trans-retinamide,

2-hydroxyethyl all-trans-retinamide, N-ethyl-all-trans-retinamide, 4-(all-trans-retinoyl)aminophenol, N-(methyldimethyldioxolane)retinamide, N-(ortho-carboxyphenyl)retinamide, N-(p-carboxyphenyl)retinamide, N-hydroxypropyl-all-trans-retinamide, N-(hydroxypropyl)-13-cis-retinamide,N-(5-tetrazolyl)-all-trans-retinamide,N-(5-tetrazolyl)-13-cis-retinamide, N-(3,4-methylenedioxyphenylmethyl)-all-trans-retinamide, N-(n-propyl)-all-trans-retinamide, N-tert-butyl-all-trans-retinamide, N-(1,1,3,3-tetramethylbutyl)-all-transretinamide, N-(4-carboxymethyl-3-hydroxyphenyl)-all-trans-retinamide, N-[β-(3,4-dimethoxyphenyl)ethyl]-all-trans-retinamide, 2-(all-trans-retinoylamino)benzotriazole, 1-(all-trans-retinoyl)-1,2,4-triazole, N-(all-trans-retinoyl)imidazole, 1-nicotinoyl-2-(all-transretinoyl)hydrazine, N-(all-trans-retinoyl)morpholine, trans-β-ionone (all-trans-retinoyl)hydrazone, N,N'-dicyclohexyl-N-(all-trans-retinoyl)urea, acetone (all-trans-retinoyl)hydrazone, N-benzoylretinylamine and retinoyl azide.

**13.** Combination according to Claim 10, characterised in that the retinoids are selected from the compounds of formula (II) in which:

A denotes any of the following groups:

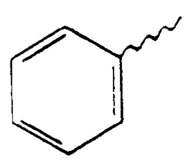
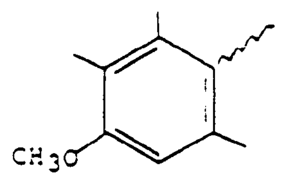
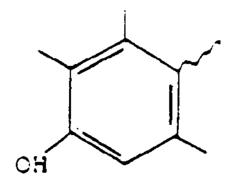
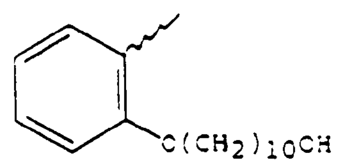
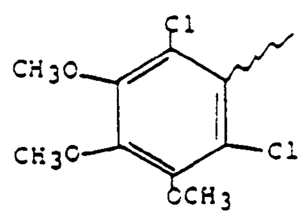
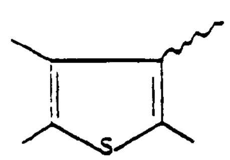
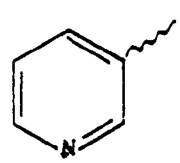
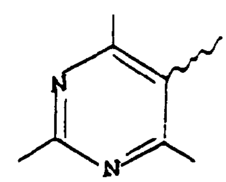

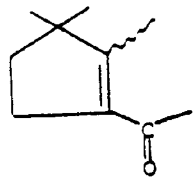

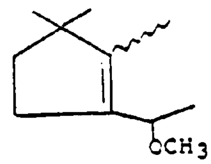

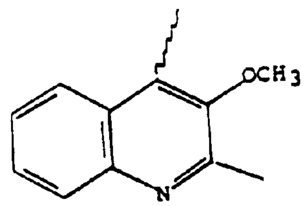

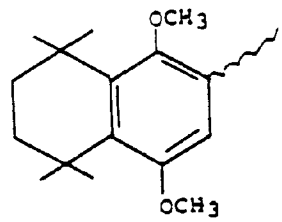

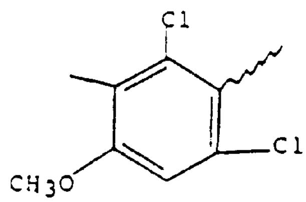

and R denotes COOH, $CONHC_2H_5$ or $COOC_2H_5$.

**14.** Combination according to Claim 10, characterised in that the retinoid is selected from the compounds of formulae:

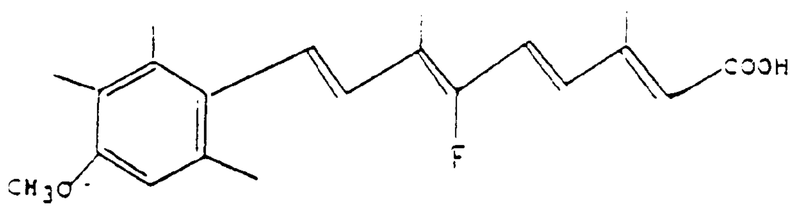

(VII)

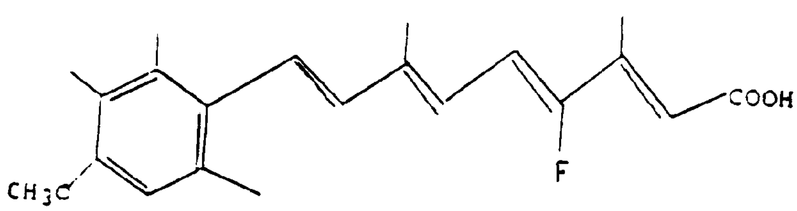

(VIII)

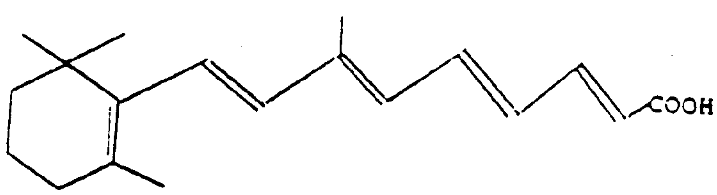

(IX)

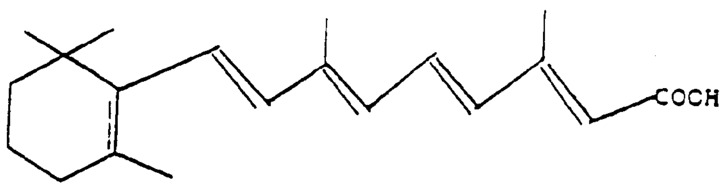

(X)

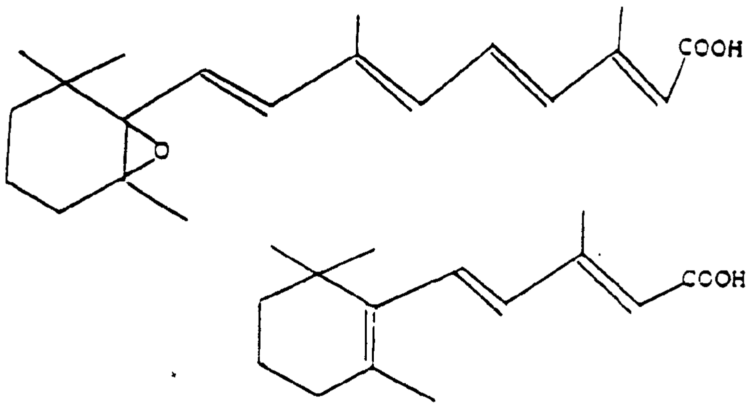

(XI)

(XII)

as well as their physiologically acceptable salts or esters.

**15.** Combination according to any one of Claims 10 to 14, characterised in that the retinoid is selected from the compounds of formula (II):

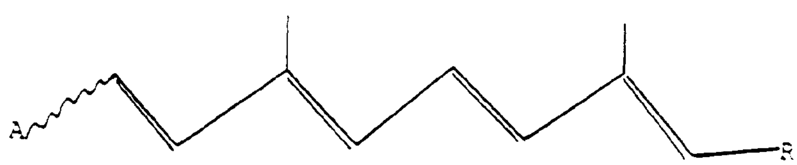

(II)

in all-trans or 13-cis form, in which R denotes a group

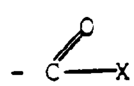

where X can denote an OH group or a group OY, Y denoting an alkyl group having 1 to 15 carbon atoms, it also being possible for X to denote an amino group, optionally mono-or disubstituted with a lower alkyl group having 1 to 6 carbon atoms, R also denoting a $-CH_2OH$ or -CHO group, and A denoting a group:

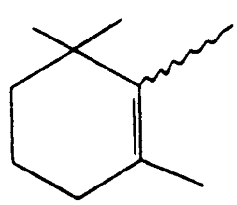

or

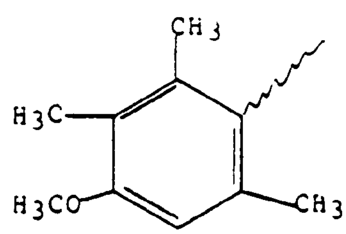

as well as the pharmaceutically or cosmetically acceptable salts.

**16.** Combination according to any one of Claims 10 to 13, characterised in that the retinoid is selected from

tretinoin, isotretinoin, retinol or vitamin A and its derivatives such as the acetate, palmitate or propionate, motretinide, etretinate and zinc all-trans-retinoate.

17. Combination according to any one of Claims 10 to 16, characterised in that the components (A) and (B) form part of one and the same composition.

18. Combination according to any one of Claims 10 to 16, characterised in that the components (A) and (B) are intended for mixing at the required time immediately before use.

19. Combination according to any one of Claims 10 to 16, characterised in that the components (A) and (B) are intended for application separately or separated by an interval of time.

20. Combination according to any one of Claims 10 to 19, characterised in that the component (B) contains the retinoid in proportions of between 0.001 and 2% by weight relative to the total weight of the composition.

21. Multicompartment device, characterised in that it comprises at least two compartments, one of which contains the component (A) and the other the component (B) as defined in any one of Claims 10 to 20.

22. Composition according to any one of Claims 1 to 9, for application in the therapeutic treatment of alopecia.

23. Combination according to any one of Claims 10 to 20, for use in the therapeutic treatment of alopecia.

24. Process for cosmetic treatment of the hair, characterised in that at least one composition or one combination as defined in any one of Claims 1 to 20 is applied to the scalp.

25. Treatment process according to Claim 24, characterised in that the component (B) containing the retinoid is applied in a first stage, and in that, after a contact time of one minute to 12 hours, the component (A) containing the pyrimidine derivative of formula (I) and the agent screening out UV radiation is applied.

26. Process for cosmetic treatment of the scalp, characterised in that a composition resulting from a mixture of the components (A) and (B) of the combination defined in any one of Claims 10 to 20 is applied to the scalp.

27. Process for solubilising a pyrimidine derivative corresponding to the formula (I), defined in Claim 1, in a physiologically acceptable medium, characterised in that at least one sunscreen selected from 2-hydroxy-4-methoxybenzophenone, 2-ethylhexyl para-dimethylaminobenzoate, pentyl para-dimethylaminobenzoate, 2-ethylhexyl para-methoxycinnamate, 4-(1,1-dimethylethyl)-4′-methoxydibenzoylmethane, N-(2-ethylhexyl)-3-[(3′-methoxy-4′-n-butoxy)benzylidene]-10-camphorsulphonamide, 3-(4-methylbenzylidene) camphor, homomenthyl and 2-ethylhexyl salicylates, para-aminobenzoic acid and 2-hydroxy-4-methoxybenzophenone-5-sulphonic acid is added to the physiologically acceptable medium.

**Claims for the following Contracting State : ES**

1. Process for the preparation of a composition intended for inducing and stimulating hair growth and decreasing its loss, characterised in that said composition is obtained by introducing, in a physiologically acceptable medium, at least one pyrimidine derivative corresponding to the formula:

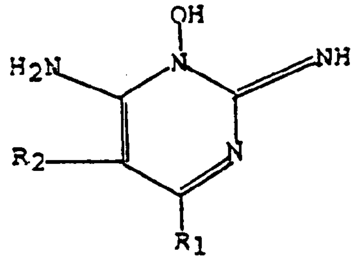

(I)

in which $R_1$ denotes a group

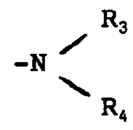

in which $R_3$ and $R_4$ may be selected from hydrogen and a lower cycloalkyl, alkyl, alkenyl or alkylaryl group, it also being possible for $R_3$ and $R_4$, with the nitrogen atom to which they are attached, to form a heterocycle selected from aziridinyl, azetidinyl, pyrrolidinyl, piperidyl, hexahydroazepinyl,heptamethylenimine, octamethylenimine, morpholine and 4-(lower alkyl)piperazinyl groups, it being possible for the heterocyclic groups to be substituted on the carbon atoms with one to three lower alkyl, hydroxy or alkoxy groups; and the group $R_2$ is selected from hydrogen and a lower haloarylalkyl, alkyl, alkenyl, alkoxyalkyl, cycloalkyl, aryl; alkylaryl, arylalkyl, alkylarylalkyl or alkoxyarylalkyl group; as well as the addition salts with physiologically acceptable acids;
and at least one agent screening out UV radiation, selected from the following compounds:

- 2-hydroxy-4-methoxybenzophenone;
- 2-ethylhexyl para-dimethylaminobenzoate;
- pentyl para-dimethylaminobenzoate;
- 2-ethylhexyl para-methoxycinnamate;
- 4-(1,1-dimethylethyl)-4′-methoxydibenzoylmethane;
- N-(2-ethylhexyl)-3-[(3′-methoxy-4′-n-butoxy)benzylidene]-10-camphorsulphonamide;
- 3-(4-methylbenzylidene)camphor;
- homomenthyl salicylate;
- 2-ethylhexyl salicylate;
- para-aminobenzoic acid not complexed with allantoin; and
- 2-hydroxy-4-methoxybenzophenone-5-sulphonic acid;

as well as mixtures thereof.

2. Composition intended for inducing and stimulating hair growth and decreasing its loss, characterised in that it contains, in a physiologically acceptable medium, at least one pyrimidine derivative corresponding to the formula:

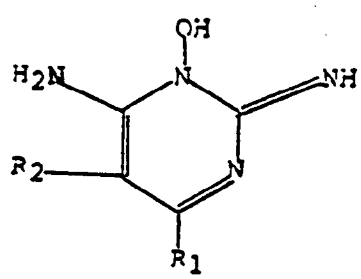

(I)

in which $R_1$ denotes a group

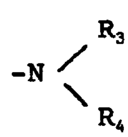

in which $R_3$ and $R_4$ may be selected from hydrogen and a lower cycloalkyl, alkyl, alkenyl or alkylaryl group, it also being possible for $R_3$ and $R_4$, with the nitrogen atom to which they are attached, to form a heterocycle selected from aziridinyl, azetidinyl, pyrrolidinyl, piperidyl, hexahydroazepinyl,heptamethylenimine,octamethylenimine, morpholine and 4-(lower alkyl)piperazinyl groups, it being possible for the heterocyclic groups to be substituted on the carbon atoms with one to three lower alkyl, hydroxy or alkoxy groups; and

the group $R_2$ is selected from hydrogen and a lower haloarylalkyl, alkyl, alkenyl, alkoxyalkyl, cycloalkyl, aryl, alkylaryl, arylalkyl, alkylarylalkyl or alkoxyarylalkyl group; as well as the addition salts with physiologically acceptable acids;
and at least one agent screening out UV radiation, selected from the following compounds:
- 2-hydroxy-4-methoxybenzophenone;
- 2-ethylhexyl para-dimethylaminobenzoate;
- pentyl para-dimethylaminobenzoate;
- 2-ethylhexyl para-methoxycinnamate;
- 4-(1,1-diméthylethyl)-4′-methoxydibenzoylmethane;
- N-(2-ethylhexyl)-3-[(3′-methoxy-4′-n-butoxy)benzylidene]-10-camphorsulphonamide;
- 3-(4-methylbenzylidene)camphor;
- homomenthyl salicylate;
- 2-ethylhexyl salicylate;
- para-aminobenzoic acid not complexed with allantoin; and
- 2-hydroxy-4-methoxybenzophenone-5-sulphonic acid;

as well as mixtures thereof.

3. Composition according to Claim 2, characterised in that the pyrimidine derivative of formula (I) is selected from the compounds of formula (I) in which $R_2$ denotes hydrogen and $R_1$ denotes a group:

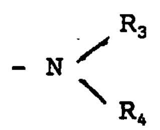

in which $R_3$ and $R_4$ form a piperidyl ring, as well as their salts.

4. Composition according to Claim 2 or 3, characterised in that the pyrimidine derivative of formula (I) is present in the composition in proportions of between 0.1 and 10% by weight, and more especially between 1 and 5% by weight, relative to the total weight of the composition.

5. Composition according to any one of Claims 2 to 4, characterised in that the agent screening out UV radiation is present in proportions sufficient to increase the solubility of the pyrimidine derivative in the physiologically acceptable medium by at least 10%, and preferably by at least 20%, relative to the solubility of the pyrimidine derivative of formula (I) in this same medium.

6. Composition according to Claim 5, characterised in that the agents screening out UV radiation are present in proportions of between 0.1 and 10%, and preferably between 0.3 and 4%, relative to the total weight of the composition.

7. Composition according to any one of Claims 2 to 6, characterised in that it is presented in anhydrous form.

8. Composition according to Claim 7, characterised in that it contains an anhydrous solvent medium consisting of a solvent or mixture of solvents selected from $C_2$-$C_4$ lower alcohols, alkylene glycols and alkylene glycol or dialkylene glycol alkyl ethers.

9. Composition according to any one of Claims 2 to 6, characterised in that it is presented in aqueous or aqueous-alcoholic form.

10. Composition according to Claim 9, characterised in that it contains a physiologically acceptable aqueous medium consisting of water and a solvent or mixture of solvents selected from lower alcohols, alkylene glycols and alkylene glycol or dialkylene glycol alkyl ethers.

11. Process for the preparation of an agent intended for inducing and stimulating hair growth and decreasing its loss, characterised in that said agent is obtained by combining:
(a) a component (A) consisting of a composition as defined in any one of Claims 2 to 10; and
(b) a component (B) containing at least one retinoid in a physiologically acceptable medium.

**12.** Combination intended for inducing and stimulating hair growth and decreasing its loss, characterised in that it comprises:

(a) a component (A) consisting of a composition as defined in any one of Claims 2 to 10; and

(b) a component (B) containing at least one retinoid in a physiologically acceptable medium.

**13.** Combination according to Claim 12, characterised in that the retinoid is selected from the compounds of formula:

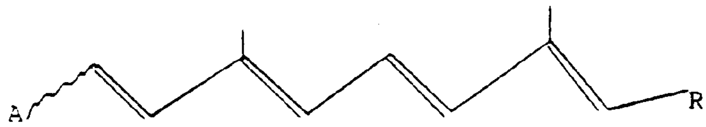 (II)

in which:

(a) A is a group selected from the groups of formulae:

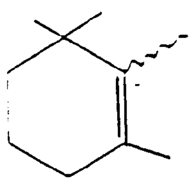

(IIIa)

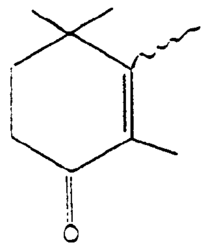 (IV)

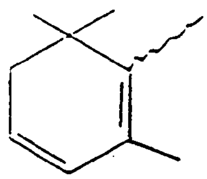 (IIIb)

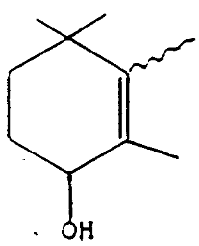 (V)

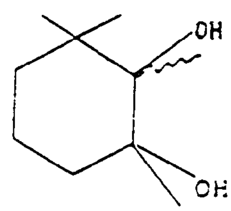 (VI)

– when A denotes a group of formula (IIIa), R is selected from the following groups: CHO; $CH_2OR_5$, in which $R_5$ denotes hydrogen or $C_1$-$C_4$ lower alkyl; the group

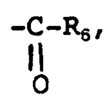

where $R_5$ denotes $C_1$-$C_{16}$ linear or branched alkyl; $CH_2SR_7$, in which $R_7$ denotes hydrogen or methyl;

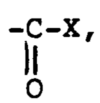

in which X denotes:

(i) OH;

(ii) $OR_8$, where $R_8$ denotes a $C_1$-$C_{15}$ alkyl radical, $C_1$-$C_4$ arylalkyl radical optionally substituted on the aryl group, $C_1$-$C_4$ arylcarboxyalkyl radical optionally substituted on the aryl group, or $C_1$-$C_4$ hydroxyalkyl or $C_1$-$C_4$ amidoalkyl radical;

(iii) $NR_9R_{10}$, in which $R_9$ and $R_{10}$, which may be identical or different, denote hydrogen, $C_1$-$C_6$ alkyl, $C_1$-$C_4$ hydroxyalkyl or optionally substituted aryl;

it being possible for $R_9$ or $R_{10}$ to represent an optionally substituted heterocycle or, together with the nitrogen atom to which they are attached, to form a heterocycle which is itself optionally substituted;

(iv) the $N_3$ group;

or alternatively a group of formula $CH_2NHR_{11}$, in which $R_{11}$ denotes an optionally substituted benzoyl radical;

– when A denotes a group of formula (IIIb), (IV), (V) or (VI), R denotes COOH as well as its salified or esterified form;

(b) A is a group selected from aryl or substituted aryl groups, a heterocycle or substituted heterocycle, an aryl-heterocyclic group optionally substituted on the heterocycle or an aryl-homocyclic group optionally substituted on the aromatic ring, R in this case denoting a COOH group, a group $COOR_{12}$ where $R_{12}$ denotes a $C_1$-$C_4$ alkyl radical or alternatively an amide group substituted with a $C_1$-$C_4$ alkyl group, as well as their physiologically acceptable salts and esters.

**14.** Combination according to either of Claims 12 and 13, characterised in that the retinoid is selected from retinal, retinol, retinyl acetate, propionate and palmitate, retinoic acid in all-trans, 13-cis, 9-cis, 11-cis, 9,13-dicis and 11,13-dicis forms, the corresponding zinc retinoates and the quaternary ammonium retinoates of formula:

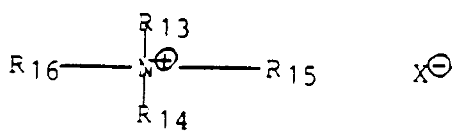

(VII)

in which $X^{\ominus}$ denotes an all-trans- or 13-cis-retinoate radical; and

(i) $R_{13}$, $R_{14}$ and $R_{15}$, which may be identical or different, denote a $C_1$-$C_4$ linear alkyl group which can bear one or more hydroxyl group (s) in the chain,

$R_{16}$ denoting $C_{12}$-$C_{18}$ linear alkenyl or alkyl;

(ii) $R_{15}$ denotes a group:

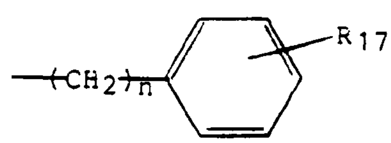

in which:

n is equal to 0 or 1,

$R_{17}$ represents a hydrogen or halogen atom or a hydroxyl, $C_1$-$C_{18}$ alkyl or hydroxyalkyl or $C_2$-$C_{18}$ acyl group;

$R_{13}$, $R_{14}$ and $R_{16}$ having the meanings stated under (i);

(iii) $R_{13}$ and $R_{14}$ can form an aliphatic heterocycle containing at least one oxygen atom, one nitrogen atom or one sulphur atom;

$R_{15}$ or $R_{16}$ having the meanings stated under (i) and (ii);

all-trans-retinoyloxyacetamide, a mixture of 2-hydroxy-1-propyl and 1-hydroxy-2-propyl all-trans-retinoates, 2-hydroxyethyl all-trans-retinoate, 4-nitrobenzyl all-trans-retinoate, benzyl all-trans-retinoate, 4-(all-trans-retinoyloxyacetyl)catechol, 2-cyclohexylethyl all-trans-retinoate, 10-carboxymethyldecyl all-trans-retinoate, 4-hydroxybutyl all-trans-retinoate, cholesteryl all-trans-retinoate, 4-bromobenzyl all-trans-retinoate, cholesteryl all-trans-retinoyloxyacetate, all-trans-retinoyloxyacetylbenzene, 4-(all-trans-retinoyloxyacetyl)bromobenzene, 4-(all-trans-retinoyloxyacetyl)nitrobenzene, 4-(all-trans-retinoyloxyacetyl) benzonitrile, all-trans-retinoyloxyacetyl-2,4-dichlorobenzene, N-(all-trans-retinoyloxy)phthalimide, N-(all-trans-retinoyloxy)-succinimide, 4-(all-trans-retinoyloxyacetyl)methoxybenzene, 4-(all-trans-retinoyloxyacetyl)phenol, 4-(all-trans-retinoyloxyacetyl)-3,4,5-trimethoxybenzene, 4-(all-trans-retinoyloxyacetyl)-2,4,6-trimethylbenzene, 4-(all-trans-retinoyloxyacetyl)toluene, 4-(all-trans-retinoyloxyacetyl)ethoxybenzene, 4-(all-trans-retinoyloxyacetyl)acetoxybenzene, 4-(all-trans-retinoyloxyacetyl)naphthalene, 4-(all-trans-retinoyloxyacetyl)biphenyl, 4-(all-trans-retinoyloxyacetyl)-2,5-dimethoxybenzene, 1-(all-trans-retinoyloxyacetyl)-2,4-dimethylbenzene, 1-(all-trans-retinoyloxyacetyl)-3,4-diacetoxybenzene, all-trans-retinamide, 2-hydroxyethyl all-trans-retinamide, N-ethyl-all-trans-retinamide, 4-(all-trans-retinoyl)aminophenol, N-(methyldimethyldioxolane)retinamide, N-(ortho-carboxyphenyl)retinamide, N-(p-carboxyphenyl)retinamide, N-hydroxypropyl-all-trans-retinamide, N-(hydroxypropyl)-13-cis-retinamide,N-(5-tetrazolyl)-all-trans-retinamide,N-(5-tetrazolyl)-13-cis-retinamide, N-(3,4-methylenedioxyphenylmethyl)-all-trans-retinamide, N-(n-propyl)-all-trans-retinamide, N-tert-butyl-all-trans-retinamide, N-(1,1,3,3-tetramethylbutyl)-all-trans-retinamide, N-(4-carboxymethyl-3-hydroxyphenyl)-all-trans-retinamide, N-[β-(3,4-dimethoxyphenyl)ethyl]-all-trans-retinamide, 2-(all-trans-retinoylamino)benzotriazole, 1-(all-trans-retinoyl)-1,2,4-triazole, N-(all-trans-retinoyl)imidazole, 1-nicotinoyl-2-(all-transretinoyl)hydrazine, N-(all-trans-retinoyl)morpholine, trans-β-ionone (all-trans-retinoyl)hydrazone, N,N′-dicyclohexyl-N-(all-trans-retinoyl)urea, acetone (all-trans-retinoyl)hydrazone, N-benzoylretinylamine and retinoyl azide.

**15.** Combination according to Claim 12, characterised in that the retinoids are selected from the compounds of formula (II) in which:

A denotes any one of the following groups:

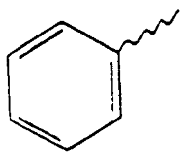

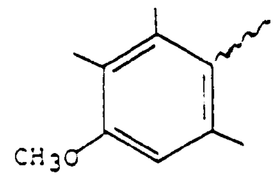

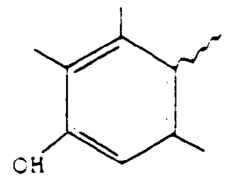

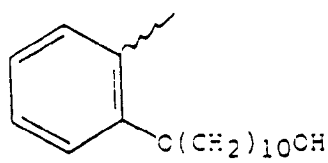

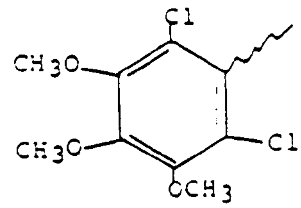

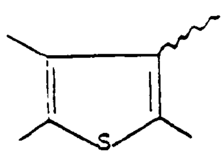

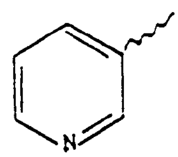

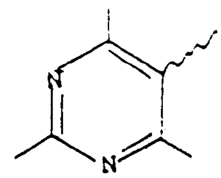

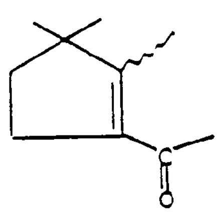

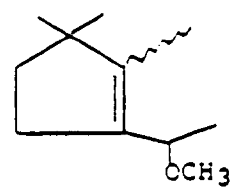

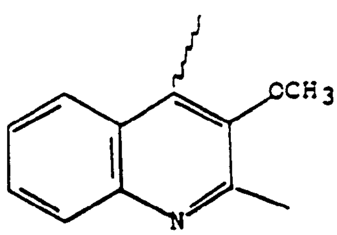

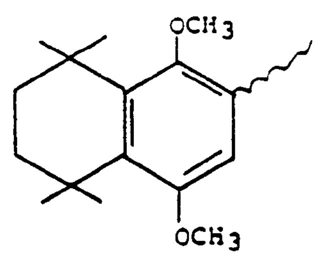

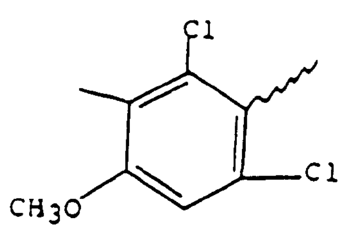

and R denotes COOH, $CONHC_2H_5$ or $COOC_2H_5$.

**16.** Combination according to Claim 12, characterised in that the retinoid is selected from the compounds of formulae:

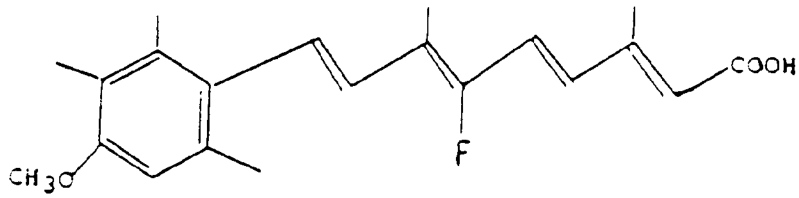

(VII)

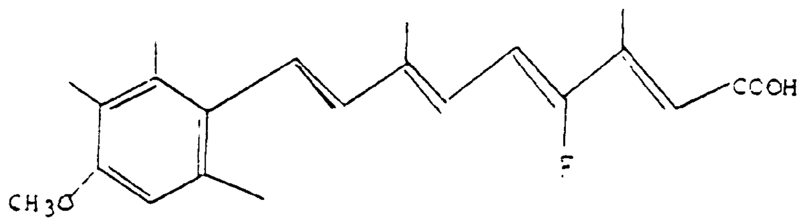

(VIII)

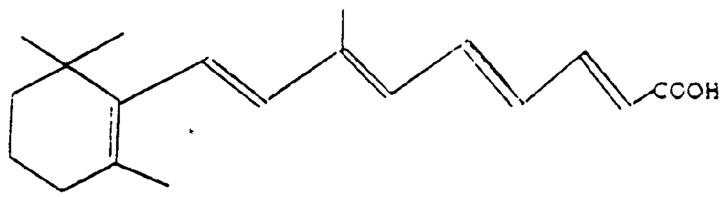

(IX)

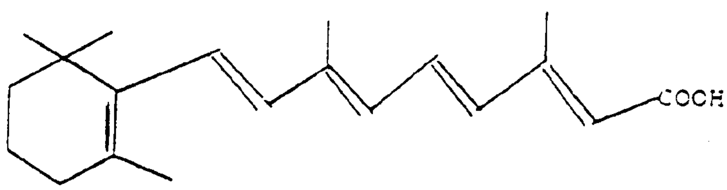

(X)

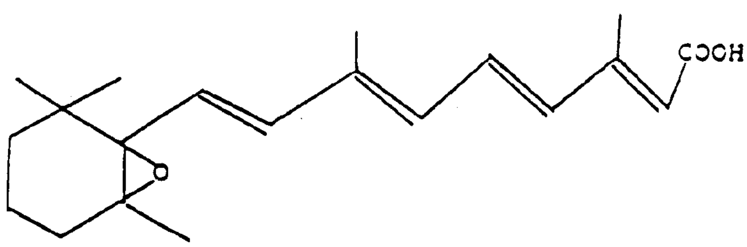

(XI)

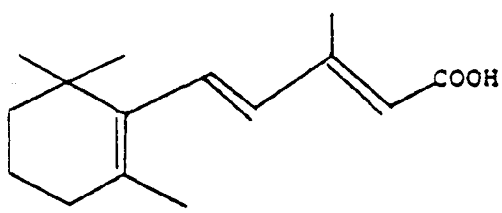

(XII)

as well as their physiologically acceptable salts or esters.

17. Combination according to any one of Claims 12 to 16, characterised in that the retinoid is selected from the compounds of formula (II):

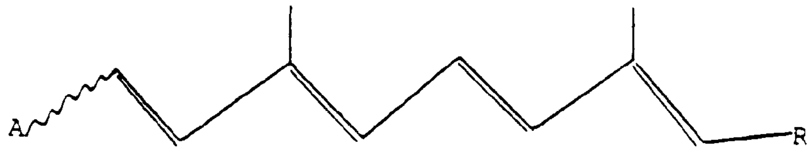

(II)

in all-trans or 13-cis form, in which R denotes a group

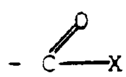

where X can denote an OH group or a group OY, Y denoting an alkyl group having 1 to 15 carbon atoms, it also being possible for X to denote an amino group, optionally mono-or-disubstituted with a lower alkyl group having 1 to 6 carbon atoms, R also denoting a $-CH_2OH$ or -CHO group, and A denoting a group:

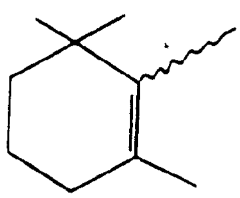

or

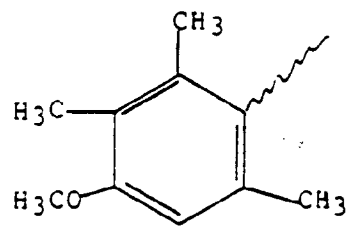

as well as the pharmaceutically or cosmetically acceptable salts.

**18.** Combination according to any one of Claims 12 to 15, characterised in that the retinoid is selected from tretinoin, isotretinoin, retinol or vitamin A and its derivatives such as the acetate, palmitate or propionate; motretinide, etretinate and zinc all-trans-retinoate.

**19.** Combination according to any one of Claims 12 to 18, characterised in that the components (A) and (B) form part of one and the same composition.

**20.** Combination according to any one of Claims 12 to 18, characterised in that the components (A) and (B) are intended for mixing at the required time immediately before use.

**21.** Combination according to any one of Claims 12 to 18, characterised in that the components (A) and (B) are intended for application separately or separated by an interval of time.

**22.** Combination according to any one of Claims 12 to 21, characterised in that the component (B) contains the retinoid in proportions of between 0.001 and 2% by weight relative to the total weight of the composition.

**23.** Multicompartment device, characterised in that it comprises at least two compartments, one of which contains the component (A) and the other the component (B) as defined in any one of Claims 12 to 22.

**24.** Use of the composition as defined in any one of Claims 2 to 10, for the preparation of a medicament intended for the treatment of alopecia.

**25.** Use of the combination as defined in any one of Claims 12 to 22, for the preparation of a medicament for the treatment of alopecia.

**26.** Process for cosmetic treatment of the hair, characterised in that at least one composition or one combination as defined in any one of Claims 1 to 20 is applied to the scalp.

**27.** Treatment process according to Claim 26, characterised in that the component (B) containing the retinoid is applied in a first stage, and in that, after a contact time of one minute to 12 hours, the component (A)

containing the pyrimidine derivative of formula (I) and the agent screening out UV radiation is applied.

**28.** Process for cosmetic treatment of the scalp, characterised in that a composition resulting from a mixture of the components (A) and (B) of the combination defined in any one of Claims 12 to 22 is applied to the scalp.

**29.** Process for solubilising a pyrimidine derivative corresponding to the formula (I), defined in Claim 1, in a physiologically acceptable medium, characterised in that at least one sunscreen selected from 2-hydroxy-4-methoxybenzophenone, 2-ethylhexyl para-dimethylaminobenzoate, pentyl para-dimethylaminobenzoate, 2-ethylhexyl para-methoxycinnamate, 4-(1,1-dimethylethyl)-4′-methoxydibenzoylmethane, N-(2-ethylhexyl)-3-[(3′-methoxy-4′-n-butoxy)benzylidene]-10-camphorsulphonamide, 3-(4-methylbenzylidene) camphor, homomenthyl and 2-ethylhexyl salicylates, para-aminobenzoic acid and 2-hydroxy-4-methoxybenzophenone-5-sulphonic acid is added to the physiologically acceptable medium.

## Patentansprüche

### Patentansprüche für folgende Vertragsstaten : DE, GB, FR, IT, NL, SE, CH, BE, AT

**1.** Zusammensetzung zum Induzieren und Stimulieren des Haarwachstums und zum Vermindern des Haarausfalles,

dadurch **gekennzeichnet** , daß

sie in einem physiologisch verträglichen Milieu mindestens ein Pyrimidinderivat der Formel (I) sowie die physiologisch verträglichen Säureadditionssalze davon:

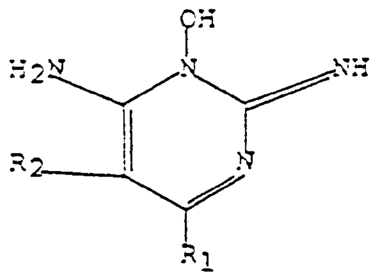

(I)

worin $R_1$ eine

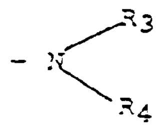

Gruppe darstellt,

worin $R_3$ und $R_4$ unter Wasserstoff, einer Alkyl-, Alkenyl-, Alkylaryl- oder Niedrigcycloalkylgruppe ausgewählt sind, wobei $R_3$ und $R_4$ auch einen Heterozyklus mit dem Stickstoffatom, an das sie gebunden sind, bilden können, ausgewählt aus Aziridinyl-, Azetidinyl-, Pyrrolidinyl-, Piperidinyl-, Hexahydroazepinyl-, Heptamethylenimin-, Octamethylenimin-, Morpholin- und Niedrigalkyl-4-piperazinylgruppen, wobei die heterozyklischen Gruppen an den Kohlenstoffatomen durch 1 bis 3 Niedrigalkyl-, Hydroxy- oder Alkoxygruppen substituiert sein können; und worin die $R_2$-Gruppe unter Wasserstoff, einer Alkyl-, Alkenyl-, Alkoxyalkyl-, Cycloalkyl-, Aryl-, Alkylaryl-, Arylalky-, Alkylarylalkyl-, Alkoxyarylalkyl- oder Haloarylniedrigalkylgruppe ausgewählt ist;

und mindestens ein Mittel umfaßt, das die UV-Strahlung filtert, ausgewählt aus den folgenden Verbindungen:

- 2-Hydroxy-4-methoxybenzophenon;
- p-Dimethylamino-2-ethylhexylbenzoat;
- p-Dimethylaminopentylbenzoat;
- p-Methoxy-2-ethylhexylcinnamat;
- 4-(1,1-Dimethylethyl)-4′-methoxydibenzoylmethan;

- (N-2-Ethylhexyl)-3-((3′-methoxy-4′-n-butoxy)benzylide n)10-camphosulfonamid;
- 3-(4-Methylbenzyliden)campher;
- Homomenthylsalicylat;
- 2-Ethylhexylsalicylat;
- p-Aminobenzoesäure, nicht komplexiert mit Allantoin;
- 2-Hydroxy-4-methoxybenzophenon-5-sulfonsäure; sowie deren Mischungen.

**2.** Zusammensetzung gemäß Anspruch 1, dadurch **gekennzeichnet** , daß das Pyrimidinderivat der Formel (I) aus Verbindungen der Formel (I) ausgewählt ist, worin $R_2$ Wasserstoff und $R_1$ eine Gruppe darstellen:

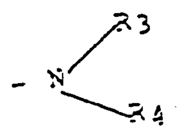

worin $R_3$ und $R_4$ einen Piperidinylring bilden, sowie aus deren Salzen.

**3.** Zusammensetzung gemäß Anspruch 1 oder 2, dadurch **gekennzeichnet** , daß das Pyrimidinderivat der Formel (I) in der Zusammensetzung in Mengen von 0,1 bis 10 Gew.%, insbesondere von 1 bis 5 Gew.%, bezogen auf Gesamtgewicht der Zusammensetzung, vorliegt.

**4.** Zusammensetzung gemäß jedem Anspruch 1 bis 3, dadurch **gekennzeichnet** , daß das Filtermittel für die UV-Strahlung in Mengenanteilen vorliegt, die hinreichen, um die Löslischkeit des Pyrimidinderivats in dem physiologisch verträglichen Milieu zu erhöhen, und zwar mit mindestens 10%, vorzugsweise mindestens 20%, bezogen auf die in diesem Milieu gelöste Menge des Pyrimidinderivats der Formel (I).

**5.** Zusammensetzung gemäß Anspruch 4, dadurch **gekennzeichnet** , daß die Filtermittel für die UV-Strahlung in Mengenanteilen von 0,1 bis 10%, vorzugsweise von 0,3 bis 4%, bezogen auf das Gesamtgewicht der Zusammensetzung, vorliegen.

**6.** Zusammensetzung gemäß jedem Anspruch 1 bis 5, dadurch **gekennzeichnet** , daß sie in wasserfreier Form vorliegt.

**7.** Zusammensetzung gemäß Anspruch 6, dadurch **gekennzeichnet** , daß sie ein wasserfreies Lösungsmittelmilieu umfaßt, zusammengesetzt aus einem Lösungsmittel oder einer Mischung von Lösungsmitteln, ausgewählt aus $C_2$-$C_4$-Niedrigalkoholen, Alkylenglycolen und Alkylethern von Alkylenglycolen oder Dialkylenglycolen.

**8.** Zusammensetzung gemäß jedem Anspruch 1 bis 5, dadurch **gekennzeichnet** , daß sie in wässriger oder wässrig-alkoholischer Form vorliegt.

**9.** Zusammensetzung gemäß Anspruch 8, dadurch **gekennzeichnet** , daß sie ein physiologisch verträgliches wässriges Milieu umfaßt, zusammengesetzt aus Wasser und einem Lösungsmittel oder einer Mischung von Lösungsmitteln, ausgewählt aus Niedrigalkoholen, Alkylenglycolen und Alkylethern von Alkylenglycolen oder Dialkylenglycolen.

**10.** Assoziat zum Induzieren und Stimulieren des Haarwachstums und zum Vermindern des Haarausfalles, dadurch **gekennzeichnet** , daß
es umfaßt:
(a) einen Bestandteil (A) aus einer Zusammensetzung wie in jedem der Ansprüche 1 bis 9 definiert; und
(b) einen Bestandteil (B), enthaltend in einem physiologisch verträglichen Milieu mindestens ein Retinoid.

**11.** Assoziat gemäß Anspruch 10, dadurch **gekennzeichnet** , daß das Retinoid unter den Verbindungen der Formel ausgewählt ist:

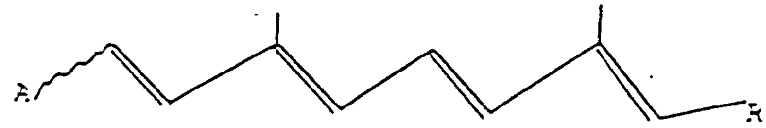

(II)

worin :

(a) A eine Gruppe ist, ausgewählt aus den Gruppen der Formeln:

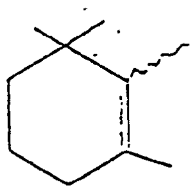

(IIIa)

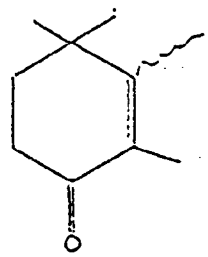

(IV)

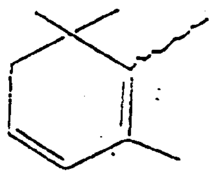

(IIIb)

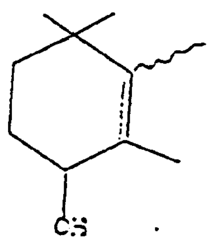

(V)

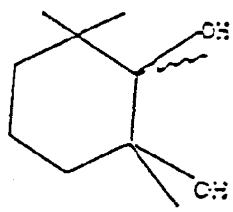

(VI)

– wenn A eine Gruppe der Formel (IIIa) darstellt, ist R unter den folgenden Gruppen ausgewählt: CHO; $CH_2OR_5$, worin $R_5$ darstellt: Wasserstoff, $C_1$-$C_4$-Niedrigalkyl; die -CO-$R_5$-Gruppe, worin $R_6$ eine lineare oder verzweigte $C_1$-$C_{16}$-Alkylgruppe darstellt; $CH_2SR_7$, worin $R_7$ Wasserstoff oder eine Methylgruppe darstellt;

-CO-X, worin X bedeutet:

(i) OH;

(ii) $OR_8$, worin $R_8$ einen $C_1$-$C_{15}$-Alkyl-, einen gegebenenfalls an der Alylgruppe substituierten Aryl-$C_1$-$C_4$-Alkyl-, einen gegebenenfalls an der Arylgruppe substituierten Arylcarboxy-$C_1$-$C_4$-alkyl-, Hydroxy-$C_1$-$C_4$-alkyl-, Amido-$C_1$-$C_4$-alkylrest darstellt;

(iii) $NR_9R_{10}$, worin $R_9$ und $R_{10}$, gleich oder verschieden, Wasserstoff, $C_1$-$C_6$-Alkyl, Hydroxy-$C_1$-$C_4$-Alkyl, gegebenenfalls substituiertes Aryl darstellen;

wobei $R_9$ oder $R_{10}$ gegebenenfalls einen substituierten Heterozyklus darstellen oder $R_9$ und $R_{10}$ zusammen mit dem Stickstoffatom, an das sie gebunden sind, einen seinerseits gegebenenfalls substituierten Heterozyklus bilden können;

(iv) die $N_3$-Gruppe; oder auch einer Gruppe der Formel $CH_2NHR_{11}$, worin $R_{11}$ einen gegebenenfalls substituierten Benzoylrest darstellt;

– wenn A eine Gruppe der Formel (IIIb), (IV), (V) oder (VI) darstellt, bedeutet R COOH sowie dessen Salz- oder Esterform;

(b) A eine Gruppe ist, ausgewählt aus Aryl- oder substituierten Arylgruppen, einem Heterozyklus oder substituierten Heterozyklus, einer gegebenenfalls am Heterozyklus substituierten Arylheterozyklusgruppe oder einer gegebenenfalls am aromatischen Kern substituierten Arylhomozyklusgruppe, wobei R in diesem Fall eine COOH-, $COOR_{12}$-Gruppe darstellt, worin $R_{12}$ einen $C_1$-$C_4$-Alkylrest oder auch eine durch eine $C_1$-$C_4$-Alkylgruppe substituierte Amidgruppe darstellt, sowie aus deren physiologisch verträglichen Salzen und deren Estern.

12. Assoziat gemäß jedem Anspruch 10 und 11, dadurch **gekennzeichnet**, daß das Retinoid ausgewählt ist

unter Retinal, Retinol, Retinylacetat, -propionat, -palmitat, Retinsäure in den Formen all-trans, 13-cis, 9-cis, 11-cis, 9,13-dicis, 11,13-dicis, den entsprechenden Retinoaten von Zink, den quaternären Ammoniumretinoaten der Formel:

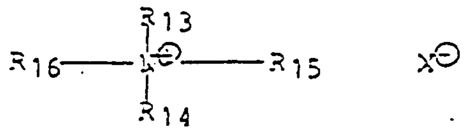

(VII)

worin $X^-$ einen all-trans- oder 13-cis-Retinoatrest darstellt; und

(i) $R_{13}$, $R_{14}$, $R_{15}$, gleich oder verschieden, eine lineare $C_1$-$C_4$-Alkoylgruppe darstellen, die an der Kette eine oder mehrere Hydroxylgruppe(n) tragen kann,

wobei $R_{16}$ einen linearen $C_{12}$-$C_{18}$-Alkoyl-oder -Alkenylrest darstellt;

(ii) $R_{15}$ eine Gruppe bedeutet:

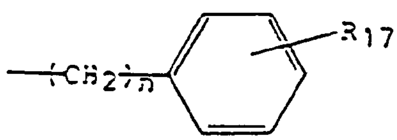

worin:

n gleich 0 oder 1 ist,

$R_{17}$ ein Wasserstoff- oder Halogenatom, eine Hydroxyl-, Alkoyl- oder Hydroxy-$C_1$-$C_{18}$-Alkoyl- oder $C_2$-$C_{18}$-Acylgruppe darstellt;

wobei $R_{13}$, $R_{14}$ und $R_{16}$ die unter (i) angegebenen Bedeutungen haben;

(iii) $R_{13}$ und $R_{14}$ einen aliphatischen Heterozyklus mit mindestens einem Sauerstoff-, Stickstoff-oder Schwefelatom bilden können;

wobei $R_{15}$ oder $R_{16}$ die unter (i) und (ii) angegebenen Bedeutungen haben;
all-trans-Retinoyloxyacetamid, dem Gemisch aus
2-Hydroxy-1-propyl- und
1-Hydroxy-2-propyl-all-trans-retinoaten,
2-Hydroxyethyl-all-trans-retinoat,
4-Nitrobenzyl-all-trans-retinoat,
Benzyl-all-trans-retinoat,
4-(all-trans-Retinoyloxyacetyl)catechol,
2-Cyclohexylethyl-all-trans-retinoat,
10-Carboxymethyldecyl-all-trans-retinoat,
4-Hydroxybutyl-all-trans-retinoat,
Cholesteryl-all-trans-retinoat,
4-Brombenzyl-all-trans-retinoat,
Cholesteryl-all-trans-retinoyloxyacetat,
all-trans-Retinoyloxyacetylbenzol,
4-(all-trans-Retinoyloxyacetyl)brombenzol,
4-(all-trans-Retinoyloxyacetyl)nitrobenzol,
4-(all-trans-Retinoyloxyacetyl)benzonitril,
all-trans-Retinoyloxyacetyl-2,4-dichlorbenzol,
N-(all-trans-Retinoyloxy)phthalimid,
N-(all-trans-Retinoyloxy)succinimid,
4-(all-trans-Retinoyloxyacetyl)phenol,
4-(all-trans-Retinoyloxyacetyl)methoxybenzol,
1-(all-trans-Retinoyloxyacetyl)-3,4,5-trimethoxybenzol,
1-(all-trans-Retinoyloxyacetyl)-2,4,6-trimethylbenzol, 4-(all-trans-Retinoyloxyacetyl)toluol,
4-(all-trans-Retinoyloxyacetyl)ethoxybenzol,
4-(all-trans-Retinoyloxyacetyl)acetoxybenzol,
1-(all-trans-Retinoyloxyacetyl)naphthalin,
4-(all-trans-Retinoyloxyacetyl)biphenyl,

1-(all-trans-Retinoyloxyacetyl)-2,5-dimethoxybenzol,
1-(all-trans-Retinoyloxyacetyl)-2,4-dimethylbenzol ,1-(all-trans-Retinoyloxyacetyl)-3,4-diacetoxybenzol,all-trans-Retinamid,
2-Hydroxyethyl-all-trans-retinamid,
N-Ethyl-all-trans-retinamid,
4-(all-trans-Retinoyl)aminophenol,
N-Methyldimethyldioxolanretinamid,
N-(o-Carboxyphenyl)retinamid,
N-(p-Carboxyphenyl)retinamid,
N-Hydroxypropyl-all-trans-retinamid,
N-(Hydroxypropyl)-13,cis-retinamid,
N-(5-Tetrazolyl)-all-trans-retinamid,
N-(5-Tetrazolyl)-13-cis-retinamid,
N-(3,4-Methylendioxyphenylmethyl)-all-trans-retinamid, N-(n-Propyl)-all-trans-retinamid,
N-t-Butyl-all-trans-retinamid,
N-(1,1,3,3-Tetramethyl)butyl-all-trans-retinamid,
N-(4-Carboxymethyl-3-hydroxyphenyl)-all-trans-retinamid,
N-(β-(3,4-Dimethoxyphenyl)ethyl)-all-trans-retinamid,
2-(all-trans-Retinoylamino)benzotriazol,
1-(all-trans-Retinoyl)-1,2,4-triazol,
N-(all-trans-Retinoyl)imidazol,
1-Nicotinoyl-2-(all-trans-retinoyl)hydrazin,
N-(all-trans-Retinoyl)morpholin, trans-β-Ionon (all-trans-retinoyl)hydrazon,
N,N′-Dicyclohexyl-N-(all-trans-retinoyl)hamstoff,
Aceton(all-trans-retinoyl)hydrazon,
N-Benzoylretinylamin, Azoretinoyl.

**13.** Assoziat gemäß Anspruch 10, dadurch **gekennzeichnet**, daß die Retinoide unter Verbindungen der Formel (II) ausgewählt sind, worin :

A jede der folgenden Gruppen darstellt:

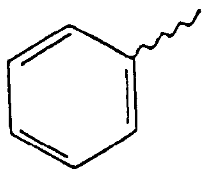

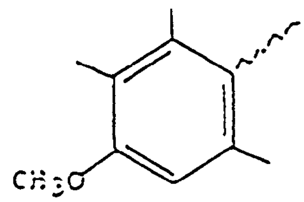

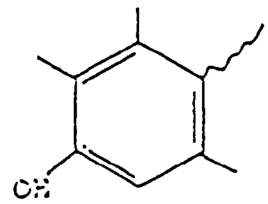

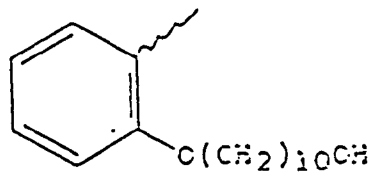

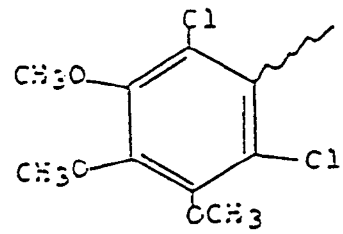

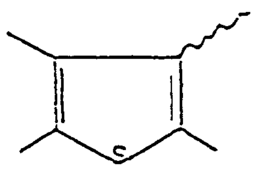

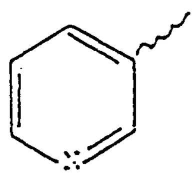

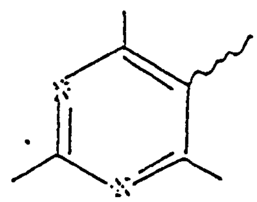

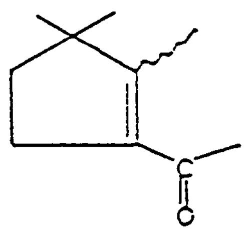

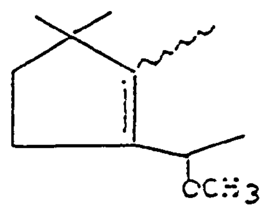

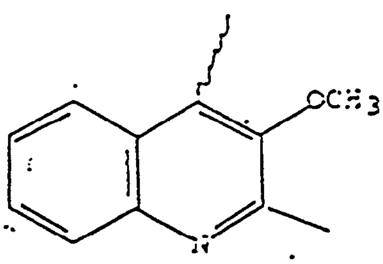

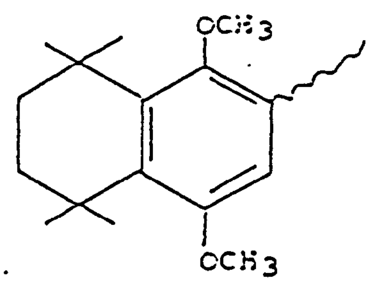

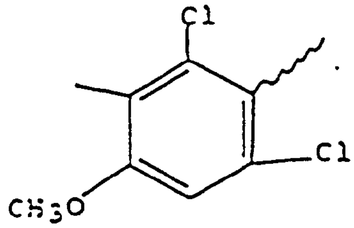

und R COOH, $CONHC_2H_5$ oder $COOC_2H_5$ darstellt.

**14.** Assoziat gemäß Anspruch 10, dadurch **gekennzeichnet**, daß das Retinoid unter den Verbindungen der folgenden Formeln sowie deren physiologisch verträglichen Salzen und Estern ausgewählt ist:

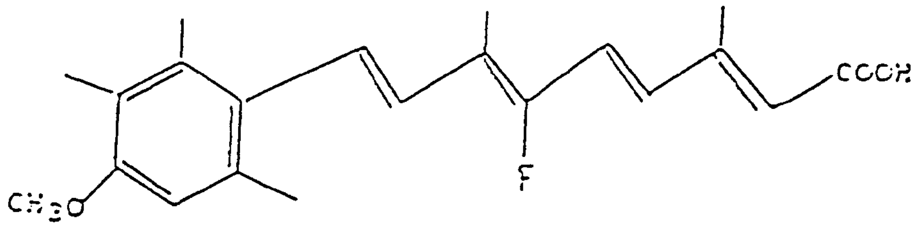

(VII)

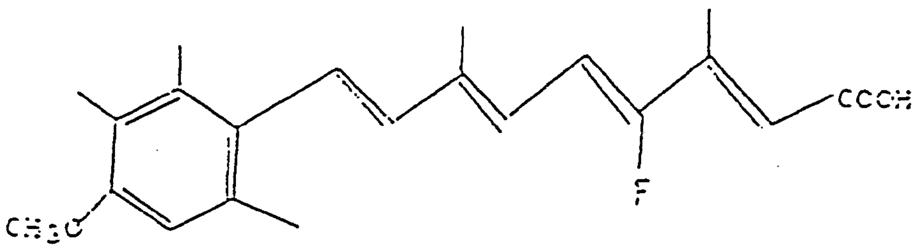

(VIII)

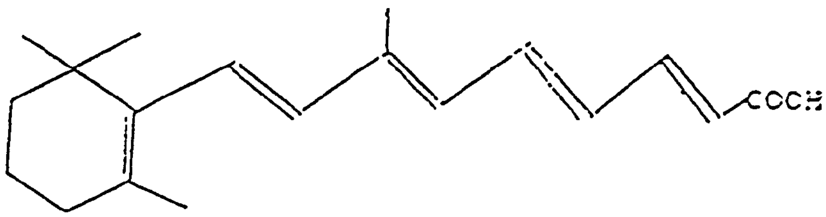

(IX)

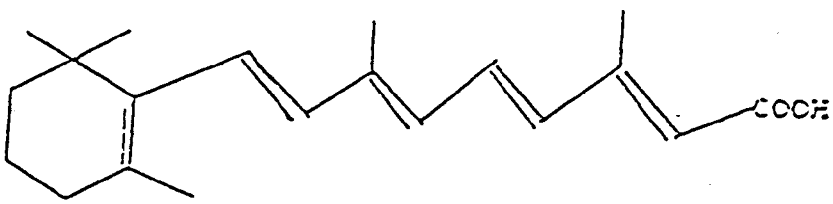

(X)

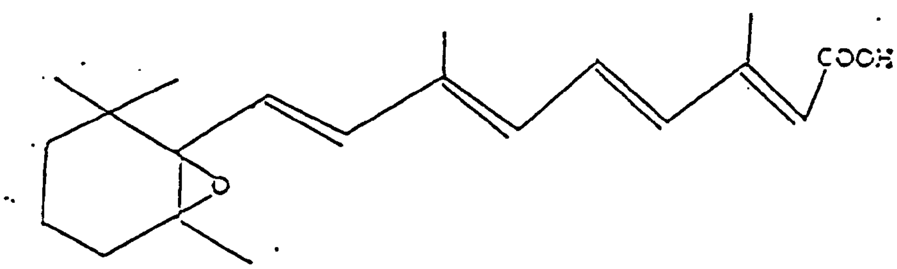

(XI)

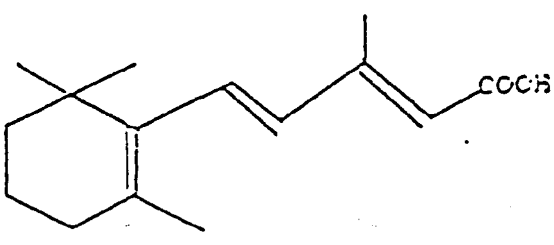

(XII)

**15.** Assoziat gemäß jedem Anspruch 10 bis 14, dadurch **gekennzeichnet**, daß das Retinoid unter den Verbindungen der Formel (II) sowie den pharmazeutisch oder kosmetisch verträglichen Salzen davon ausgewählt ist:

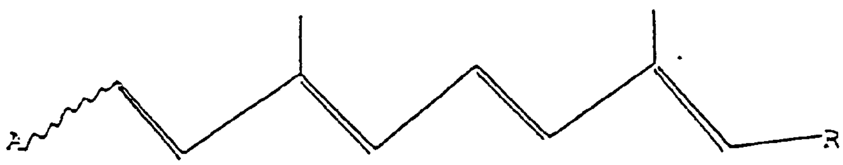

(II)

in der Form all-trans oder 13-cis, worin R eine Gruppe darstellt:

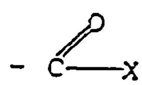

worin X eine OH-, OY-Gruppe bezeichnet, wobei Y eine Alkylgruppe mit 1 bis 15 Kohlenstoffatomen bedeutet, wobei X auch eine Aminogruppe darstellen kann, die gegebenenfalls durch eine Niedrigalkylgruppe mit 1 bis 6 Kohlenstoffatomen mono- oder disubstituiert ist, und worin R auch eine $-CH_2OH-$, -CHO-Gruppe darstellen kann, und worin A eine Gruppe bedeutet:

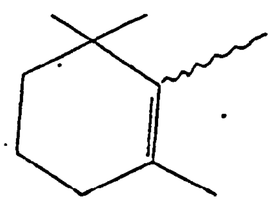

oder

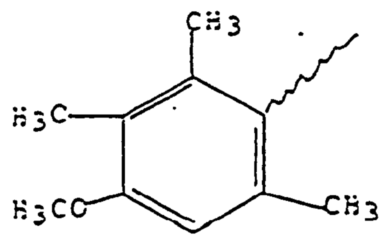

**16.** Assoziat gemäß jedem Anspruch 10 bis 13, dadurch **gekennzeichnet**, daß das Retinoid aus Tretinoin, Isotretinoin, Retinol oder Vitamin A und seinen Derivaten wie dem Acetat, Palmitat oder Propionat, Motretinid, Etretinat, Zink-all-trans-retinoat ausgewählt ist.

**17.** Assoziat gemäß jedem Anspruch 10 bis 16, dadurch **gekennzeichnet** , daß die Bestandteile (A) und (B) Teile ein- und derselben Zusammensetzung ausmachen.

**18.** Assoziat gemäß jedem Anspruch 10 bis 16, dadurch **gekennzeichnet**, daß die Bestandteile (A) und (B) dazu bestimmt sind, kurz vor der Aufbringung unvorbereitet vermischt zu werden.

**19.** Assoziat gemäß jedem anspruch 10 bis 16, dadurch **gekennzeichnet**, daß die Bestandteile (A) und (B) dazu bestimmt sind, in getrennter Weise oder nach einem Zeitablauf aufgebracht zu werden.

**20.** Assoziat gemäß jedem Anspruch 10 bis 19, dadurch **gekennzeichnet**, daß der Bestandteil (B) das Retinoid in Mengenverhältnissen von 0,001 bis 2 Gew.%, bezogen auf Gesamtgewicht der Zusammensetzung, enthält.

**21.** Vorrichtung aus mehreren Teilen, dadurch **gekennzeichnet**, daß sie mindestens zwei Teile umfaßt, wovon der eine den Bestandteil (A) und der andere den Bestandteil (B) wie in jedem Anspruch 10 bis 20 definiert enthält.

**22.** Zusammensetzung gemäß jedem Anspruch 1 bis 9 zur Anwendung in der therapeutischen Behandlung der Alopezie.

**23.** Assoziat gemäß jedem Anspruch 10 bis 20, zur Verwendung in der therapeutischen Behandlung der Alo-

pezie.

**24.** Verfahren zur kosmetischen Behandlung der Haare, dadurch **gekennzeichnet**, daß man auf die behaarte Haut mindestens eine Zusammensetzung oder ein Assoziat wie in jedem Anspruch 1 bis 20 definiert aufbringt.

**25.** Verfahren gemäß Anspruch 24, dadurch **gekennzeichnet**, daß man zu einem ersten Zeitpunkt den Bestandteil (B), enthaltend das Retinoid, und nach einer Kontaktdauer von 1 Minute bis 12 h den Bestandteil A, enthaltend das Pyrimidinderivat der Formel (I) und das Filtermittel für die UV-Strahlungen, aufbringt.

**26.** Verfahren zur kosmetischen Behandlung behaarter Haut, dadurch **gekennzeichnet**, daß man auf die behaarte Haut eine Zusammensetzung aufbringt, die sich aus der Mischung der Bestandteile (A) und (B) des in jedem Anspruch 10 bis 20 definierten Assoziats ergibt.

**27.** Verfahren zum Löslichmachen eines im Anspruch 1 definierten Pyrimidinderivats der Formel (I) in einem physiologisch verträglichen Milieu, dadurch **gekennzeichnet**, daß man dem physiologisch verträglichen Milieu mindestens ein Sonnenfiltermittel zufügt, ausgewählt unter
2-Hydroxy-4-methoxybenzophenon,
p-Dimethylamino-2-ethylhexylbenzoat,
p-Dimethylaminopentylbenzoat,
p-Methoxy-2-ethylhexylcinnamat,
4-(1,1-Dimethylethyl)-4′-methoxydibenzoylmethan,
(N-2-Ethylhexyl)-3-((3′-methoxy-4′-n-butoxy)benzyliden)-10-camphosulfonamid, 3-(4-Methylbenzyliden)campher, den Salicylaten von Homomenthyl und 2-Ethylhexyl, p-Aminobenzoesäure,
2-Hydroxy-4-methoxybenzophenon-5-sulfonsäure.

**Patentansprüche für folgenden Vertragsstaat : ES**

**1.** Verfahren zur Herstellung einer Zusammensetzung zum Induzieren und Stimulieren des Haarwachstums und zum Vermindern des Haarausfalles,
dadurch **gekennzeichnet** ,
daß die Zusammensetzung erhältlich ist, indem man in ein physiologisch verträgliches Milieu mindestens ein Pyrimidinderivat der Formel (I) sowie die physiologisch verträglichen Säureadditionssalze davon:

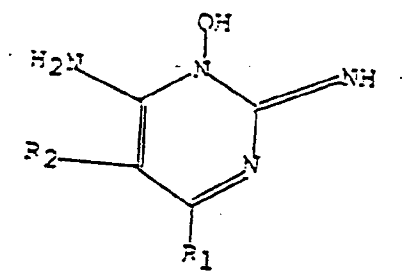

(I)

worin $R_1$ eine

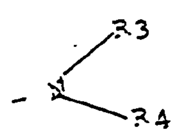

Gruppe darstellt worin $R_3$ und $R_4$ unter Wasserstoff, einer Alkyl-, Alkenyl-, Alkylaryl- oder Niedrigcycloalkylgruppe ausgewählt sind, wobei $R_3$ und $R_4$ auch einen Heterozyklus mit dem Stickstoffatom, an das sie gebunden sind, bilden können, ausgewählt aus Aziridinyl-, Azetidinyl-, Pyrrolidinyl-, Piperidinyl-,Hexahydroazepinyl-, Heptamethylenimin-, Octamethylenimin-, Morpholin- und Niedrigalkyl-4-piperazinylgruppen, wobei die heterozyklischen Gruppen an den Kohlenstoffatomen durch 1 bis 3 Niedrigalkyl-, Hydroxy-

oder Alkoxygruppen substituiert sein können; und worin die $R_2$-Gruppe unter Wasserstoff, einer Alkyl-, Alkenyl-, Alkoxyalkyl-, Cycloalkyl-, Aryl-, Alkylaryl-, Arylalky-, Alkylarylalkyl-, Alkoxyarylalkyl- oder Haloarylniedrigalkylgruppe ausgewähltist;

und mindestens ein Mittel einbringt, das die UV-Strahlung filtert, ausgewählt aus den folgenden Verbindungen:

- 2-Hydroxy-4-methoxybenzophenon;
- p-Dimethylamino-2-ethylhexylbenzoat;
- p-Dimethylaminopentylbenzoat;
- p-Methoxy-2-ethylhexylcinnamat;
- 4-(1,1-Dimethylethyl)-4'-methoxydibenzoylmethan;
- (N-2-Ethylhexyl)-3-((3'-methoxy-4'-n-butoxy)benzyliden)-10-camphosulfonamid;
- 3-(4-Methylbenzyliden)campher;
- Homomenthylsalicylat;
- 2-Ethylhexylsalicylat;
- p-Aminobenzoesäure, nicht komplexiert mit Allantoin;
- 2-Hydroxy-4-methoxybenzophenon-5-sulfonsäure; sowie deren Mischungen.

**2.** Zusammensetzung zum Induzieren und Stimulieren des Haarwachstums und zum Vermindern des Haarausfalles,

dadurch **gekennzeichnet** , daß sie

in einem physiologisch verträglichen Milieu mindestens ein Pyrimidinderivat der Formel (I) sowie die physiologisch verträglichen Säureadditionssalze davon:

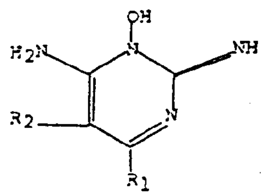

(I)

worin $R_1$ eine

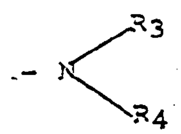

Gruppe darstellt, worin $R_3$ und $R_4$ unter Wasserstoff, einer Alkyl-, Alkenyl-, Alkylaryl- oder Niedrigcycloalkylgruppe ausgewählt sind, wobei $R_3$ und $R_4$ auch einen Heterozyklus mit dem Stickstoffatom, an das sie gebunden sind, bilden können, ausgewählt aus Aziridinyl-, Azetidinyl-, Pyrrolidinyl-, Piperidinyl-, Hexahydroazepinyl-, Heptamethylenimin-, Octamethylenimin-, Morpholin- und Niedrigalkyl-4-piperazinylgruppen, wobei die heterozyklischen Gruppen an den Kohlenstoffatomen durch 1 bis 3 Niedrigalkyl-, Hydroxy- oder Alkoxygruppen substituiert sein können; und worin die $R_2$-Gruppe unter Wasserstoff, einer Alkyl-, Alkenyl-, Alkoxyalkyl-, Cycloalkyl-, Aryl-, Alkylaryl-, Arylalky-, Alkylarylalkyl-, Alkoxyarylalkyl- oder Haloarylniedrigalkylgruppe ausgewählt ist;

und mindestens ein Mittel umfaßt, das die UV-Strahlung filtert, ausgewählt aus den folgenden Verbindungen:

- 2-Hydroxy-4-methoxybenzophenon;
- p-Dimethylamino-2-ethylhexylbenzoat;
- p-Dimethylaminopentylbenzoat;
- p-Methoxy-2-ethylhexylcinnamat;
- 4-(1,1-Dimethylethyl)-4'-methoxydibenzoylmethan;
- (N-2-Ethylhexyl)-3-((3'-methoxy-4'-n-butoxy)benzylide n)10-camphosulfonamid;
- 3-(4-Methylbenzyliden)campher;
- Homomenthylsalicylat;
- 2-Ethylhexylsalicylat;
- p-Aminobenzoesäure, nicht komplexiert mit Allantoin;
- 2-Hydroxy-4-methoxybenzophenon-5-sulfonsäure; sowie deren Mischungen.

**3.** Zusammensetzung gemäß Anspruch 2, dadurch **gekennzeichnet** , daß das Pyrimidinderivat der Formel (I) aus Verbindungen der Formel (I) ausgewählt ist, worin $R_2$ Wasserstoff und $R_1$ eine Gruppe darstellen:

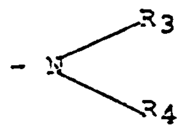

worin $R_3$ und $R_4$ einen Piperidinylring bilden, sowie aus deren Salzen.

**4.** Zusammensetzung gemäß Anspruch 2 oder 3, dadurch **gekennzeichnet** , daß das Pyrimidinderivat der Formel (I) in der Zusammensetzung in Mengen von 0,1 bis 10 Gew.%, insbesondere von 1 bis 5 Gew.%, bezogen auf Gesamtgewicht der Zusammensetzung, vorliegt.

**5.** Zusammensetzung gemäß jedem Anspruch 2 bis 4, dadurch **gekennzeichnet** , daß das Filtermittel für die UV-Strahlung in Mengenanteilen vorliegt, die hinreichen, um die Löslichkeit des Pyrimidinderivats in dem physiologisch verträglichen Milieu zu erhöhen, und zwar mit mindestens 10%, vorzugsweise mindestens 20%, bezogen auf die in diesem Milieu gelöste Menge des Pyrimidinderivats der Formel (I).

**6.** Zusammensetzung gemäß Anspruch 5, dadurch **gekennzeichnet** , daß die Filtermittel für die UV-Strahlung in Mengenanteilen von 0,1 bis 10%, vorzugsweise von 0,3 bis 4%, bezogen auf das Gesamtgewicht der Zusammensetzung, vorliegen.

**7.** Zusammensetzung gemäß jedem Anspruch 2 bis 6, dadurch **gekennzeichnet** , daß sie in wasserfreier Form vorliegt.

**8.** Zusammensetzung gemäß Anspruch 7, dadurch **gekennzeichnet** , daß sie ein wasserfreies Lösungsmittelmilieu umfaßt, zusammengesetzt aus einem Lösungsmittel oder einer Mischung von Lösungsmitteln, ausgewählt aus $C_2$-$C_4$-Niedrigalkoholen, Alkylenglycolen und Alkylethern von Alkylenglycolen oder Dialkylenglycolen.

**9.** Zusammensetzung gemäß jedem Anspruch 2 bis 6, dadurch **gekennzeichnet** , daß sie in wässriger oder wässrig-alkoholischer Form vorliegt.

**10.** Zusammensetzung gemäß Anspruch 9, dadurch **gekennzeichnet** , daß sie ein physiologisch verträgliches wässriges Milieu umfaßt, zusammengesetzt aus Wasser und einem Lösungsmittel oder einer Mischung von Lösungsmitteln, ausgewählt aus Niedrigalkoholen, Alkylenglycolen und Alkylethern von Alkylenglycolen oder Dialkylenglycolen.

**11.** Verfahren zur Herstellung eines Mittels zum Induzieren und Stimulieren des Haarwachstums und zum Vermindern des Haarausfalles,
dadurch **gekennzeichnet** , daß
es erhältlich ist, indem man:
(a) einen Bestandteil (A) aus einer Zusammensetzung wie in jedem Anspruch 2 bis 10 definiert mit
(b) einem Bestandteil (B), enthaltend in einem physiologisch verträglichen Milieu mindestens ein Retinoid, vereinigt.

**12.** Assoziat zum Induzieren und Stimulieren des Haarwachstums und zum Vermindern des Haarausfalles, dadurch **gekennzeichnet** , daß
es umfaßt:
(a) einen Bestandteil (A) aus einer Zusammensetzung wie in jedem der Ansprüche 1 bis 9 definiert; und
(b) einen Bestandteil (B), enthaltend in einem physiologisch verträglichen Milieu mindestens ein Retinoid.

**13.** Assoziat gemäß Anspruch 12, dadurch **gekennzeichnet** , daß das Retinoid unter den Verbindungen der Formel ausgewählt ist:

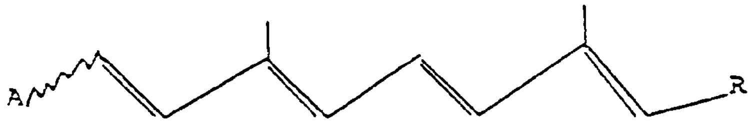

(II)

worin :

(a) A eine Gruppe ist, ausgewählt aus den Gruppen der Formeln:

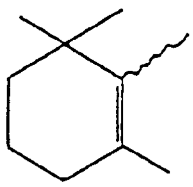

(IIIa)

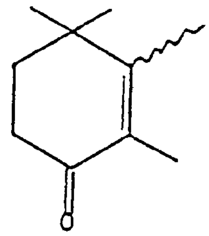

(IV)

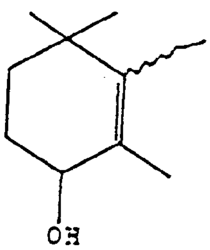

(V)

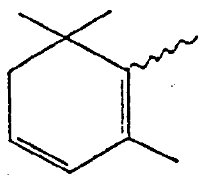

(IIIb)

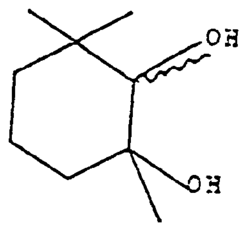

(VI)

– wenn A eine Gruppe der Formel (IIIa) darstellt, ist R unter den folgenden Gruppen ausgewählt: CHO; $CH_2OR_5$, worin $R_5$ darstellt:

Wasserstoff, $C_1$-$C_4$-Niedrigalkyl; die -CO-$R_6$-Gruppe, worin $R_6$ eine lineare oder verzweigte $C_1$-$C_{16}$-Alkylgruppe darstellt;

$CH_2SR_7$, worin $R_7$ Wasserstoff oder eine Methylgruppe darstellt;

-CO-X, worin X bedeutet:

(i) OH;

(ii) $OR_5$, worin $R_8$ einen $C_1$-$C_{15}$-Alkyl-, einen gegebenenfalls an der Arylgruppe substituierten Aryl-$C_1$-$C_4$-Alkyl-, einen gegebenenfalls an der Arylgruppe substituierten Arylcarboxy-$C_1$-$C_4$-alkyl-, Hydroxy-$C_1$-$C_4$-alkyl-, Amido-$C_1$-$C_4$-alkylrest darstellt;

(iii) $NR_9R_{10}$, worin $R_9$ und $R_{10}$, gleich oder verschieden, Wasserstoff, $C_1$-$C_6$ Alkyl, Hydroxy-$C_1$-$C_4$-Alkyl, gegebenenfalls substituiertes Aryl darstellen;

wobei $R_9$ oder $R_{10}$ gegebenenfalls einen substituierten Heterozyklus darstellen oder $R_9$ und $R_{10}$ zusammen mit dem Stickstoffatom, an das sie gebunden sind, einen seinerseits gegebenenfalls substituierten Heterozyklus bilden können;

(iv) die $N_3$-Gruppe;

oder auch einer Gruppe der Formel $CH_2NHR_{11}$, worin $R_{11}$ einen gegebenenfalls substituierten Benzoylrest darstellt;

– wenn A eine Gruppe der Formel (IIIb), (IV), oder (V) oder (VI) darstellt, bedeutet R COOH sowie dessen Salz- oder Esterform;

(b) A eine Gruppe ist, ausgewählt aus Aryl- oder substituierten Arylgruppen, einem Heterozyklus oder substituierten Heterozyklus, einer gegebenenfalls am Heterozyklus substituierten Arylheterozyklusgruppe oder einer gegebenenfalls am aromatischen Kern substituierten Arylhomozyklusgruppe, wobei R in diesem Fall eine COOH-, $COOR_{12}$-Gruppe darstellt, worin $R_{12}$ einen $C_1$-$C_4$-Alkylrest oder auch eine durch eine $C_1$-$C_4$-Alkylgruppe substituierte Amidgruppe darstellt, sowie aus deren physiologisch verträglichen Salzen und deren Estern.

**14.** Assoziat gemäß jedem Anspruch 12 und 13, dadurch **gekennzeichnet**, daß das Retinoid ausgewählt ist unter Retinal, Retinol, Retinylacetat, -propionat, -palmitat, Retinsäure in den Formen all-trans, 13-cis, 9-cis, 11-cis, 9,13-dicis, 11,13-dicis, den entsprechenden Retinoaten von Zink, den quaternären Ammoniumretinoaten der Formel:

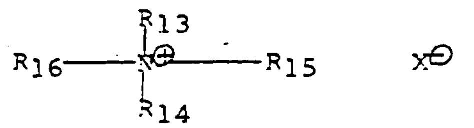

(VII)

worin $X^-$ einen all-trans- oder 13-cis-Retinoatrest darstellt; und

(i) $R_{13}$, $R_{14}$, $R_{15}$, gleich oder verschieden, eine lineare $C_1$-$C_4$-Alkoylgruppe darstellen, die an der Kette eine oder mehrere Hydroxylgruppe(n) tragen kann, wobei $R_{16}$ einen linearen $C_{12}$-$C_{18}$-Alkoyl-oder -Alkenylrest darstellt;

(ii) $R_{15}$ eine Gruppe bedeutet:

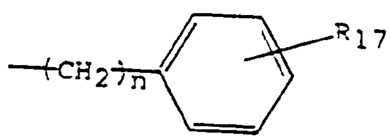

worin:

n gleich 0 oder 1 ist,

$R_{17}$ ein Wasserstoff- oder Halogenatom, eine Hydroxyl-, Alkoyl- oder Hydroxy-$C_1$-$C_{18}$-Alkoyl- oder $C_2$-$C_{18}$-Acylgruppe darstellt;

wobei $R_{13}$, $R_{14}$ und $R_{16}$ die unter (i) angegebenen Bedeutungen haben;

(iii) $R_{13}$ und $R_{14}$ einen aliphatischen Heterozyklus mit mindestens einem Sauerstoff-, Stickstoff-oder Schwefelatom bilden können;

wobei $R_{15}$ oder $R_{16}$ die unter (i) und (ii) angegebenen Bedeutungen haben;
all-trans-Retinoyloxyacetamid, dem Gemisch aus
2-Hydroxy-1-propyl- und
1-Hydroxy-2-propyl-all-trans-retinoaten,
2-Hydroxyethyl-all-trans-retinoat,
4-Nitrobenzyl-all-trans-retinoat,
Benzyl-all-trans-retinoat,
4-(all-trans-Retinoyloxyacetyl)catechol,
2-Cyclohexylethyl-all-trans-retinoat,
10-Carboxymethyldecyl-all-trans-retinoat,
4-Hydroxybutyl-all-trans-retinoat,
Cholesteryl-all-trans-retinoat,
4-Brombenzyl-all-trans-retinoat,
Cholesteryl-all-trans-retinoyloxyacetat,
all-trans-Retinoyloryacetylbenzol,
4-(all-trans-Retinoyloxyacetyl)brombenzol,
4-(all-trans-Retinoyloxyacetyl)nitrobenzol,
4-(all-trans-Retinoyloxyacetyl)benzonitril,
all-trans-Retinoyloxyacetyl-2,4-dichlorbenzol,
N-(all-trans-Retinoyloxy)phthalimid,
N-(all-trans-Retinoyloxy)succinimid,
4-(all-trans-Retinoyloxyacetyl)phenol,
4-(all-trans-Retinoyloxyacetyl)methoxybenzol,
1-(all-trans-Retinoyloxyacetyl)-3,4,5-trimethoxybenzol,
1-(all-trans-Retinoyloxyacetyl)-2,4,6-trimethylbenzol, 4-(all-trans-Retinoyloxyacetyl)toluol,
4-(all-trans-Retinoyloryacetyl)ethorybenzol,
4-(all-trans-Retinoyloxyacetyl)acetoxybenzol,
1-(all-trans-Retinoyloxyacetyl)naphthalin,

4-(all-trans-Retinoyloxyacetyl)biphenyl,
1-(all-trans-Retinoyloxyacetyl)-2,5-dimethoxybenzol,
1-(all-trans-Retinoyloxyacetyl)-2,4-dimethylbenzol ,1-(all-trans-Retinoyloxyacetyl)-3,4-diacetoxybenzol,all-trans-Retinamid,
2-Hydroxyethyl-all-trans-retinamid,
N-Ethyl-all-trans-retinamid,
4-(all-trans-Retinoyl)aminophenol,
N-Methyldimethyldioxolanretinamid,
N-(o-Carboxyphenyl)retinamid,
N-(p-Carboxyphenyl)retinamid,
N-Hydroxypropyl-all-trans-retinamid,
N-(Hydroxypropyl)-13,cis-retinamid,
N-(5-Tetrazolyl)-all-trans-retinamid,
N-(5-Tetrazolyl)-13-cis-retinamid,
N-(3,4-Methylendioxyphenylmethyl)-all-trans-retinamid, N-(n-propyl)-all-trans-retinamid,
N-t-Butyl-all-trans-retinamid,
N-(1,1,3,3-Tetramethyl)butyl-all-trans-retinamid,
N-(4-Carboxymethyl-3-hydroxyphenyl)-all-trans-retinamid,
N-(β-(3,4-Dimethoxyphenyl)ethyl)-all-trans-retinam id, 2-(all-trans-Retinoylamino)benzotriazol,
1-(all-trans-Retinoyl)-1,2,4-triazol,
N-(all-trans-Retinoyl)imidazol,
1-Nicotinoyl-2-(all-trans-retinoyl)hydrazin,
N-(all-trans-Retinoyl)morpholin, trans-β-Ionon (all-trans-retinoyl)hydrazon,
N,N′-Dicyclohexyl-N-(all-trans-retinoyl)harnstoff,
Aceton(all-trans-retinoyl)hydrazon,
N-Benzoylretinylamin, Azoretinoyl.

**15.** Assoziat gemäß Anspruch 12, dadurch **gekennzeichnet** , daß die Retinoide unter Verbindungen der Formel (II) ausgewählt sind, worin :
A jede der folgenden Gruppen darstellt:

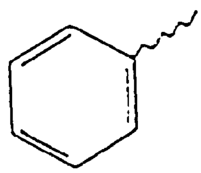

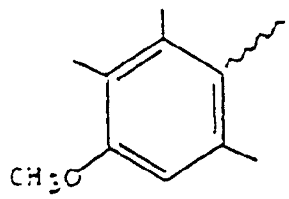

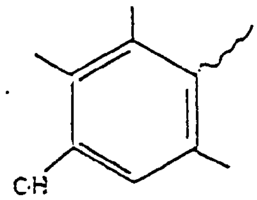

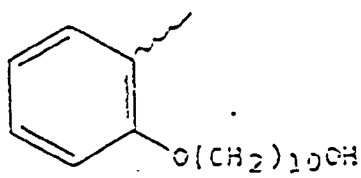

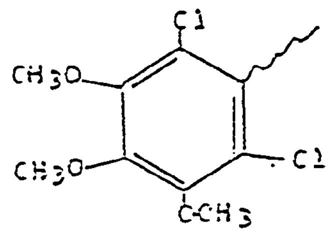

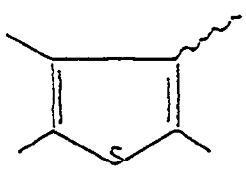

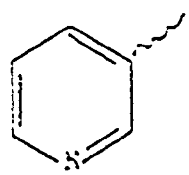

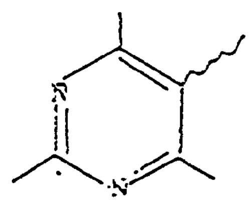

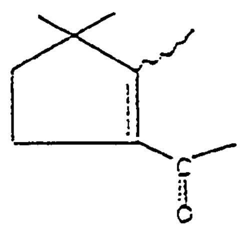

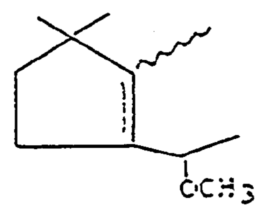

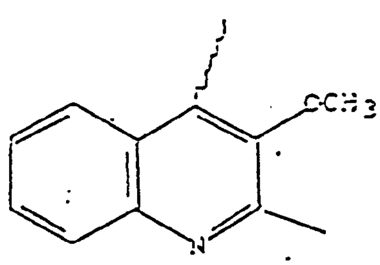

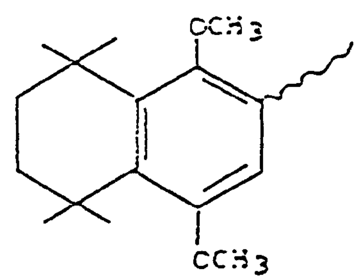

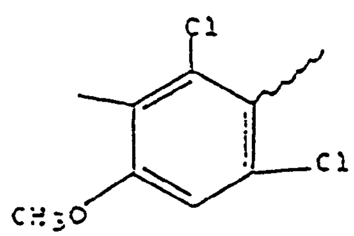

und R COOH, $CONHC_2H_5$ oder $COOC_2H_5$ darstellt.

**16.** Assoziat gemäß Anspruch 12, dadurch **gekennzeichnet**, daß das Retinoid unter den Verbindungen der folgenden Formeln sowie deren physiologisch verträglichen Salzen und Estern ausgewählt ist:

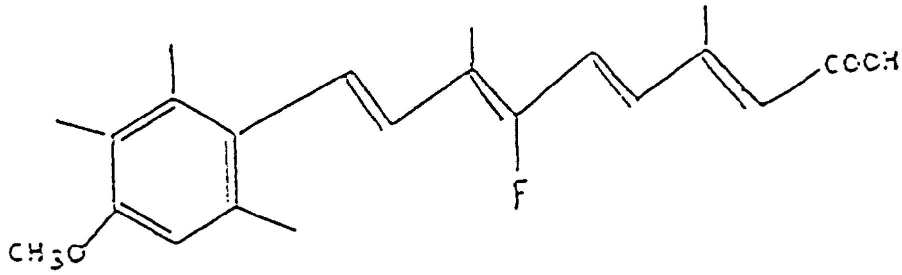

(VII)

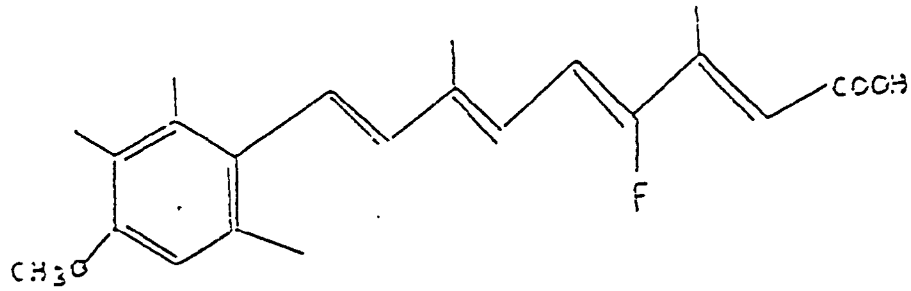

(VIII)

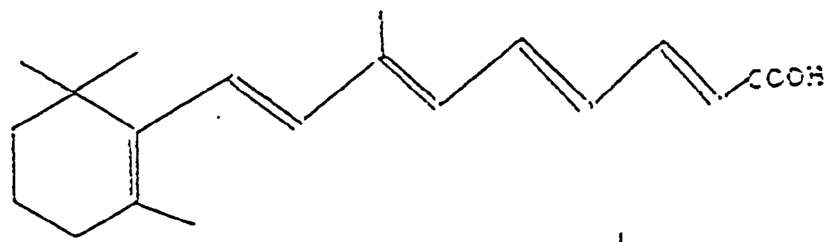

(IX)

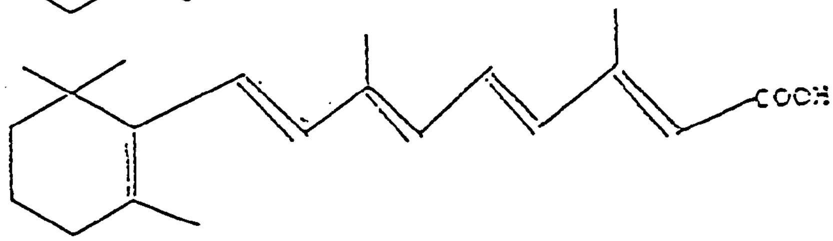

(X)

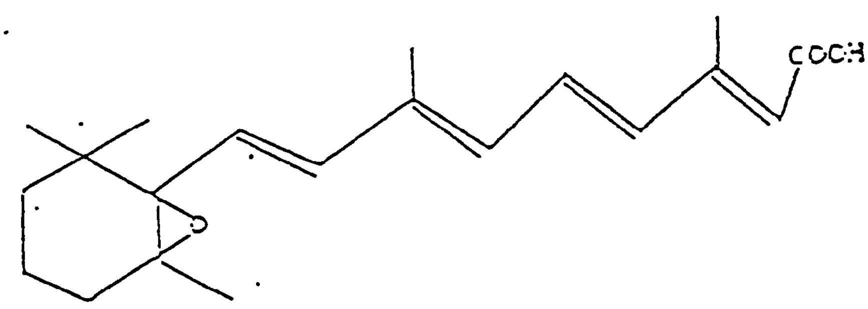

(XI)

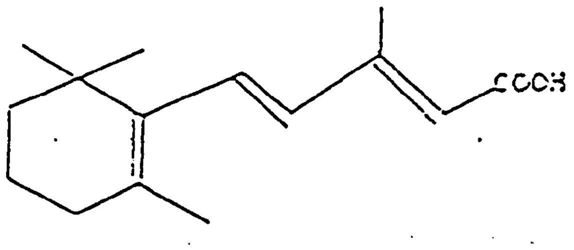

(XII)

**17.** Assoziat gemäß jedem Anspruch 12 bis 16, dadurch **gekennzeichnet**, daß das Retinoid unter den Verbindungen der Formel (II) sowie den pharmazeutisch oder kosmetisch verträglichen Salzen davon ausgewählt ist:

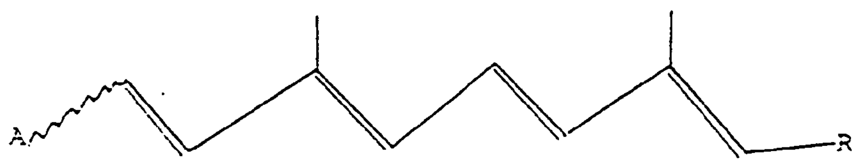

(II)

in der Form all-trans oder 13-cis, worin R eine Gruppe darstellt:

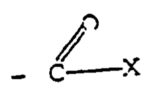

worin X eine OH-, OY-Gruppe bezeichnet, wobei Y eine Alkylgruppe mit 1 bis 15 Kohlenstoffatomen bedeutet, wobei X auch eine Aminogruppe darstellen kann, die gegebenenfalls durch eine Niedrigalkylgruppe mit 1 bis 6 Kohlenstoffatomen mono- oder disubstituiert ist, und worin R auch eine $-CH_2OH$-, -CHO-Gruppe darstellen kann, und worin A eine Gruppe bedeutet:

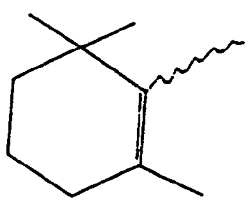

oder

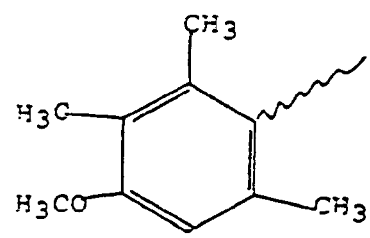

**18.** Assoziat gemäß jedem Anspruch 12 bis 15, dadurch **gekennzeichnet**, daß das Retinoid aus Tretinoin, Isotretinoin, Retinol oder Vitamin A und seinen Derivaten wie dem Acetat, Palmitat oder Propionat, Motretinid, Etretinat, Zink-all-trans-retinoat ausgewählt ist.

**19.** Assoziat gemäß jedem Anspruch 12 bis 18, dadurch **gekennzeichnet**, daß die Bestandteile (A) und (B) Teile ein- und derselben Zusammensetzung ausmachen.

**20.** Assoziat gemäß jedem Anspruch 12 bis 18, dadurch **gekennzeichnet**, daß die Bestandteile (A) und (B) dazu bestimmt sind, kurz vor der Aufbringung unvorbereitet vermischt zu werden.

**21.** Assoziat gemäß jedem anspruch 12 bis 18, dadurch **gekennzeichnet**, daß die Bestandteile (A) und (B) dazu bestimmt sind, in getrennter Weise oder nach einem Zeitablauf aufgebracht zu werden.

**22.** Assoziat gemäß jedem Anspruch 12 bis 21, dadurch **gekennzeichnet**, daß der Bestandteil (B) das Retinoid in Mengenverhältnissen von 0,001 bis 2 Gew.%, bezogen auf Gesamtgewicht der Zusammensetzung, enthält.

23. Vorrichtung aus mehreren Teilen, dadurch **gekennzeichnet**, daß sie mindestens zwei Teile umfaßt, wovon der eine den Bestandteil (A) und der andere den Bestandteil (B) wie in jedem Anspruch 12 bis 22 definiert enthält.

24. Verwendung der wie in jedem Anspruch 2 bis 10 definierten Zusammensetzung zur Herstellung eines Medikaments zur Behandlung der Alopezie.

25. Verwendung des wie in jedem Anspruch 12 bis 22 definierten Assoziats zur Herstellung eines Medikaments zur Behandlung der Alopezie.

26. Verfahren zur kosmetischen Behandlung der Haare, dadurch **gekennzeichnet**, daß man auf die behaarte Haut mindestens eine Zusammensetzung oder ein Assoziat wie in jedem Anspruch 1 bis 22 definiert aufbringt.

27. Verfahren gemäß Anspruch 26, dadurch **gekennzeichnet**, daß man zu einem ersten Zeitpunkt den Bestandteil (B), enthaltend das Retinoid, und nach einer Kontaktdauer von 1 Minute bis 12 h den Bestandteil A, enthaltend das Pyrimidinderivat der Formel (I) und das Filtermittel für die UV-Strahlungen, aufbringt.

28. Verfahren zur kosmetischen Behandlung behaarter Haut, dadurch **gekennzeichnet**, daß man auf die behaarte Haut eine Zusammensetzung aufbringt, die sich aus der Mischung der Bestandteile (A) und (B) des in jedem Anspruch 12 bis 22 definierten Assoziats ergibt.

29. Verfahren zum Löslichmachen eines im Anspruch 1 definierten Pyrimidinderivats der Formel (I) in einem physiologisch verträglichen Milieu,
dadurch **gekennzeichnet**, daß
man dem physiologisch verträglichen Milieu mindestens ein Sonnenfiltermittel zufügt, ausgewählt unter
2-Hydroxy-4-methoxybenzophenon,
p-Dimethyolamino-2-ethylhexylbenzoat,
p-Dimethylaminopentylbenzoat,
p-Methoxy-2-ethylhexylcinnamat,
4-(1,1-Dimethylethyl)-4′-methoxydibenzoylmethan,
(N-2-Ethylhexyl)-3-((3′-methoxy-4′-n-butoxy)benzyliden)10-camphosulfonamid,
3-(4-Methylbenzyliden)campher,
den Salicylaten von Homomenthyl und 2-Ethylhexyl,
p-Aminobenzoesäure,
2-Hydroxy-4-methoxybenzophenon-5-sulfonsäure.